# EUROPÄISCHE PATENTSCHRIFT

(11) **EP 1 458 685 B1**
(45) Veröffentlichungstag und Bekanntmachung des Hinweises auf die Patenterteilung: **02.05.2007**
(21) Anmeldenummer: 02792798.7
(22) Anmeldetag: 25.11.2002
(51) Int. Cl.: C07D 231/20, C07D 213/68, A01N 43/56, A01N 43/40

(54) **SUBSTITUIERTE PHENYLDERIVATE**
SUBSTITUTED PHENYL DERIVATIVES
DERIVES DE PHENYLE SUBSTITUES

(30) Priorität: 15.12.2001 DE 10161765
(43) Veröffentlichungstag der Anmeldung: 22.09.2004
(73) Patentinhaber: Bayer CropScience GmbH, 65929 Frankfurt/Main (DE)
(72) Erfinder: HELMKE, Hendrick, 65853 Liederbach (DE); HOFFMANN, Michael, Gerhard, 65439 Flörsheim (DE); HAAF, Klaus, 65779 Kelkheim (DE); WILLMS, Lothar, 65719 Hofheim (DE); AULER, Thomas, 65812 Bad Soden (DE); BIERINGER, Hermann, 65817 Eppstein (DE); MENNE, Hubert, 65719 Hofheim Ts. (DE)
(86) Internationale Anmeldenummer: PCT/EP2002/013231
(87) Internationale Veröffentlichungsnummer: WO 2003/051846

(56) Entgegenhaltungen:
- EP-A- 0 348 002
- WO-A-99/18057
- US-A- 3 884 955
- US-A- 5 698 495
- DATABASE CA [Online] CHEMICAL ABSTRACTS SERVICE, COLUMBUS, OHIO, US; HIROSE, HISAHIRO ET AL: "Positively charging electrophotographic photoreceptor" retrieved from STN Database accession no. 122:326459 XP002248533 -& JP 07 056366 A (KONISHIROKU PHOTO IND, JAPAN) 3. März 1995 (1995-03-03)
- DATABASE CA [Online] CHEMICAL ABSTRACTS SERVICE, COLUMBUS, OHIO, US; KAKIMOTO, MASAAKI ET AL: "Polyimides with good solubility and their manufacture" retrieved from STN Database accession no. 134:281290 XP002248534 -& JP 2001 098071 A (ZAIDAN HOJIN RIKOGAKU SHINKOKAI, JAPAN) 10. April 2001 (2001-04-10)

## Beschreibung

Es ist bekannt, daß substituierte Phenylderivate herbizide und pflanzenwachstumsregulierende Eigenschaften besitzen können (vgl. z.B. DE 3602-379-A, JP 10007657, US 5698495, US 5786392, WO 9718196). Diese weisen jedoch bei ihrer Anwendung häufig Nachteile auf, wie beispielsweise eine hohe Persistenz, unzureichende Selektivität in wichtigen Nutzpflanzenkulturen oder mangelnde Wirkung gegenüber Schadpflanzen.

Es wurden nun speziell substituierte Phenylderivate gefunden, die mit Vorteilen als Herbizide und Pflanzenwachstumsregulatoren eingesetzt werden können.

Gegenstand der vorliegenden Erfindung sind somit Verbindungen der Formel (I) und/oder deren Salze, 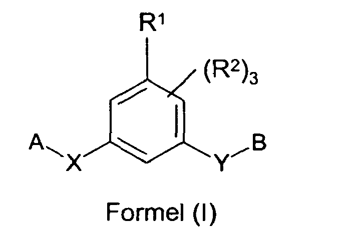 worin
- A: einen substituierten Pyrazolyl-, Thienyl- oder Pyridylrest der Formeln (Ia), (Ic) oder (Id) darstellt, 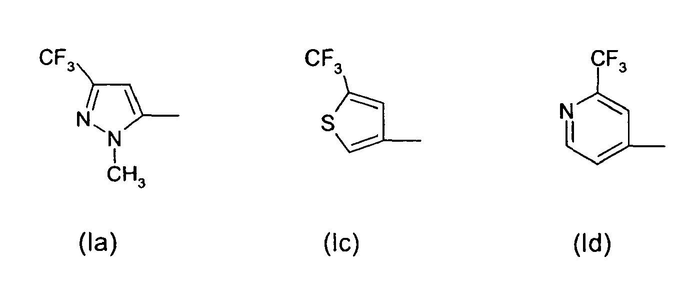
- X: O, S oder CH₂ bedeutet,
- R¹: Hydroxy, Halogen, CN, NC, CHO oder CO(C₁-C₈)Alkyl ist, wobei die Alkylgruppe unsubstituiert oder substituiert ist, z.B. durch einen oder mehrere Reste aus der Gruppe Hydroxy, Halogen, CN, (C₁-C₈)Alkoxy, (C₁-C₈)Alkylthio, (C₁-C₈)Alkylsulfinyl, (C₁-C₈)Alkylsulfonyl und [(C₁-C₈)Alkoxy]-carbonyl substituiert ist, oder CONH₂, CSNH₂, Nitro, SF₅, (C₁-C₈)Alkyl, (C₂-C₈)Alkenyl oder (C₂-C₈)Alkinyl ist, wobei die letztgenannten 3 Reste unsubstituiert oder substituiert sind, z.B. durch einen oder mehrere Reste aus der Gruppe Hydroxy, Halogen, CN, (C₁-C₈)Alkoxy, (C₁-C₈)Alkylthio, (C₁-C₈)Alkylsulfinyl, (C₁-C₈)Alkylsulfonyl und [(C₁-C₈)Alkoxy]-carbonyl substituiert sind, oder (C₁-C₈)Alkoxy, [(C₁-C₈)Alkyl]-carbonyl oder (C₁-C₈)Alkylsulfonyl ist, wobei die Reste unsubstituiert oder substituiert sind, z.B. durch einen oder mehrere Reste aus der Gruppe Hydroxy, Halogen, CN, (C₁-C₈)Alkoxy und (C₁-C₈)Alkylthio substituiert sind, oder
S(O)ₚ-R³ ist, wobei
p = 0, 1 oder 2 und
R³ (C₁-C₈)Alkyl, (C₁-C₈)Haloalkyl oder NR⁴R⁵ bedeutet, wobei R⁴,R⁵ unabhängig voneinander gleich oder verschieden H, (C₁-C₈)Alkyl, (C₂-C₈)Alkenyl, (C₇-C₁₀)Arylalkyl, (C₇-C₁₀)Alkylaryl oder (C₆-C₁₀)Aryl sind, wobei jeder der letztgenannten fünf Reste unsubstituiert oder substituiert ist, z.B. durch ein oder mehrere Reste aus der Gruppe Hydroxy, Halogen, CN, (C₁-C₈)Alkoxy und (C₁-C₈)Alkylthio substituiert ist, oder NR⁴R⁵ bedeutet, wobei R⁴,R⁵ unabhängig voneinander gleich oder verschieden H, (C₁-C₈)Alkyl, (C₂-C₈)Alkenyl, (C₇-C₁₀)Arylalkyl, (C₇-C₁₀)Alkylaryl oder (C₆-C₁₀)Aryl sind, wobei jeder der letztgenannten fünf Reste unsubstituiert oder substituiert ist, z.B. durch ein oder mehrere Reste aus der Gruppe Hydroxy, Halogen, CN, (C₁-C₈)Alkoxy und (C₁-C₈)Alkylthio substituiert ist,
oder R¹ eine Gruppe der Formel 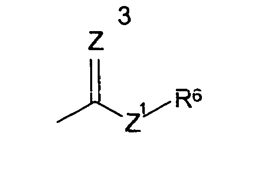 ist, wobei R⁶(C₁-C₈)Alkyl ist, welches unsubstituiert oder substituiert ist, z.B. durch ein oder mehrere Reste aus der Gruppe Hydroxy, Halogen, CN, (C₁-C₈)Alkoxy und (C₁-C₈)Alkylthio substituiert ist, und
Z = O oder S bedeutet, und
Z¹ = O oder S bedeutet,
- R²: gleich oder verschieden H, Halogen, CN oder (C₁-C₈)Alkyl sind, welche unsubstituiert oder substituiert sind, z.B. durch ein oder mehrere Reste aus der Gruppe Hydroxy, Halogen, CN, (C₁-C₈)Alkoxy und (C₁-C₈)Alkylthio substituiert sind,
- Y: O-(CR⁸R⁹)_{q}, S(O)_{q}, NH, CO(CR⁸R⁹)_{q} oder CR⁸R⁹ bedeutet und Y für den Fall, daß B ein optional substituierter Arylrest, ein optional substituierter Heterocyclylrest, Halogen oder CN ist, auch eine Bindung sein kann, wobei R⁸ und R⁹ gleich oder verschieden H, Hydroxy, Halogen, CN, (C₁-C₈)Alkoxy oder (C₁-C₈)Alkyl bedeuten, wobei jeder der beiden letztgenannten Reste unsubstituiert oder substituiert ist, z.B. durch ein oder mehrere Reste aus der Gruppe Hydroxy, Halogen, CN, (C₁-C₈)Alkoxy und (C₁-C₈)Alkylthio, und
q = 0, 1 oder 2 ist, und
- B: ein optional substituierter Arylrest, z.B. ein optional substituierter Phenylrest, oder ein optional substituierter heterocyclischer Rest, z.B. ein optional substituierter heteroaromatischer Rest, wie optional substituiertes Pyridyl, Pyrazolyl oder Thienyl,
H, OH, Halogen, CN, Nitro, SF₅, (C₁-C₈)Alkyl, (C₂-C₈)Alkenyl oder (C₂-C₈)Alkinyl ist, wobei die letztgenannten 3 Reste unsubstituiert oder substituiert sind, z.B. durch einen oder mehrere Reste aus Gruppe Hydroxy, Halogen, CN, (C₁-C₈)Alkoxy, (C₁-C₈)Alkylthio, (C₁-C₈)Alkylsulfinyl, (C₁-C₈)Alkylsulfonyl, [(C₁-C₈)Alkoxy]-carbonyl, (C₁-C₈)Haloalkoxy, (C₁-C₈)Haloalkylthio und (C₁-C₈)Alkoxy-(C₁-C₈)Alkoxy substituiert sind, oder
ein Acylrest, z.B. [(C₁-C₈)Alkyl]-carbonyl wie lineares oder verzweigtes [(C₁-C₈)-Alkyl]-carbonyl oder [(C₃-C₆)Cycloalkyl]-carbonyl, (C₆-C₁₄)Arylcarbonyl, (C₁-C₈)Alkylsulfonyl oder (C₆-C₁₄)Arylsulfonyl, wobei jeder der genannten Reste unsubstituiert oder substituiert ist, z.B. durch einen oder mehrere Reste aus der Gruppe Hydroxy, Halogen, (C₁-C₈)Alkoxy, (C₁-C₈)Alkylthio, (C₁-C₈)Alkylsulfinyl, (C₁-C₈)Alkylsulfonyl, [(C₁-C₈)Alkoxy]-carbonyl, (C₁-C₈)Haloalkoxy, (C₁-C₈)Haloalkylthio und CN substituiert ist, oder
- NR¹¹R¹²: ist, wobei
R¹¹,R¹² unabhängig voneinander gleich oder verschieden H, (C₁- C₈)Alkyl, (C₂-C₈)Alkenyl, (C₇-C₁₀)Arylalkyl, (C₇-C₁₀)Alkylaryl, (C₆-C₁₀)Aryl oder Heteroaryl sind, wobei jeder der letztgenannten sechs Reste unsubstituiert oder substituiert ist, z.B. durch ein oder mehrere Reste aus der Gruppe Hydroxy, Halogen, CN, (C₁-C₈)Alkoxy und (C₁-C₈)Alkylthio substituiert ist, oder ein Acylrest sind, z.B. [(C₁-C₈)Alkyl]-carbonyl wie lineares oder verzweigtes [(C₁-C₈)-Alkyl]-carbonyl oder [(C₃-C₆)Cycloalkyl]-carbonyl, (C₆-C₁₄)Arylcarbonyl, (C₆-C₁₄)Aryl-(C₁-C₈)Alkylcarbonyl, (C₁-C₈)Alkylsulfonyl oder (C₆-C₁₄)Arylsulfonyl, wobei jeder der genannten Reste unsubstituiert oder substituiert ist, z.B. durch einen oder mehrere Reste aus der Gruppe Hydroxy, Halogen, (C₁-C₈)Alkoxy, (C₁-C₈)Alkylthio, (C₁-C₈)Alkylsulfinyl, (C₁-C₈)Alkylsulfonyl, [(C₁-C₈)Alkoxy]-carbonyl, (C₁-C₈)Haloalkoxy, (C₁-C₈)Haloalkylthio und CN substituiert ist, oder
- B: ist eine Gruppe der Formel 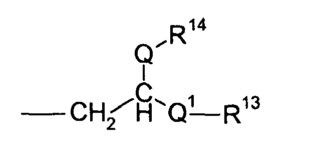 , wobei R¹³ (C₁-C₈)Alkyl ist, welches unsubstituiert oder substituiert ist, z.B. durch ein oder mehrere Reste aus der Gruppe Hydroxy, Halogen, CN, (C₁-C₈)Alkoxy und (C₁-C₈)Alkylthio,
R¹⁴ (C₁-C₈)Alkyl ist, welches unsubstituiert oder substituiert ist, z.B. durch ein oder mehrere Reste aus der Gruppe Hydroxy, Halogen, CN, (C₁-C₈)Alkoxy und (C₁-C₈)Alkylthio,
oder R¹³ und R¹⁴ zusammen einen Ring bilden,
Q = O oder S bedeutet, und
Q¹ = O oder S bedeutet.

In der Formel (I) und im folgenden können die kohlenstoffhaltigen Reste wie Alkyl-, Alkoxy-, Haloalkyl-, Alkylamino- und Alkylthioreste sowie die entsprechenden ungesättigten und/oder substituierten Reste im Kohlenstoffgerüst jeweils geradkettig oder verzweigt oder für Kohlenstoffanzahlen ab 3 auch cyclisch sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n-, i- oder Cyclo-Propyl, n-, i-, t-, 2-oder Cyclo-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet beispielsweise Fluor, Chlor, Brom oder Iod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein kohlenwasserstoffhaltiger Rest ist ein geradkettiger, verzweigter oder cyclischer und gesättigter oder ungesättigter aliphatischer oder aromatischer Rest, der Kohlenwasserstoffeinheiten aufweist, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl.

Aryl oder Arylrest bedeutet ein mono-, bi- oder polycyclisches, unsubstituiertes oder substituiertes aromatisches System, beispielsweise Phenyl, Naphthyl, Indenyl, Indanyl oder Pentalenyl, Fluorenyl, vorzugsweise Phenyl, welches z.B. durch einen oder mehrere, vorzugsweise 1, 2 oder 3 Reste aus der Gruppe Halogen, wie F, Cl, Br, I, vorzugsweise F, Cl und Br, ferner Alkyl, Haloalkyl, Alkoxy, Haloalkoxy, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsulfinyl und Alkylsulfonyl substituiert sein kann, und bei den Resten mit C-Atomen, solche mit 1 bis 4 C-Atomen, insbesondere 1 oder 2, bevorzugt sind. Bevorzugt sind dabei in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano.

Ein heterocyclischer Rest oder Ring (Heterocyclyl) kann gesättigt, ungesättigt oder heteroaromatisch und unsubstituiert oder substituiert sein, er kann auch ankondensiert sein; er enthält vorzugsweise ein oder mehrere Heteroatome im Ring, vorzugsweise aus der Gruppe N, O und S; vorzugsweise ist er ein gesättigter oder ungesättigter Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome wie N, O oder S enthält, oder ist ein partiell oder vollständig hydrierter Rest, z.B. Pyrrolidyl, Piperidyl, Pyrazolyl, Morpholinyl, Indolyl, Chinolinyl, Pyrimidinyl, Triazolyl, Oxazolyl, Pyridyl, Pyrimidinyl, Pyridazinyl, Pyrazinyl, Thiazolyl, Pyrrolyl,Oxazolinyl, Isoxazolinyl, Isoxazolyl, Imidazolyl und Benzoxazolyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte kohlenwasserstoffhaltige Reste, z.B. substituiertes Alkyl, Alkenyl, Alkinyl, Aryl, Phenyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Cyano und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy, Cyano und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Cyanophenyl.

Ein Acylrest bedeutet den Rest einer organischen Säure, der formal durch Abspaltung einer OH-Gruppe aus der organischen Säure entsteht, z.B. der Rest einer Carbonsäure und Reste davon abgeleiteter Säuren wie der Thiocarbonsäure, gegebenenfalls N-substituierten Iminocarbonsäuren oder die Reste von Kohlensäuremonoestern, gegebenenfalls N-substituierter Carbaminsäuren, Sulfonsäuren, Sulfinsäuren, Phosphonsäuren, Phosphinsäuren.

Ein Acylrest ist bevorzugt Formyl oder aliphatisches Acyl aus der Gruppe CO-R^{x}, CS-R^{x}, CO-OR^{x}, CS-OR^{x}, CS-SR^{x}, SOR^{Y} oder SO₂R^{Y}, wobei R^{x} und R^{Y} jeweils einen C₁-C₁₀-Kohlenwasserstoffrest bedeutet, der unsubstituiert oder substituiert ist, oder Aminocarbonyl oder Aminosulfonyl, wobei die beiden letztgenannten Reste unsubstituiert, N-monosubstituiert oder N,N-disubstituiert sind. Acyl bedeutet beispielsweise Formyl, Halogenalkylcarbonyl, Alkylcarbonyl wie (C₁-C₄)Alkylcarbonyl, Phenylcarbonyl, wobei der Phenylring substituiert sein kann, z.B. wie oben für Phenyl angegeben, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, z.B. solche bei denen ein Heteroatom wie N, O oder S protoniert vorliegt. Diese Salze sind beispielsweise Salze von Mineralsäuren wie Salzsäure, Bromwasserstoffsäure und Schwefelsäure oder auch Salze von organischen Säuren wie Ameisensäure, Essigsäure, Oxalsäure, Zitronensäure oder aromatischen Carbonsäuren wie Benzoesäuren.

Soweit Y ein Strukturelement O-(CR⁸R⁹)_{q} oder CO(CR⁸R⁹)_{q} ist, kann der Rest B an O bzw. CO oder an (CR⁸R⁹)_{q} gebunden sein, bevorzugt ist B an (CR⁸R⁹)_{q} gebunden.

Gegenstand der vorliegenden Erfindung sind auch Methoden zur Herstellung der Verbindungen der allgemeinen Formel (I) und/oder deren Salzen. Die erfindungsgemäßen Verbindungen der Formel (I) können über bekannte Methoden dargestellt werden. Von besonderem Interesse sind z.B. folgende Synthesen:

Setzt man beispielsweise eine Verbindung der Formel (II) mit Nucleophilen des Typs A-X-H und mit Nucleophilen des Typs B-Y-H um, so kann der Reaktionsverlauf beim erfindungsgemäßen Verfahren (a1) durch das folgende Formelschema beschrieben werden:

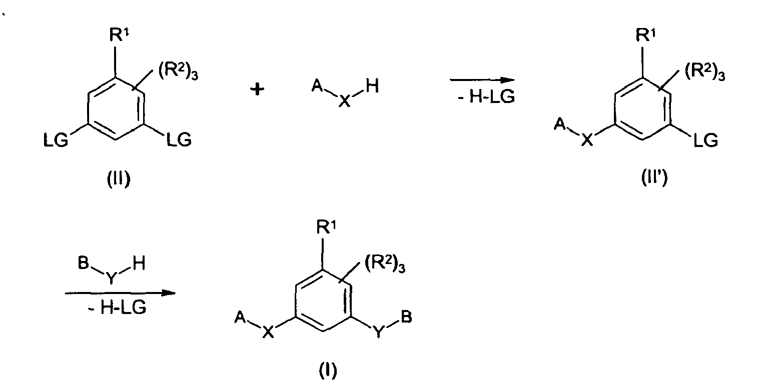

Die beim erfindungsgemäßen Verfahren (a1) zur Herstellung von Verbindungen der Formel (I) als Ausgangsstoffe verwendeten Phenylderivate sind durch die Formel (II) allgemein definiert. In der Formel (II) haben R¹ und R² die oben in Formel (I) angegebene Bedeutung, einschließlich der angegebenen Vorzugsbereiche, und LG sind gleiche oder voneinander verschiedene Abgangsgruppen wie Halogen oder Pseudohalogen, z.B. CN.
Die beim erfindungsgemäßen Verfahren (a1) zur Herstellung von Verbindungen der Formel (I) als Ausgangsstoffe verwendeten Nucleophile sind durch die Formel A-X-H und B-Y-H allgemein definiert, wobei A, X, B, und Y diejenigen Bedeutungen haben, die oben im Zusammenhang mit der Beschreibung der erfindungsgemäßen Verbindungen der Formel (I) angegeben wurden, einschließlich der angegebenen Vorzugsbereiche, und H steht für Wasserstoff.

Die Ausgangsstoffe der allgemeinen Formel (II), der Formel A-X-H und der Formel B-Y-H sind bekannt und/oder kommerziell erhältlich (siehe z.B. Chem. Het. Compounds 33, 1997, 995 - 996; Synthesis (2000) S. 1078 - 1080). Die Umsetzung zu Verbindungen der Formel (I) kann nach bekannten Verfahren erfolgen (siehe z.B. J.Med.Chem. 29 (1986) 887-889; J.Med.Chem. 39 (1996) 347-349). Die Reaktion kann in Abwesenheit oder Gegenwart eines Lösemittels, welches die Reaktion begünstigt oder zumindest nicht beeinträchtigt, durchgeführt werden. Bevorzugt sind polare, aprotische oder protische Solventien, wie N,N-Dimethylformamid, N,N-Dimethylacetamid, Dimethylsulfoxid, Sulfolane, Acetonitril, Methylethylketon oder Ether, wie Dioxan oder Tetrahydrofuran, oder Alkohole oder Wasser oder Mischungen der genannten Lösemittel. Die Reaktionen werden durchgeführt bei Temperaturen zwischen Raumtemperatur und Rückflußtemperatur der Reaktionsmischung, bevorzugt bei erhöhter Temperatur, insbesondere Rückflußtemperatur. Die Reaktionen können in Gegenwart einer Base, wie Alkalihydroxid, Erdalkalihydroxid, Alkalialkoxid, Alkalihalogenid, Alkalihydrid oder einer organischen Base durchgeführt werden, beispielsweise seien Kaliumydroxid, Natriumhydroxid, Natriumethanolat, Natriummethanolat, Cäsiumfluorid, Triethylamin, Natriumhydrid genannt. Die Reaktion kann als Eintopfreaktion oder in getrennten Stufen durchgeführt werden.

Setzt man beispielsweise eine Verbindung der Formel (II) mit Nucleophilen des Typs B-Y-H und mit Nucleophilen des Typs A-X-H um, so kann der Reaktionsverlauf beim erfindungsgemäßen Verfahren (a2) durch das folgende Formelschema beschrieben werden:

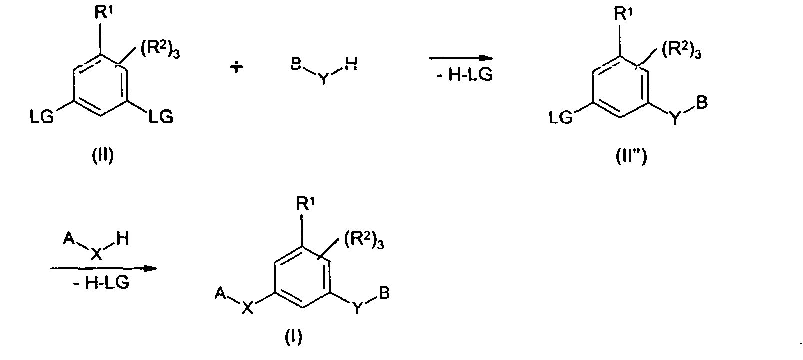

Die beim erfindungsgemäßen Verfahren (a2) zur Herstellung von Verbindungen der Formel (I) als Ausgangsstoffe verwendeten Phenylderivate sind durch die Formel (II) allgemein definiert. In der Formel (II) haben R¹ und R² die oben in Formel (I) angegebene Bedeutung, einschließlich der angegebenen Vorzugsbereiche, und LG sind gleiche oder voneinander verschiedene Abgangsgruppen wie Halogen oder Pseudohalogen, z.B. CN.
Die beim erfindungsgemäßen Verfahren (a2) zur Herstellung von Verbindungen der Formel (I) als Ausgangsstoffe verwendeten Nucleophile sind durch die Formel A-X-H und B-Y-H allgemein definiert, wobei A, X, B, und Y vorzugsweise diejenigen Bedeutungen haben, die oben im Zusammenhang mit der Beschreibung der erfindungsgemäßen Verbindungen der Formel (I) angegeben wurden, einschließlich der angegebenen Vorzugsbereiche, und H steht für Wasserstoff. Die Ausgangsstoffe der allgemeinen Formel (II), der Formel A-X-H und der Formel B-Y-H sind bekannt und/oder kommerziell erhältlich (siehe z.B. Chem. Het. Compounds 33, 1997, 995 - 996; Synthesis (2000) S. 1078 - 1080). Die Umsetzung zu Verbindungen der Formel (I) kann nach bekannten Verfahren erfolgen (siehe z.B. J.Med.Chem. 29 (1986) 887-889; J.Med.Chem. 39 (1996) 347-349). Die Reaktion kann in Abwesenheit oder Gegenwart eines Lösemittels, welches die Reaktion begünstigt oder zumindest nicht beeinträchtigt, durchgeführt werden. Bevorzugt sind polare, aprotische oder protische Solventien, wie N,N-Dimethylformamid, N,N-Dimethylacetamid, Dimethylsulfoxid, Sulfolane, Acetonitril, Methylethylketon oder Ether, wie Dioxan oder Tetrahydrofuran, oder Alkohole oder Wasser oder Mischungen der genannten Lösemittel. Die Reaktionen werden durchgeführt bei Temperaturen zwischen Raumtemperatur und Rückflußtemperatur der Reaktionsmischung, bevorzugt bei erhöhter Temperatur, insbesondere Rückflußtemperatur. Die Reaktionen können in Gegenwart einer Base, wie Alkalihydroxid, Erdalkalihydroxid, Alkalialkoxid, Alkalihalogenid, Alkalihydrid oder einer organischen Base durchgeführt werden, beispielsweise seien Kaliumydroxid, Natriumhydroxid, Natriumethanolat, Natriummethanolat, Cäsiumfluorid, Triethylamin, Natriumhydrid genannt. Die Reaktion kann als Eintopfreaktion oder in getrennten Stufen durchgeführt werden.

Setzt man beispielsweise eine Verbindung der Formel (III) oder (III') mit Boronsäurederivate des Typs (IV) oder (IV') um, so kann der Reaktionsverlauf beim erfindungsgemäßen Verfahren (b) durch das folgende Formelschema einer Kupplungsreaktion beschrieben werden:

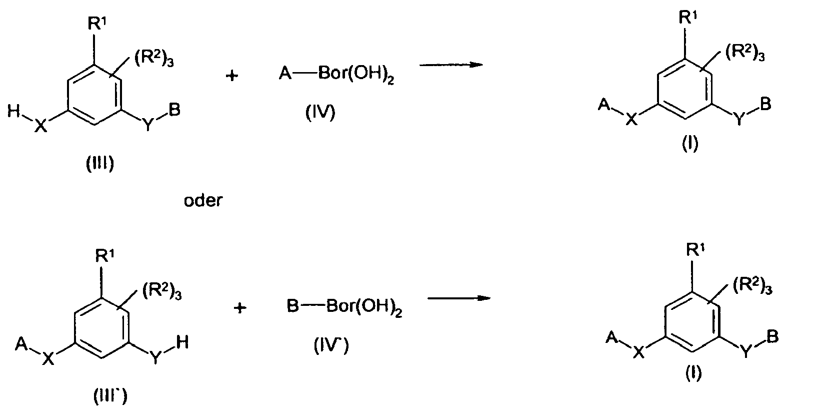

Die beim erfindungsgemäßen Verfahren (b) zur Herstellung von Verbindungen der Formel (I) als Ausgangsstoffe verwendeten Phenylderivate sind durch die Formel (III) und (III') allgemein definiert. In der Formel (III) und (III') haben R¹, R², X, Y, A und B die oben in Formel (I) angegebene Bedeutung, einschließlich der angegebenen Vorzugsbereiche. Die beim erfindungsgemäßen Verfahren (b) zur Herstellung von Verbindungen der Formel (I) als Ausgangsstoffe verwendeten Boronsäurederivate der Formel (IV) und (IV') sind durch die Formel A-Bor(OH)₂ und B-Bor(OH)₂ gekennzeichnet, wobei A und B die Bedeutungen haben, die oben im Zusammenhang mit der Beschreibung der erfindungsgemäßen Verbindungen der Formel (I) angegeben wurden, einschließlich der angegebenen Vorzugsbereiche.

Die Kupplungsreaktion wird normalerweise in Gegenwart eines Übergangsmetallkomplexes durchgeführt, wie es z.B. in Tetrahedron Letters 39 (1998) 2933ff. beschrieben ist. Bevorzugte Übergangsmetalle sind Cu, Pd oder Ni. Die Reaktion kann in Abwesenheit oder Gegenwart eines Lösemittels, welches die Reaktion begünstigt oder zumindest nicht beeinträchtigt, durchgeführt werden. Die Ausgangsstoffe der allgemeinen Formel (III) und (III') und der allgemeinen Formel (IV) und (IV') sind bekannt und/oder kommerziell erhältlich und/oder können nach bekannten Verfahren hergestellt werden (siehe z.B. J.Organomet.Chem. 309 (1986) 241-246; J.Amer.Chem.Soc. 112 (1990) 8024-8034; EP 1108720; ). Die Reaktion kann in Abwesenheit oder in Gegenwart eines Lösemittels, welches die Reaktion begünstigt oder zumindest nicht beeinträchtigt, durchgeführt werden. Bevorzugt sind polare oder unpolare, aprotische oder protische Solventien, wie N,N-Dimethylformamid, N,N-Dimethylacetamid, Dimethylsulfoxid, Sulfolane, Dichlormethan, Dichlorethan, Acetonitril oder Ether, wie Dioxan oder Tetrahydrofuran, oder Mischungen der genannten Lösemittel. Die Reaktionen werden durchgeführt bei Temperaturen zwischen Raumtemperatur und Rückflußtemperatur der Reaktionsmischung, bevorzugt bei erhöhter Temperatur, insbesondere Rückflußtemperatur. Die Reaktionen können in Gegenwart einer anorganischen oder organischen Base durchgeführt werden, beispielsweise seien Triethylamin, Pyridin oder Thaliumhydroxid genannt. Die Reaktionen können in Gegenwart oder Abwesenheit von Molsieb durchgeführt werden.

Setzt man beispielsweise ein Boronsäurederivat der Formel (V) oder (V') mit Nucleophilen des Typs A-X-H oder B-Y-H um, so kann der Reaktionsverlauf beim erfindungsgemäßen Verfahren (c) durch das folgende Formelschema einer Kupplungsreaktion beschrieben werden:

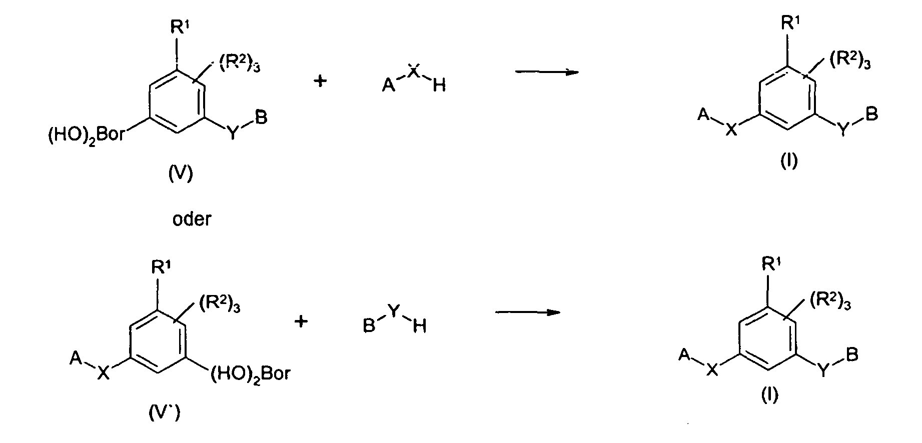

Die beim erfindungsgemäßen Verfahren (c) zur Herstellung von Verbindungen der Formel (I) als Ausgangsstoffe verwendeten Phenylderivate sind durch die Formel (V) und (V') allgemein definiert. In der Formel (V) und (V') haben R¹,R², X, Y, A und B die oben in Formel (I) angegebene Bedeutung, einschließlich der angegebenen Vorzugsbereiche. Die beim erfindungsgemäßen Verfahren (c) zur Herstellung von Verbindungen der Formel (I) als Ausgangsstoffe verwendeten Verbindungen der Formel A-X-H und B-Y-H sind bekannt und/oder kommerziell erhältlich, wobei A, B, X und Y die Bedeutungen haben, die oben im Zusammenhang mit der Beschreibung der erfindungsgemäßen Verbindungen der Formel (I) angegeben wurden, einschließlich der angegebenen Vorzugsbereiche, und H bedeutet Wasserstoff. Die Reaktion wird normalerweise in Gegenwart eines Übergangsmetallkomplexes durchgeführt, wie es z.B. in Tetrahedron Letters 39 (1998) 2933ff. beschrieben ist. Bevorzugte Übergangsmetalle sind Cu, Pd oder Ni. Die Reaktion kann in Abwesenheit oder in Gegenwart eines Lösemittels, welches die Reaktion begünstigt oder zumindest nicht beeinträchtigt, durchgeführt werden. Die Ausgangsstoffe der allgemeinen Formel (V) und (V') sind bekannt und/oder kommerziell erhältlich und/oder können nach bekannten Verfahren hergestellt werden (siehe z.B. EP 1108720 und J.Organomet.Chem. 309 (1986) 241-246). Die Reaktion kann in Abwesenheit oder Gegenwart eines Lösemittels, welches die Reaktion begünstigt oder zumindest nicht beeinträchtigt, durchgeführt werden. Bevorzugt sind polare oder unpolare, aprotische oder protische Solventien, wie N,N-Dimethylformamid, N,N-Dimethylacetamid, Dimethylsulfoxid, Sulfolane, Dichlormethan, Dichlorethan, Acetonitril oder Ether, wie Dioxan oder Tetrahydrofuran, oder Mischungen der genannten Lösemittel. Die Reaktionen werden durchgeführt bei Temperaturen zwischen Raumtemperatur und Rückflußtemperatur der Reaktionsmischung, bevorzugt bei erhöhter Temperatur, insbesondere Rückflußtemperatur. Die Reaktionen können in Gegenwart einer anorganischen oder organischen Base durchgeführt werden, beispielsweise seien Triethylamin, Pyridin oder Thaliumhydroxid genannt. Die Reaktionen können in Gegenwart oder Abwesenheit von Molsieb durchgeführt werden.

Reduziert und acyliert man beispielsweise eine Verbindung der Formel (VI), so kann der Reaktionsverlauf zu Verbindungen der Formel (I) mit Y = CH₂ und B = NH-Acyl beim erfindungsgemäßen Verfahren (d) durch das folgende Formelschema beschrieben werden:

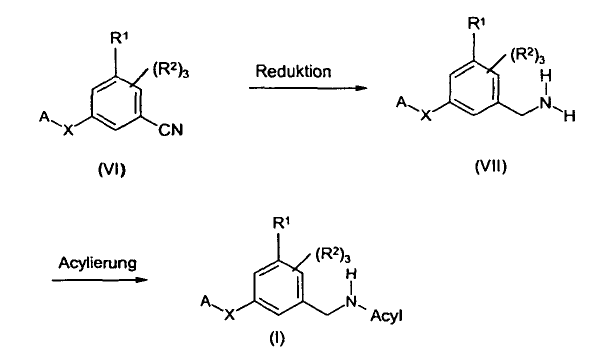

Die beim erfindungsgemäßen Verfahren (d) zur Herstellung von Verbindungen der Formel (I) als Ausgangsstoffe verwendeten Benzonitrilderivate sind durch die Formel (VI) allgemein definiert. In der Formel (VI) haben R¹, R², A und X die oben in Formel (I) angegebene Bedeutung, einschließlich der angegebenen Vorzugsbereiche. Die Ausgangsstoffe der allgemeinen Formel (VI) sind bekannt und/oder kommerziell erhältlich und/oder können nach bekannten Verfahren hergestellt werden (siehe z.B. Russ.J.Org.Chem. 32 (1996) 1505-1509). Die Reduktion von Nitrilen zu Aminen ist in der Literatur vielfältig beschrieben (siehe z.B. Eugen Müller, Methoden der organischen Chemie (Houben-Weyl) Band XI/1, Stickstoffverbindungen II, S. 343 ff., Georg Thieme Verlag, Stuttgart 1957). Unter anderem kommen edelmetallkatalysierte Hydrierungen in Betracht, wobei Palladium und Platin katalysierte Reaktionen von besonderem Interesse sind aber auch Reduktionen mit Raney-Nickel möglich sind. Ferner sind auch Reduktionen durch komplexe Hydridreagentien wie z.B. Lithiumaluminiumhydrid, Boran-THF-Komplex, Superhydrid, oder Diboran möglich. Die Reduktion kann bei Temperaturen von 0 - 250°C und bei Drucken von 1 - 100 bar durchgeführt werden. Verbindungen der allgemeinen Formel (VII) können durch Umsetzung mit Acylierungsreagentien wie Säurehalogenide, Isocyanate, Carbamoylchloride, Chlorameisensäureestern, Sulfonylchloride, Sulfamoylchloride, Sulfenylchloriden, Isothiocyanaten zu Verbindungen der allgemeinen Formel (I) umgesetzt werden, worin Y = CH₂ und B = NH-Acyl ist, und A, X, R¹, R² die in Formel (I) angegebene Bedeutung haben. Ein Zugang zu allgemeinen und speziellen chemischen Methoden der Acylierung findet sich z.B. in: Jerry March, Advanced Organic Chemistry (Reaction, Mechanisms and Structure) 4^{th} Edition, John Wiley & Sons, New York, 1992.

Verseift man beispielsweise eine Verbindung der Formel (VI) und setzt sie mit einem Amin NH₂-R¹² um, so kann der Reaktionsverlauf zu Verbindungen der Formel (I) mit Y = CO und B = NHR¹² beim erfindungsgemäßen Verfahren (e) durch das folgende Formelschema beschrieben werden:

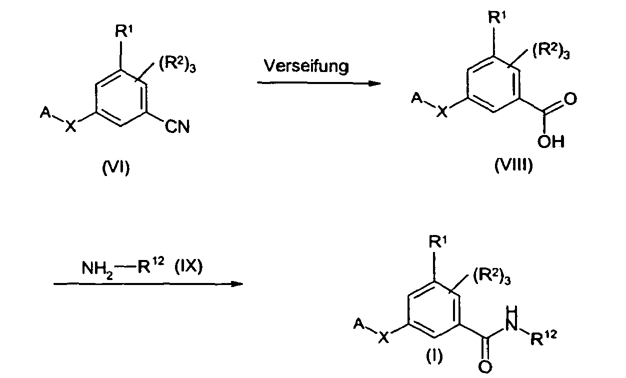

Die beim erfindungsgemäßen Verfahren (e) zur Herstellung von Verbindungen der Formel (I) als Ausgangsstoffe verwendeten Benzonitrilderivate sind durch die Formel (VI) allgemein definiert. In der Formel (VI) haben R¹, R², A und X die oben in Formel (I) angegebene Bedeutung einschließlich der angegebenen Vorzugsbereiche. Die Verbindungen der Formel (VI) lassen sich nach bekannten Verfahren herstellen (siehe z.B. Russ. J. Org. Chem. 32, 1996, S. 1505 - 1509). Die Verseifung von Nitrilen zu Carbonsäuren ist in der Literatur vielfältig beschrieben (siehe z.B. J. Am. Chem. Soc. 107 (1985) 7967ff., J. Am. Chem. Soc. 78 (1956) 450ff., J. Org. Chem. 51 (1986) 4169ff., Org. Synth. Collect. Vol. 1 - 4). Die Umsetzung der Verbindungen der Formel (VIII) und (IX) erfolgt vorzugsweise in einem inerten organischen Lösungsmittel, wie Tetrahydrofuran (THF), Dichlormethan, 1,2-Dichlorethan, Chloroform, Dimethylformamid, bei Temperaturen zwischen -10 °C und dem Siedepunkt des Lösungsmittel, vorzugsweise bei 0 °C bis 60 °C, wobei im ersten Reaktionsschritt die Carbonsäure der Formel (VIII) in das korrespondierende Säurehalogenid überführt wird. Die Herstellung des Säurehalogenids erfolgt gemäß literaturbekannten Verfahren, wobei man z.B. Oxalylchlorid, Thionylchlorid, Phosphorpentachlorid, Phosphoroxychlorid, oder Phosphortribromid in Gegenwart katalytischer oder äquimolarer Mengen Dimethylformamid zur Halogenierung verwendet. Nachfolgend erfolgt eine Umsetzung mit dem Amin der Formel (IX), worin R¹² wie in Formel (I) definiert ist, vorzugsweise in Gegenwart von Basen oder basischer Kataysatoren. Als Basen bzw. basische Katalysatoren eignen sich Alkalicarbonate, Alkalialkoholate, Erdalkalicarbonate, Erdalkalialkoholate oder organische Basen wie Triethylamin, 1,8-Diazabicylco[5.4.0]undec-7-en (DBU) oder 4-Dimethylaminopyridin (DMAP). Die jeweilige Base wird dabei beispielsweise im Bereich von 0,1 bis 3 Moläquivalenten bezogen auf die Verbindung der Formel (III) eingesetzt. Die Verbindung der Formel (IX) kann im Verhältnis zur Verbindung der Formel (VIII) beispielsweise äquimolar oder mit bis zu 2 Moläquvälenten Überschuß eingesetzt werden. Grundsätzlich sind die entsprechenden Verfahren in der Literatur bekannt (vergleiche: Organikum, VEB Deutscher Verlag der Wissenschaften, Berlin 1988, Jerry March, Advanced Organic Chemistry (Reaction, Mechanisms and Structure) 4^{th} Edition, John Wiley & Sons, New York, 1992.
Setzt man beispielsweise eine Verbindung der Formel (VI) mit einer Organometallverbindung (z.B. Grignardreagentien, Organozinkverbindungen oder Organolithiumverbindungen) um, so kann der Reaktionsverlauf zu Verbindungen der Formel (I) mit Y = CO beim erfindungsgemäßen Verfahren (f) durch das folgende Formelschema beschrieben werden:

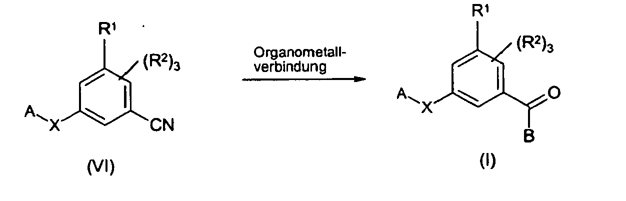

Die beim erfindungsgemäßen Verfahren (f) zur Herstellung von Verbindungen der Formel (I) als Ausgangsstoffe verwendeten Benzonitrilderivate sind durch die Formel (VI) allgemein definiert. In der Formel (VI) haben R¹, R², A und X die oben in Formel (I) angegebene Bedeutung einschließlich der angegebenen Vorzugsbereiche. Die verwendeten Organometallverbindungen, z.B. der Formel B-Mg-Br, B-Li oder B-Zn-Cl, sind kommerziell erhältlich und/oder nach bekannten Verfahren zugänglich (siehe z.B. M. Schlosser: Organometallics in Synthesis, John Wiley & Sons 1994). Die Verbindungen der Formel (VI) lassen sich nach bekannten Verfahren herstellen (siehe z.B. Russ. J. Org. Chem. 32, 1996, S. 1505 - 1509). Die Umsetzung von Benzonitrilen, z.B. zu Benzophenonderivaten, ist in der Literatur vielfältig beschrieben (siehe z.B. Tetrahedron Lett. 2000, 41 (6), 937-939; J. Org. Chem. 2000, 65 (12), 3861-3863; Synth. Commun. 1998, 28 (21), 4067-4075; J. Med. Chem. 1998, 41 (22), 4400-4407; Synth. Commun. 1996, 26 (4), 721-727; Synthesis (1991) 1, 56-58; Angew. Chem., Int. Ed. Engl. 1965, 4, 1077; J. Am. Chem. Soc. 1970, 92, 336). Die Umsetzung von Verbindungen der Formel (VI) mit den Organometallverbindungen erfolgt vorzugsweise in einem inerten organischen Lösungsmittel, wie Tetrahydrofuran (THF), Dioxan, Diethylether oder Diisopropylether bei Temperaturen zwischen -78 °C und dem Siedepunkt des Lösungsmittel, vorzugsweise bei 0 °C bis 120 °C. Die Reaktion kann in Gegenwart in Abwesenheit oder Gegenwart eines Katalysators, wie z.B. Lil, Cul oder CuBr, durchgeführt werden.
Kollektionen aus Verbindungen der Formel (I) und/oder deren Salzen, die nach oben genannten Schemata synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, England, H+P Labortechnik GmbH, Bruckmannring 28, 85764 Oberschleißheim, Deutschland oder der Firma Radleys, Shirehill, Saffron Walden, Essex, CB - II:3AZ, England angeboten werden. Für die parallelisierte Aufreinigung von Verbindungen der allgemeinen Formel (I) und deren Salze beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen kann die Herstellung von Verbindungen der allgemeinen Formel (I) und/oder deren Salze vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen- unterstützte -Synthesemethoden sind in der Fachliteratur hinreichend beschrieben, z.B. Barry A. Bunin in "The Combinatorial Index", Verlag Academic Press, 1998. Die Verwendung von Festphasen- unterstützten - Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) und/oder deren Salze in Form von Substanzkollektionen, die Bibliotheken genannt werden. Gegenstand der vorliegenden Erfindung sind auch Bibliotheken, die mindestens zwei Verbindungen der Formel (I) und/oder deren Salze enthalten.

Die erfindungsgemäßen Verbindungen der Formel (I) und/oder deren Salze, im folgenden zusammen auch als erfindungsgemäße Verbindungen bezeichnet, weisen eine ausgezeichnete herbizide Wirksamkeit gegen ein breites Spektrum wirtschaftlich wichtiger Schadpflanzen wie mono- und dikotyler Schadpflanzen auf. Auch schwer bekämpfbare perennierende Unkräuter, die aus Rhizomen, Wurzelstöcken oder anderen Dauerorganen austreiben, werden durch die erfindungsgemäßen Verbindungen gut erfaßt. Dabei können die erfindungsgemäßen Verbindungen z.B. im Vorsaat-, Vorauflauf- oder Nachauflaufverfahren ausgebracht werden, z.B. auf die Pflanzen, Pflanzensamen oder die Fläche auf der die Pflanzen wachsen. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Bromusarten und Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Verbindungen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der erfindungsgemäßen Verbindungen auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. zweikeimblättriger Kulturen wie Soja, Baumwolle, Raps, Zuckerrüben, insbesondere Soja, oder Gramineen-Kulturen wie Weizen, Gerste, Hafer, Roggen, Reis oder Mais, nur unwesentlich oder gar nicht geschädigt. Die vorliegenden Verbindungen eignen sich aus diesen Gründen sehr gut zur selektiven Bekämpfung von unerwünschtem Pflanzenwuchs (z.B. Schadpflanzen) in landwirtschaftlichen Nutzpflanzungen oder in Zierpflanzungen.

Darüberhinaus weisen die erfindungsgemäßen Verbindungen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da die Lagerbarkeit hierdurch verringert oder völlig verhindert werden kann.

Aufgrund ihrer herbiziden und pflanzenwachstumsregulatorischen Eigenschaften können die erfindungsgemäßen Verbindungen auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind. Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991 ), 95-106).
Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Verbindungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form verschiedener Formulierungen, z.B. in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.
Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.
Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.
Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.
Die agrochemischen Zubereitungen enthalten in der Regel 0,1 bis 99 Gew.-%, insbesondere 0.1 bis 95 Gew.-%, Wirkstoff der Formel (I) und/oder deren Salze. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.
Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstunashemmer und den pH-Wert und die Viskosität beeinflussende Mittel.

Als Kombinationspartner für die erfindungsgemäßen Verbindungen in Mischungsformulierungen oder im Tank-Mix sind beispielsweise bekannte Wirkstoffe wie Herbizide, Insektizide, Fungizide oder Safener einsetzbar, wie sie z.B. aus Weed Research 26 (1986) 441-445 oder "The Pesticide Manual", 12th edition, The British Crop Protection Council and the Royal Soc. of Chemistry, 2000 und dort zitierter Literatur beschrieben sind. Als bekannte Herbizide, die mit den erfindungsgemäßen Verbindungen kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azafenidin; azimsulfuron (DPX-A8947); aziprotryn; barban; BAS 516 H, d.h. 5-Fluor-2-phenyl-4H-3,1-benzoxazin-4-on; BAS 620 H; BAS 65400H; BAY FOE 5043; benazolin; benfluralin; benfuresate; bensulfuron-methyl; bensulide; bentazone; benzofenap; benzofluor; benzoylprop-ethyl; benzthiazuron; bialaphos; bifenox; bispyribac-Na; bromacil; bromobutide; bromofenoxim; bromoxynil; bromuron; buminafos; busoxinone; butachlor; butamifos; butenachlor; buthidazole; butralin; butroxydim; butylate; cafenstrole (CH-900); caloxydim; carbetamide; carfentrazone-ethyl; CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cloransulam-methyl; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diclosulam, d.h. N-(2,6-Dichlorphenyl)-5-ethoxy-7-fluor-[1,2,4]triazolo[1,5-c]pyrimidin-2-sulfonamid; diethatyl; difenoxuron; difenzoquat; diflufenican; diflufenzopyr (BAS 654 00H), dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1 H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); flupyrsulfuron-methyl-sodium; fluridone; flurochloridone; fluroxypyr; flurtamone; fluthiacet-methyl; fomesafen; foramsulfuron und dessen Salze wie das Natriumsalz; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazamox; imazapyr; imazaquin und Salze wie das Ammoniumsalz; imazethamethapyr; imazethapyr; imazosulfuron; indanofan (MK-243), iodosulfuron-methyl und dessen Salze wie das Natriumsalz; ioxynil; isocarbamid; isopropalin; isoproturon; isouron; isoxaben; isoxaflutole; isoxapyrifop; karbutilate; lactofen; lenacil; linuron; MCPA; MCPB; mecoprop; mefenacet; mefluidid; mesosulfuron-methyl und dessen Salze wie das Natriumsalz; metamitron; metazachlor; methabenzthiazuron; metham; methazole; methoxyphenone; methyldymron; metobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxasulfuron; oxaziclomefone (MY-100); oxyfluorfen; paraquat; pebulate; pendimethalin; pentoxazone (KPP-314); perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyroflufen-ethyl; pyrazolinate; pyrazon; pyrazosulfuron-ethyl; pyrazoxyfen; pyribenzoxim (LGC-40836); pyributicarb; pyridate; pyriminobac-methyl; pyrithiobac (KIH-2031 ); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione; sulfentrazon (FMC-97285, F-6285); sulfazuron; sulfometuron-methyl; sulfosate (ICI-A0224); sulfosulfuron; TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triaziflam, triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; JTC-101; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Die erfindungsgemäßen Verbindungen können auch in Kombination mit einem oder mehreren als Safener wirkenden Verbindungen eingesetzt werden. Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, kann die erforderliche Aufwandmenge der erfindungsgemäßen Verbindungen variieren. Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### Beispiele

### A. Chemische Beispiele

- Abkürzungen:: Die %-Angaben und Mengenverhältnisse beziehen sich auf das Gewicht, wenn nicht näher spezifiziert.
i. Vak. = unter reduziertem Druck
h = Stunde(n)

1. 3,5-Bis-(1-Methyl-3-trifluormethylpyrazol-5-yloxy)-benzonitril 2.00 g (14.4 mmol) 3,5-Difluorbenzonitril wurden unter Stickstoffatmosphäre in 15 ml Sulfolan vorgelegt und bei Raumtemperatur portionsweise mit 4.77 g (34.5 mmol) Kaliumcarbonat versetzt. Anschließend gab man 5.25 g (31.60 mmol) 1-Methyl-3-(trifluormethyl)-2-pyrazol-2-on zu und erwärmte für 10 h auf 150°C, kühlte auf Raumtemperatur ab, gab Wasser und Essigester zur Reaktionslösung und rührte einige Minuten nach. Die Phasen wurden getrennt und die organische Phase mehrfach mit Wasser, dann mit Natriumhydoxidlösung und zuletzt mit gesättigter Kochsalzlösung gewaschen und anschießend über Magnesiumsulfat getrocknet, filtriert und eingeengt. Die Säulenchromatographie des Rohproduktes ergab das 3,5-Bis-(1-Methyl-3-trifluormethylpyrazol-5-yloxy)-benzonitril in Form weißer Kristalle.
   Ausbeute: 1.19 g (19 % d.Th.); Schmelzpunkt: 139°C.
2. 3-Fluor-5-(1-Methyl-3-trifluormethylpyrazol-5-yloxy)-benzonitril 5.00 g (35.9 mmol) 3,5-Difluorbenzonitril wurden unter Stickstoffatmosphäre in 60 ml N,N-Dimethylformamid vorgelegt und bei Raumtemperatur mit 6.46 g (46.7 mmol) Kalium-carbonat und 6.57 g (39.5 mmol) 1-Methyl-3-(trifluormethyl)-2-pyrazol-2-on versetzt. Man erwärmte für 2 h auf 150°C, kühlte auf Raumtemperatur ab und gab Wasser zur Reaktions-lösung hinzu. Man extrahierte zweimal mit Heptan/Essigester (1:1) und zweimal mit Essig-ester. Die vereinigten Phasen wurden mit Wasser gewaschen und anschießend über Magnesiumsulfat getrocknet, filtriert und eingeengt. Säulenchromatographie des Roh-produktes ergaben 4.19 g 3-Fluor-5-(1-Methyl-3-trifluormethylpyrazol-5-yloxy)-benzonitril in Form weißer Kristalle sowie als Nebenprodukt 2.8 g 3,5-Bis-(1-Methyl-3-trifluormethyl-pyrazol-5-yloxy)-benzonitril in Form weißer Kristalle.
   Ausbeute: 4.19 g (39 % d.Th.); Schmelzpunkt: 84°C.
3. 3,5-Bis-(2-Trifluormethylpyridin-4-yloxy)-benzonitril 0.556 g (4.0 mmol) 3,5-Difluorbenzonitril wurden unter Stickstoffatmosphäre in 10 ml N,N-Dimethylacetamid vorgelegt und bei Raumtemperatur portionsweise mit 1.22 g (8.8 mmol) Kaliumcarbonat versetzt. Anschließend gab man 1.305 g (8.00 mmol) 2-(Trifluormethyl)-pyridin-4-ol zu und erwärmte für 30 h auf 150°C, kühlte auf Raumtemperatur ab, gab Wasser und Essigester/Heptan (1:1) zur Reaktionslösung und rührte einige Minuten nach. Die Phasen wurden getrennt und die organische Phase mehrfach mit Wasser und zuletzt mit gesättigter Kochsalzlösung gewaschen und anschießend über Natriumsulfat getrocknet, filtriert und eingeengt. HPLC des Rohproduktes ergab 3,5-Bis-(2-Trifluormethylpyridin-4-yloxy)-benzonitril in Form weißer Kristalle.
   Ausbeute: 0.153 g (9 % d.Th.); 1 H NMR (CDCl₃/TMS): δ (ppm) = 7.08 (dd, 2H, pyridin C-H), 7.13 (t, 1H, phenyl C-H), 7.30 (d, 2H, pyridin C-H), 7.34 (d, 2H, phenyl C-H), 8.70 (d, 2H, pyridin C-H).
4. 3,5-Bis-(1-Methyl-3-trifluormethylpyrazol-5-yloxy)-phenyl-1-carboxamid 500 mg (1.16 mmol) 3,5-Bis-(1-Methyl-3-trifluormethylpyrazol-5-yloxy)-benzonitril wurden unter Stickstoffatmosphäre in 1.5 ml Dioxan vorgelegt und bei Raumtemperatur mit 64 mg (0.5 mmol) Kaliumcarbonat versetzt. Anschließend gab man bei 10 - 15 °C 0.5 mL 30%ige Wasserstoffperoxidlösung in Wasser und rührte 1.5 h bei Raumtemperatur ab. Zur Aufarbeitung gab man 10 mL Wasser hinzu und filtrierte den ausgefallenen Niederschlag ab. Trocknen des Niederschlages ergab das 3,5-Bis-(1-Methyl-3-trifluormethylpyrazol-5-yloxy)-phenyl-1-carboxamid in Form weißer Kristalle.
   Ausbeute: 532 mg (97 % d.Th.); Schmelzpunkt: 203°C.
5. 3-(1-Methyl-3-trifluormethylpyrazol-5-yloxy)-5-(3-trifluormethylpyrazol-1-yl)benzonitril 0.131 g (0.96 mmol) 3-Trifluormethylpyrazol wurden unter Stickstoffatmosphäre in 5 ml Dimethylacetamid vorgelegt und bei 0°C mit 0.033 g (1.1 mmol) Natriumhydrid (80%ig) versetzt. Man läßt auf Raumtemperatur kommen und setzt 0.250 g (0.88 mmol) g 3-Fluor-5-(1-Methyl-3-trifluormethylpyrazol-5-yloxy)-benzonitril zu und erwärmte für 8 h auf 140°C, kühlte auf Raumtemperatur ab und gab Wasser zur Reaktionslösung und rührte einige Minuten nach. Man extrahierte zweimal mit Heptan/Essigester (1:1) und zweimal mit Essigester. Die vereinigten Phasen wurden mit Wasser gewaschen und anschießend über Magnesiumsulfat getrocknet, filtriert und eingeengt. Säulenchromatographie des Rohproduktes ergaben 0.240 g 3-(1-Methyl-3-trifluormethylpyrazol-5-yloxy)-5-(3-trifluormethylpyrazol-1-yl)benzonitril in Form weißer Kristalle mit einem Schmelzpunkt von 116 - 117°C.
6. 5-Carbonitril-3-(1-Methyl-3-trifluormethylpyrazol-5-yloxy)benzonitril 2.00 g (13.7 mmol) 5-Carbonitril-3-fluorbenzonitril wurden unter Stickstoffatmosphäre in 25 ml N,N-Dimethylformamid vorgelegt und bei Raumtemperatur portionsweise mit 2.27 g (16.4 mmol) Kaliumcarbonat versetzt. Anschließend gab man 2.50 g (15.1 mmol) 1-Methyl-3-(trifluormethyl)-2-pyrazol-2-on zu und erwärmte für 2 h auf 150°C, kühlte auf Raumtemperatur ab, gab Wasser und Essigester zur Reaktionslösung und rührte einige Minuten nach. Die Phasen wurden getrennt und die organische Phase mehrfach mit Wasser und mit Kochsalzlösung gewaschen und anschießend über Magnesiumsulfat getrocknet, filtriert und eingeengt. Die Säulenchromatographie des Rohproduktes ergab 5-Carbonitril-3-(1-Methyl-3-trifluormethylpyrazol-5-yloxy)benzonitril in Form weißer Kristalle.
   Ausbeute: 2,49 g (62 % d.Th.); 1 H NMR (CDCl₃/TMS): δ (ppm) = 3.80 (s, 3H, methyl-H), 6.54 (s, 1 H, pyrazolyl C-H), 8.20 (d, 2H, phenyl C-H), 8.38 (t, 1 H, phenyl C-H).

Die in der nachfolgenden Tabelle 1 aufgeführten Verbindungen der Formeln (Ia), (Ib), (Ic) und (Id) können analog zu den oben genannten Beispielen erhalten werden. Die Verbindungen der Formeln (Ia), (Ib), (Ic) und (Id) sind Verbindungen der Formel (I), die sich im Rest A-X wie nachfolgend angegeben unterscheiden:

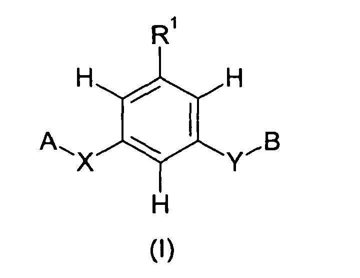

| Verbindung der Formel | (Ia) | (Ic) | (Id) |
|---|---|---|---|
| A-X | 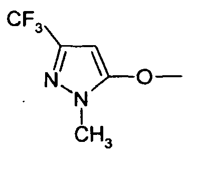 | 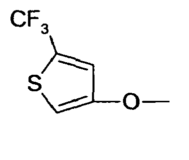 | 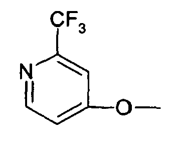 |

In der Tabelle 1 werden folgende Abkürzungen verwendet: Me = Methyl, Et = Ethyl, nPr = n-Propyl, iPr = iso-Propyl, cPr = cyclo-Propyl, nBu = n-Butyl, iBu = iso-Butyl, cBu = cyclo-Butyl, tBu = tertiär-Butyl, cPentyl = cyclo-Pentyl, cHexyl = cyclo-Hexyl, Ph = Phenyl, Bn = Benzyl.

Soweit in der Tabelle 1 Y = O-CH₂ ist, ist der Rest B an die CH₂-Gruppe gebunden.

**Tabelle 1:**

| | | B | Y | R¹ | Schmelzpunkt [°C] | | |
|---|---|---|---|---|---|---|---|
| | | | | | a | c | d |
| 1. | a-d | Phenyl | O | CN | | | |
| 2. | a-d | Napht-1-yl | O | CN | | | |
| 3. | a-d | Napht-2-yl | O | CN | | | |
| 4. | a-d | Pyridin-2-yl | O | CN | | | |
| 5. | a-d | Pyridin-3-yl | O | CN | | | |
| 6. | a-d | Pyridin-4-yl | O | CN | | | |
| 7. | a-d | 2-F-Phenyl | O | CN | | | |
| 8. | a-d | 3-F-Phenyl | O | CN | farbloses Harz | | |
| 9. | a-d | 4-F-Phenyl | O | CN | | | |
| 10. | a-d | 2,3-F₂-Phenyl | O | CN | | | |
| 11. | a-d | 2,4-F₂-Phenyl | O | CN | | | |
| 12. | a-d | 2,5-F₂-Phenyl | O | CN | | | |
| 13. | a-d | 2,6-F₂-Phenyl | O | CN | | | |
| 14. | a-d | 3,4-F₂-Phenyl | O | CN | gelbes Harz | | |
| 15. | a-d | 3,5-F₂-Phenyl | O | CN | gelbes Harz | | |
| 16. | a-d | 2,4,6-F₃-Phenyl | O | CN | | | |
| 17. | a-d | 2,3,4-F₃-Phenyl | O | CN | | | |
| 18. | a-d | 2-Cl-Phenyl | O | CN | | | |
| 19. | a-d | 3-Cl-Phenyl | O | CN | | | |
| 20. | a-d | 4-Cl-Phenyl | O | CN | | | |
| 21. | a-d | 2,3-Cl₂-Phenyl | O | CN | | | |
| 22. | a-d | 2,4- Cl₂-Phenyl | O | CN | | | |
| 23. | a-d | 2,5- Cl₂-Phenyl | O | CN | | | |
| 24. | a-d | 2,6-Cl₂-Phenyl | O | CN | | | |
| 25. | a-d | 3,4- Cl₂-Phenyl | O | CN | gelbes Harz | | |
| 26. | a-d | 3,5-Cl₂-Phenyl | O | CN | | | |
| 27. | a-d | 2,4,6-Cl₃-Phenyl | O | CN | | | |
| 28. | a-d | 2,3,4-Cl₃-Phenyl | O | CN | | | |
| 29. | a-d | 3,4,5- Cl₃-Phenyl | O | CN | | | |
| 30. | a-d | 2-F-4-Cl-Phenyl | O | CN | gelbes Harz | | |
| 31. | a-d | 2-Cl-4-F-Phenyl | O | CN | | | |
| 32. | a-d | 2-F-3-Cl-Phenyl | O | CN | | | |
| 33. | a-d | 2-Cl-3-F-Phenyl | O | CN | | | |
| 34. | a-d | 2-F-5-Cl-Phenyl | O | CN | | | |
| 35. | a-d | 2-Cl-5-F-Phenyl | O | CN | | | |
| 36. | a-d | 2-Cl-6-F-Phenyl | O | CN | | | |
| 37. | a-d | 2-Br-Phenyl | O | CN | | | |
| 38. | a-d | 3-Br-Phenyl | O | CN | | | |
| 39. | a-d | 4-Br-Phenyl | O | CN | | | |
| 40. | a-d | 2,3-Br₂-Phenyl | O | CN | | | |
| 41. | a-d | 2,4-Br₂-Phenyl | O | CN | | | |
| 42. | a-d | 2,5-Br₂-Phenyl | O | CN | | | |
| 43. | a-d | 2-I-Phenyl | O | CN | | | |
| 44. | a-d | 3-I-Phenyl | O | CN | | | |
| 45. | a-d | 4-I-Phenyl | O | CN | | | |
| 46. | a-d | 2-F-4-MeO-Phenyl | O | CN | | | |
| 47. | a-d | 2-F-5-MeO-Phenyl | O | CN | | | |
| 48. | a-d | 2-MeO-Phenyl | O | CN | | | |
| 49. | a-d | 3-MeO-Phenyl | O | CN | | | |
| 50. | a-d | 4-MeO-Phenyl | O | CN | | | |
| 51. | a-d | 2,4-(MeO)₂-Phenyl | O | CN | | | |
| 52. | a-d | 2,3-(MeO)₂-Phenyl | O | CN | | | |
| 53. | a-d | 2,5-(MeO)₂-Phenyl | O | CN | | | |
| 54. | a-d | 2-Me-Phenyl | O | CN | | | |
| 55. | a-d | 3-Me-Phenyl | O | CN | | | |
| 56. | a-d | 4-Me-Phenyl | O | CN | | | |
| 57. | a-d | 2,4-(Me)₂-Phenyl | O | CN | | | |
| 58. | a-d | 2,3-(Me)₂-Phenyl | O | CN | | | |
| 59. | a-d | 2,5-(Me)₂-Phenyl | O | CN | | | |
| 60. | a-d | 2,6-(Me)₂-Phenyl | O | CN | | | |
| 61. | a-d | 2-CF₃-Phenyl | O | CN | | | |
| 62. | a-d | 3-CF₃-Phenyl | O | CN | gelbes Wachs | | |
| 63. | a-d | 4-CF₃-Phenyl | O | CN | | | |
| 64. | a-d | 2,4-(CF₃)₂-Phenyl | O | CN | | | |
| 65. | a-d | 2,6-Cl₂-4-(CF₃)₂-Phenyl | O | CN | | | |
| 66. | a-d | 2-CF₃O-Phenyl | O | CN | | | |
| 67. | a-d | 3-CF₃O-Phenyl | O | CN | | | |
| 68. | a-d | 4-CF₃O-Phenyl | O | CN | | | |
| 69. | a-d | 5-F-Pyridin-2-yl | O | CN | | | |
| 70. | a-d | 5-Cl-Pyridin-2-yl | O | CN | | | |
| 71. | a-d | 5-F-Pyridin-4-yl | O | CN | | | |
| 72. | a-d | 5-Cl-Pyridin-4-yl | O | CN | | | |
| 73. | a-d | 2-CF₃-Pyridin-4-yl | O | CN | | | siehe Bsp. 3 |
| 74. | a-d | 2-CF₃-Thiophen-4-yl | O | CN | | braunes Wachs | |
| 75. | a-d | 1-CH₃-5-CF₃-pyrazol-3-yl | O | CN | gelbes Öl | | |
| 76. | a-d | 1-CH₃-3-CF₃-pyrazol-5-yl | O | CN | siehe Bsp. 1 | gelbes Harz | gelbes Öl |
| 77. | a-d | 1-CH₃-3-CF₃-pyrazol-5-yl | O | CONH₂ | siehe Bsp. 4 | | |
| 78. | a-d | 2-CF₃-Thiadiazol-5-yl | O | CN | | | |
| 79. | a-d | 2-CN-Phenyl | O | CN | | | |
| 80. | a-d | 3-CN-Phenyl | O | CN | 121 - 122 | | |
| 81. | a-d | 4-CN-Phenyl | O | CN | | | |
| 82. | a-d | 3,5-(CN)₂-Phenyl | O | CN | | | |
| 83. | a-d | 2-CN-4-F-Phenyl | O | CN | | | |
| 84. | a-d | 4-CN-2-F-Phenyl | O | CN | | | |
| 85. | a-d | 2-CF₃-Oxadiazol-5-yl | O | CN | | | |
| 86. | a-d | Phenyl | O | Me | | | |
| 87. | a-d | Napht-1-yl | O | Me | | | |
| 88. | a-d | Pyridin-2-yl | O | Me | | | |
| 89. | a-d | Pyridin-3-yl | O | Me | | | |
| 90. | a-d | Pyridin-4-yl | O | Me | | | |
| 91. | a-d | 2-F-Phenyl | O | Me | | | |
| 92. | a-d | 3-F-Phenyl | O | Me | | | |
| 93. | a-d | 4-F-Phenyl | O | Me | | | |
| 94. | a-d | 2,3-F₂-Phenyl | O | Me | | | |
| 95. | a-d | 2,4-F₂-Phenyl | O | Me | | | |
| 96. | a-d | 2,5-F₂-Phenyl | O | Me | | | |
| 97. | a-d | 2,6-F₂-Phenyl | O | Me | | | |
| 98. | a-d | 3,4-F₂-Phenyl | O | Me | | | |
| 99. | a-d | 3,5-F₂-Phenyl | O | Me | | | |
| 100. | a-d | 2,4,6-F₃-Phenyl | O | Me | | | |
| 101. | a-d | 2,3,4-F₃-Phenyl | O | Me | | | |
| 102. | a-d | 3,4,5-F₃-Phenyl | O | Me | | | |
| 103. | a-d | 2-Cl-Phenyl | O | Me | | | |
| 104. | a-d | 3- Cl-Phenyl | O | Me | | | |
| 105. | a-d | 4-Cl-Phenyl | O | Me | | | |
| 106. | a-d | 2,3-Cl₂-Phenyl | O | Me | | | |
| 107. | a-d | 2,4-Cl₂-Phenyl | O | Me | | | |
| 108. | a-d | 2,5-Cl₂-Phenyl | O | Me | | | |
| 109. | a-d | 2,6- Cl₂-Phenyl | O | Me | | | |
| 110. | a-d | 3,4- Cl₂-Phenyl | O | Me | | | |
| 111. | a-d | 3,5- Cl₂-Phenyl | O | Me | | | |
| 112. | a-d | 2,4,6-Cl₃-Phenyl | O | Me | | | |
| 113. | a-d | 2,3,4-Cl₃-Phenyl | O | Me | | | |
| 114. | a-d | 2,3,6-Cl₃-Phenyl | O | Me | | | |
| 115. | a-d | 2-F-4-Cl-Phenyl | O | Me | | | |
| 116. | a-d | 2-Cl-4-F-Phenyl | O | Me | | | |
| 117. | a-d | 2-F-3-Cl-Phenyl | O | Me | | | |
| 118. | a-d | 2-Cl-3-F-Phenyl | O | Me | | | |
| 119. | a-d | 2-F-5-Cl-Phenyl | O | Me | | | |
| 120. | a-d | 2-Cl-5-F-Phenyl | O | Me | | | |
| 121. | a-d | 2-Cl-6-F-Phenyl | O | Me | | | |
| 122. | a-d | 2-Br-Phenyl | O | Me | | | |
| 123. | a-d | 3-Br-Phenyl | O | Me | | | |
| 124. | a-d | 4-Br-Phenyl | O | Me | | | |
| 125. | a-d | 2,3-Br₂-Phenyl | O | Me | | | |
| 126. | a-d | 2,4-Br₂-Phenyl | O | Me | | | |
| 127. | a-d | 2,5-Br₂-Phenyl | O | Me | | | |
| 128. | a-d | 2-I-Phenyl | O | Me | | | |
| 129. | a-d | 3-I-Phenyl | O | Me | | | |
| 130. | a-d | 4-I-Phenyl | O | Me | | | |
| 131. | a-d | 2-F-4-MeO-Phenyl | O | Me | | | |
| 132. | a-d | 2-F-5-MeO-Phenyl | O | Me | | | |
| 133. | a-d | 2-MeO-Phenyl | O | Me | | | |
| 134. | a-d | 3-MeO-Phenyl | O | Me | | | |
| 135. | a-d | 4-MeO-Phenyl | O | Me | | | |
| 136. | a-d | 2,4-(MeO)₂-Phenyl | O | Me | | | |
| 137. | a-d | 2,3-(MeO)₂-Phenyl | O | Me | | | |
| 138. | a-d | 2,5-(MeO)₂-Phenyl | O | Me | | | |
| 139. | a-d | 2-Me-Phenyl | O | Me | | | |
| 140. | a-d | 3-Me-Phenyl | O | Me | | | |
| 141. | a-d | 4-Me-Phenyl | O | Me | | | |
| 142. | a-d | 2,4-(Me)₂-Phenyl | O | Me | | | |
| 143. | a-d | 2,3-(Me)₂-Phenyl | O | Me | | | |
| 144. | a-d | 2,5-(Me)₂-Phenyl | O | Me | | | |
| 145. | a-d | 2-CF₃-Phenyl | O | Me | | | |
| 146. | a-d | 3-CF₃-Phenyl | O | Me | | | |
| 147. | a-d | 4-CF₃-Phenyl | O | Me | | | |
| 148. | a-d | 2,4-(CF₃)₂-Phenyl | O | Me | | | |
| 149. | a-d | 296-Cl₂-4-(CF₃)₂-Phenyl | O | Me | | | |
| 150. | a-d | 2-CF₃O-Phenyl | O | Me | | | |
| 151. | a-d | 3-CF₃O-Phenyl | O | Me | | | |
| 152. | a-d | 4-CF₃O-Phenyl | O | Me | | | |
| 153. | a-d | 5-F-Pyridin-2-yl | O | Me | | | |
| 154. | a-d | 5-Cl-Pyridin-2-yl | O | Me | | | |
| 155. | a-d | 5-F-Pyridin-4-yl | O | Me | | | |
| 156. | a-d | 5-Cl-Pyridin-4-yl | O | Me | | | |
| 157. | a-d | 2-CF₃-Pyridin-4-yl | O | Me | | | |
| 158. | a-d | 1-CH₃-5-CF₃-pyrazol-3-yl | O | Me | | | |
| 159. | a-d | 1-CH₃-3-CF₃-pyrazol-5-yl | O | Me | | | |
| 160. | a-d | 2-CF₃-Thiophen-4-yl | O | Me | | | |
| 161. | a-d | 2-CF₃-Thiadiazol-5-yl | O | Me | | | |
| 162. | a-d | 2-CN-Phenyl | O | Me | | | |
| 163. | a-d | 3-CN-Phenyl | O | Me | | | |
| 164. | a-d | 4-CN-Phenyl | O | Me | | | |
| 165. | a-d | 3,5-(CN)₂-Phenyl | O | Me | | | |
| 166. | a-d | 2-CN-4-F-Phenyl | O | Me | | | |
| 167. | a-d | 4-CN-2-F-Phenyl | O | Me | | | |
| 168. | a-d | 2-CF₃-Oxadiazol-5-yl | O | Me | | | |
| 169. | a-d | Phenyl | O | MeO | | | |
| 170. | a-d | Napht-1-yl | O | MeO | | | |
| 171. | a-d | Pyridin-2-yl | O | MeO | | | |
| 172. | a-d | Pyridin-3-yl | O | MeO | | | |
| 173. | a-d | Pyridin-4-yl | O | MeO | | | |
| 174. | a-d | 2-F-Phenyl | O | MeO | | | |
| 175. | a-d | 3-F-Phenyl | O | MeO | | | |
| 176. | a-d | 4-F-Phenyl | O | MeO | | | |
| 177. | a-d | 2,3-F₂-Phenyl | O | MeO | | | |
| 178. | a-d | 2,4-F₂-Phenyl | O | MeO | | | |
| 179. | a-d | 2,5-F₂-Phenyl | O | MeO | | | |
| 180. | a-d | 2,6-F₂-Phenyl | O | MeO | | | |
| 181. | a-d | 3,4-F₂-Phenyl | O | MeO | | | |
| 182. | a-d | 3,5-F₂-Phenyl | O | MeO | | | |
| 183. | a-d | 2,4,6-F₃-Phenyl | O | MeO | | | |
| 184. | a-d | 2,3,4-F₃-Phenyl | O | MeO | | | |
| 185. | a-d | 3,4,5-F₃-Phenyl | O | MeO | | | |
| 186. | a-d | 2-Cl-Phenyl | O | MeO | | | |
| 187. | a-d | 3- Cl-Phenyl | O | MeO | | | |
| 188. | a-d | 4-Cl-Phenyl | O | MeO | | | |
| 189. | a-d | 2,3-Cl₂-Phenyl | O | MeO | | | |
| 190. | a-d | 2,4- Cl₂-Phenyl | O | MeO | | | |
| 191. | a-d | 2,5- Cl₂-Phenyl | O | MeO | | | |
| 192. | a-d | 2,6- Cl₂-Phenyl | O | MeO | | | |
| 193. | a-d | 3,4- Cl₂-Phenyl | O | MeO | | | |
| 194. | a-d | 3,5- Cl₂-Phenyl | O | MeO | | | |
| 195. | a-d | 2,4,6- Cl₃-Phenyl | O | MeO | | | |
| 196. | a-d | 2,3,4- Cl₃-Phenyl | O | MeO | | | |
| 197. | a-d | 2-F-4-Cl-Phenyl | O | MeO | | | |
| 198. | a-d | 2-Cl-4-F-Phenyl | O | MeO | | | |
| 199. | a-d | 2-F-3-Cl-Phenyl | O | MeO | | | |
| 200. | a-d | 2-Cl-3-F-Phenyl | O | MeO | | | |
| 201. | a-d | 2-F-5-Cl-Phenyl | O | MeO | | | |
| 202. | a-d | 2-Cl-5-F-Phenyl | O | MeO | | | |
| 203. | a-d | 2-Cl-6-F-Phenyl | O | MeO | | | |
| 204. | a-d | 2-Br-Phenyl | O | MeO | | | |
| 205. | a-d | 3-Br-Phenyl | O | MeO | | | |
| 206. | a-d | 4-Br-Phenyl | O | MeO | | | |
| 207. | a-d | 2,4-Br₂-Phenyl | O | MeO | | | |
| 208. | a-d | 2,5-Br₂-Phenyl | O | MeO | | | |
| 209. | a-d | 2-I-Phenyl | O | MeO | | | |
| 210. | a-d | 3-I-Phenyl | O | MeO | | | |
| 211. | a-d | 4-I-Phenyl | O | MeO | | | |
| 212. | a-d | 2-F-4-MeO-Phenyl | O | MeO | | | |
| 213. | a-d | 2-F-5-MeO-Phenyl | O | MeO | | | |
| 214. | a-d | 2-MeO-Phenyl | O | MeO | | | |
| 215. | a-d | 3-MeO-Phenyl | O | MeO | | | |
| 216. | a-d | 4-MeO-Phenyl | O | MeO | | | |
| 217. | a-d | 2,4-(MeO)₂-Phenyl | O | MeO | | | |
| 218. | a-d | 3,4-(MeO)₂-Phenyl | O | MeO | | | |
| 219. | a-d | 2-Me-Phenyl | O | MeO | | | |
| 220. | a-d | 3-Me-Phenyl | O | MeO | | | |
| 221. | a-d | 4-Me-Phenyl | O | MeO | | | |
| 222. | a-d | 2,4-(Me)₂-Phenyl | O | MeO | | | |
| 223. | a-d | 2-CF₃-Phenyl | O | MeO | | | |
| 224. | a-d | 3-CF₃-Phenyl | O | MeO | | | |
| 225. | a-d | 4-CF₃-Phenyl | O | MeO | | | |
| 226. | a-d | 2,4-(CF₃)₂-Phenyl | O | MeO | | | |
| 227. | a-d | 2,6-Cl₂-4-(CF₃)₂-Phenyl | O | MeO | | | |
| 228. | a-d | 2-CF₃O-Phenyl | O | MeO | | | |
| 229. | a-d | 3-CF₃O-Phenyl | O | MeO | | | |
| 230. | a-d | 4-CF₃O-Phenyl | O | MeO | | | |
| 231. | a-d | 5-F-Pyridin-2-yl | O | MeO | | | |
| 232. | a-d | 5-Cl-Pyridin-2-yl | O | MeO | | | |
| 233. | a-d | 5-F-Pyridin-4-yl | O | MeO | | | |
| 234. | a-d | 5-Cl-Pyridin-4-yl | O | MeO | | | |
| 235. | a-d | 2-CF₃-Pyridin-4-yl | O | MeO | | | |
| 236. | a-d | 2-CF₃-Thiophen-4-yl | O | MeO | | | |
| 237. | a-d | 1-CH₃-5-CF₃-pyrazol-3-yl | O | MeO | | | |
| 238. | a-d | 1-CH₃-3-CF₃-pyrazol-5-yl | O | MeO | | | |
| 239. | a-d | 2-CF₃-Thiadiazol-5-yl | O | MeO | | | |
| 240. | a-d | 2-CN-Phenyl | O | MeO | | | |
| 241. | a-d | 3-CN-Phenyl | O | MeO | | | |
| 242. | a-d | 4-CN-Phenyl | O | MeO | | | |
| 243. | a-d | 3,5-(CN)₂-Phenyl | O | MeO | | | |
| 244. | a-d | 2-CN-4-F-Phenyl | O | MeO | | | |
| 245. | a-d | 4-CN-2-F-Phenyl | O | MeO | | | |
| 246. | a-d | 2-CF₃-Oxadiazol-5-yl | O | MeO | | | |
| 247. | a-d | Phenyl | O | CHO | | | |
| 248. | a-d | Napht-1-yl | O | CHO | | | |
| 249. | a-d | Pyridin-2-yl | O | CHO | | | |
| 250. | a-d | Pyridin-3-yl | O | CHO | | | |
| 251. | a-d | Pyridin-4-yl | O | CHO | | | |
| 252. | a-d | 2-F-Phenyl | O | CHO | | | |
| 253. | a-d | 3-F-Phenyl | O | CHO | | | |
| 254. | a-d | 4-F-Phenyl | O | CHO | | | |
| 255. | a-d | 2,3-F₂-Phenyl | O | CHO | | | |
| 256. | a-d | 2,4-F₂-Phenyl | O | CHO | | | |
| 257. | a-d | 2,5-F₂-Phenyl | O | CHO | | | |
| 258. | a-d | 2,6-F₂-Phenyl | O | CHO | | | |
| 259. | a-d | 3,4-F₂-Phenyl | O | CHO | | | |
| 260. | a-d | 3,5-F₂-Phenyl | O | CHO | | | |
| 261. | a-d | 2,4,6-F₃-Phenyl | O | CHO | | | |
| 262. | a-d | 2,3,4-F₃-Phenyl | O | CHO | | | |
| 263. | a-d | 2-Cl-Phenyl | O | CHO | | | |
| 264. | a-d | 3-Cl-Phenyl | O | CHO | | | |
| 265. | a-d | 4- Cl-Phenyl | O | CHO | | | |
| 266. | a-d | 2,3-Cl₂-Phenyl | O | CHO | | | |
| 267. | a-d | 2,4- Cl₂-Phenyl | O | CHO | | | |
| 268. | a-d | 2,5- Cl₂-Phenyl | O | CHO | | | |
| 269. | a-d | 2,6- Cl₂-Phenyl | O | CHO | | | |
| 270. | a-d | 3,4- Cl₂-Phenyl | O | CHO | | | |
| 271. | a-d | 3,5-Cl₂-Phenyl | O | CHO | | | |
| 272. | a-d | 2,3,4-Cl₃-Phenyl | O | CHO | | | |
| 273. | a-d | 2-F-4-Cl-Phenyl | O | CHO | | | |
| 274. | a-d | 2-Cl-4-F-Phenyl | O | CHO | | | |
| 275. | a-d | 2-F-3-Cl-Phenyl | O | CHO | | | |
| 276. | a-d | 2-Cl-3-F-Phenyl | O | CHO | | | |
| 277. | a-d | 2-F-5-Cl-Phenyl | O | CHO | | | |
| 278. | a-d | 2-Cl-5-F-Phenyl | O | CHO | | | |
| 279. | a-d | 2-Cl-6-F-Phenyl | O | CHO | | | |
| 280. | a-d | 2-Br-Phenyl | O | CHO | | | |
| 281. | a-d | 3-Br-Phenyl | O | CHO | | | |
| 282. | a-d | 4-Br-Phenyl | O | CHO | | | |
| 283. | a-d | 2,3-Br₂-Phenyl | O | CHO | | | |
| 284. | a-d | 2,5-Br₂-Phenyl | O | CHO | | | |
| 285. | a-d | 2-I-Phenyl | O | CHO | | | |
| 286. | a-d | 3-I-Phenyl | O | CHO | | | |
| 287. | a-d | 4-I-Phenyl | O | CHO | | | |
| 288. | a-d | 2-F-4-MeO-Phenyl | O | CHO | | | |
| 289. | a-d | 2-F-5-MeO-Phenyl | O | CHO | | | |
| 290. | a-d | 2-MeO-Phenyl | O | CHO | | | |
| 291. | a-d | 3-MeO-Phenyl | O | CHO | | | |
| 292. | a-d | 4-MeO-Phenyl | O | CHO | | | |
| 293. | a-d | 2,4-(MeO)₂-Phenyl | O | CHO | | | |
| 294. | a-d | 2,3-(MeO)₂-Phenyl | O | CHO | | | |
| 295. | a-d | 2-Me-Phenyl | O | CHO | | | |
| 296. | a-d | 3-Me-Phenyl | O | CHO | | | |
| 297. | a-d | 4-Me-Phenyl | O | CHO | | | |
| 298. | a-d | 2,4-(Me)₂-Phenyl | O | CHO | | | |
| 299. | a-d | 2,3-(Me)₂-Phenyl | O | CHO | | | |
| 300. | a-d | 2,5-(Me)₂-Phenyl | O | CHO | | | |
| 301. | a-d | 2-CF₃-Phenyl | O | CHO | | | |
| 302. | a-d | 3-CF₃-Phenyl | O | CHO | | | |
| 303. | a-d | 4-CF₃-Phenyl | O | CHO | | | |
| 304. | a-d | 2,4-(CF₃)₂-Phenyl | O | CHO | | | |
| 305. | a-d | 2,6-Cl₂-4-(CF₃)₂-Phenyl | 0 | CHO | | | |
| 306. | a-d | 2-CF₃O-Phenyl | O | CHO | | | |
| 307. | a-d | 3-CF₃O-Phenyl | O | CHO | | | |
| 308. | a-d | 4-CF₃O-Phenyl | O | CHO | | | |
| 309. | a-d | 5-F-Pyridin-2-yl | O | CHO | | | |
| 310. | a-d | 5-Cl-Pyridin-2-yl | O | CHO | | | |
| 311. | a-d | 5-F-Pyridin-4-yl | O | CHO | | | |
| 312. | a-d | 5-Cl-Pyridin-4-yl | O | CHO | | | |
| 313. | a-d | 2-CF₃-Pyridin-4-yl | O | CHO | | | |
| 314. | a-d | 2-CF₃-Thiophen-4-yl | O | CHO | | | |
| 315. | a-d | 1-CH₃-5-CF₃-pyrazol-3-yl | O | CHO | | | |
| 316. | a-d | 1-CH₃-3-CF₃-pyrazol-5-yl | O | CHO | | | |
| 317. | a-d | 2-CF₃-Thiadiazol-5-yl | O | CHO | | | |
| 318. | a-d | 2-CN-Phenyl | O | CHO | | | |
| 319. | a-d | 3-CN-Phenyl | O | CHO | | | |
| 320. | a-d | 4-CN-Phenyl | O | CHO | | | |
| 321. | a-d | 3,5-(CN)₂-Phenyl | O | CHO | | | |
| 322. | a-d | 2-CN-4-F-Phenyl | O | CHO | | | |
| 323. | a-d | 4-CN-2-F-Phenyl | O | CHO | | | |
| 324. | a-d | 2-CF₃-Oxadiazol-5-yl | O | CHO | | | |
| 325. | a-d | Phenyl | O | NO₂ | | | |
| 326. | a-d | Napht-1-yl | O | NO₂ | | | |
| 327. | a-d | Pyridin-2-yl | O | NO₂ | | | |
| 328. | a-d | Pyridin-3-yl | O | NO₂ | | | |
| 329. | a-d | Pyridin-4-yl | O | NO₂ | | | |
| 330. | a-d | 2-F-Phenyl | O | NO₂ | | | |
| 331. | a-d | 3-F-Phenyl | O | NO₂ | | | |
| 332. | a-d | 4-F-Phenyl | O | NO₂ | | | |
| 333. | a-d | 2,3-F₂-Phenyl | O | NO₂ | | | |
| 334. | a-d | 2,4-F₂-Phenyl | O | NO₂ | | | |
| 335. | a-d | 2,5-F₂-Phenyl | O | NO₂ | | | |
| 336. | a-d | 2,6-F₂-Phenyl | O | NO₂ | | | |
| 337. | a-d | 3,4-F₂-Phenyl | O | NO₂ | | | |
| 338. | a-d | 3,5-F₂-Phenyl | O | NO₂ | | | |
| 339. | a-d | 2,4,6-F₃-Phenyl | O | NO₂ | | | |
| 340. | a-d | 2,3,5-F₃-Phenyl | O | NO₂ | | | |
| 341. | a-d | 3,4,5-F₃-Phenyl | O | NO₂ | | | |
| 342. | a-d | 2-Cl-Phenyl | O | NO₂ | | | |
| 343. | a-d | 3-Cl-Phenyl | O | NO₂ | | | |
| 344. | a-d | 4-Cl-Phenyl | O | NO₂ | | | |
| 345. | a-d | 2,3-Cl₂-Phenyl | O | NO₂ | | | |
| 346. | a-d | 2,4- Cl₂-Phenyl | O | NO₂ | | | |
| 347. | a-d | 2,5- Cl₂-Phenyl | O | NO₂ | | | |
| 348. | a-d | 2,6-Cl₂-Phenyl | O | NO₂ | | | |
| 349. | a-d | 3,4-Cl₂-Phenyl | O | NO₂ | | | |
| 350. | a-d | 3,5- Cl₂-Phenyl | O | NO₂ | | | |
| 351. | a-d | 2,4,6-Cl₃-Phenyl | O | NO₂ | | | |
| 352. | a-d | 2,3,4-Cl₃-Phenyl | O | NO₂ | | | |
| 353. | a-d | 3,4,5-Cl₃-Phenyl | O | NO₂ | | | |
| 354. | a-d | 2-F-4-Cl-Phenyl | O | NO₂ | | | |
| 355. | a-d | 2-Cl-4-F-Phenyl | O | NO₂ | | | |
| 356. | a-d | 2-F-3-Cl-Phenyl | O | NO₂ | | | |
| 357. | a-d | 2-Cl-3-F-Phenyl | O | NO₂ | | | |
| 358. | a-d | 2-F-5-Cl-Phenyl | O | NO₂ | | | |
| 359. | a-d | 2-Cl-5-F-Phenyl | O | NO₂ | | | |
| 360. | a-d | 2-Cl-6-F-Phenyl | O | NO₂ | | | |
| 361. | a-d | 2-Br-Phenyl | O | NO₂ | | | |
| 362. | a-d | 3-Br-Phenyl | O | NO₂ | | | |
| 363. | a-d | 4-Br-Phenyl | O | NO₂ | | | |
| 364. | a-d | 2,3-Br₂-Phenyl | O | NO₂ | | | |
| 365. | a-d | 2,4-Br₂-Phenyl | O | NO₂ | | | |
| 366. | a-d | 2,5-Br₂-Phenyl | O | NO₂ | | | |
| 367. | a-d | 2-I-Phenyl | O | NO₂ | | | |
| 368. | a-d | 3-I-Phenyl | O | NO₂ | | | |
| 369. | a-d | 4-I-Phenyl | O | NO₂ | | | |
| 370. | a-d | 2-F-4-MeO-Phenyl | O | NO₂ | | | |
| 371. | a-d | 2-F-5-MeO-Phenyl | O | NO₂ | | | |
| 372. | a-d | 2-MeO-Phenyl | O | NO₂ | | | |
| 373. | a-d | 3-MeO-Phenyl | O | NO₂ | | | |
| 374. | a-d | 4-MeO-Phenyl | O | NO₂ | | | |
| 375. | a-d | 2,4-(MeO)₂-Phenyl | O | N02 | | | |
| 376. | a-d | 2,5-(MeO)₂-Phenyl | O | NO₂ | | | |
| 377. | a-d | 2-Me-Phenyl | O | NO₂ | | | |
| 378. | a-d | 3-Me-Phenyl | O | NO₂ | | | |
| 379. | a-d | 4-Me-Phenyl | O | NO₂ | | | |
| 380. | a-d | 2,4-(Me)₂-Phenyl | O | NO₂ | | | |
| 381. | a-d | 2,5-(Me)₂-Phenyl | O | NO₂ | | | |
| 382. | a-d | 2-CF₃-Phenyl | O | NO₂ | | | |
| 383. | a-d | 3-CF₃-Phenyl | O | NO₂ | | | |
| 384. | a-d | 4-CF₃-Phenyl | O | NO₂ | | | |
| 385. | a-d | 2,4-(CF₃)₂-Phenyl | O | NO₂ | | | |
| 386. | a-d | 2,6-Cl₂-4-(CF₃)₂-Phenyl | O | NO₂ | | | |
| 387. | a-d | 2-CF₃O-Phenyl | O | NO₂ | | | |
| 388. | a-d | 3-CF₃O-Phenyl | O | NO₂ | | | |
| 389. | a-d | 4-CF₃O-Phenyl | O | NO₂ | | | |
| 390. | a-d | 5-F-Pyridin-2-yl | O | NO₂ | | | |
| 391. | a-d | 5-Cl-Pyridin-2-yl | O | NO₂ | | | |
| 392. | a-d | 5-F-Pyridin-4-yl | O | NO₂ | | | |
| 393. | a-d | 5-Cl-Pyridin-4-yl | O | NO₂ | | | |
| 394. | a-d | 2-CF₃-Pyridin-4-yl | O | NO₂ | | | |
| 395. | a-d | 2-CF₃-Thiophen-4-yl | O | NO₂ | | | |
| 396. | a-d | 1-CH₃-5-CF₃-pyrazol-3-yl | 0 | NO₂ | hellrotes Öl | | |
| 397. | a-d | 1-CH₃-3-CF₃-pyrazol-5-yl | O | NO₂ | 135 | | |
| 398. | a-d | 2-CF₃-Thiadiazol-5-yl | O | NO₂ | | | |
| 399. | a-d | 2-CN-Phenyl | O | NO₂ | | | |
| 400. | a-d | 3-CN-Phenyl | O | NO₂ | | | |
| 401. | a-d | 4-CN-Phenyl | O | NO₂ | | | |
| 402. | a-d | 3,5-(CN)₂-Phenyl | O | NO₂ | | | |
| 403. | a-d | 2-CN-4-F-Phenyl | O | NO₂ | | | |
| 404. | a-d | 4-CN-2-F-Phenyl | O | NO₂ | | | |
| 405. | a-d | 2-CF₃-Oxadiazol-5-yl | O | NO₂ | | | |
| 406. | a-d | NH-Phenyl | CO | CN | | | |
| 407. | a-d | NH-Napht-1-yl | CO | CN | | | |
| 408. | a-d | NH-Pyridin-2-yl | CO | CN | | | |
| 409. | a-d | NH-Pyridin-3-yl | CO | CN | | | |
| 410. | a-d | NH-Pyridin-4-yl | CO | CN | | | |
| 411. | a-d | NH-2-F-Phenyl | CO | CN | | | |
| 412. | a-d | NH-3-F-Phenyl | CO | CN | | | |
| 413. | a-d | NH-4-F-Phenyl | CO | CN | | | |
| 414. | a-d | NH-2,3-F₂-Phenyl | CO | CN | | | |
| 415. | a-d | NH-2,4-F₂-Phenyl | CO | CN | | | |
| 416. | a-d | NH-2,5-F₂-Phenyl | CO | CN | | | |
| 417. | a-d | NH-2,6-F₂-Phenyl | CO | CN | | | |
| 418. | a-d | NH-3,4-F₂-Phenyl | CO | CN | | | |
| 419. | a-d | NH-3,5-F₂-Phenyl | CO | CN | | | |
| 420. | a-d | NH-2,4,6-F₃-Phenyl | CO | CN | | | |
| 421. | a-d | NH-2,3,4-F₃-Phenyl | CO | CN | | | |
| 422. | a-d | NH-2-Cl-Phenyl | CO | CN | | | |
| 423. | a-d | NH-3-Cl-Phenyl | CO | CN | | | |
| 424. | a-d | NH-4-Cl-Phenyl | CO | CN | | | |
| 425. | a-d | NH-2,3-Cl₂-Phenyl | CO | CN | | | |
| 426. | a-d | NH-2,4-Cl₂-Phenyl | CO | CN | | | |
| 427. | a-d | NH-2,5-Cl₂-Phenyl | CO | CN | | | |
| 428. | a-d | NH-2,6-Cl₂-Phenyl | CO | CN | | | |
| 429. | a-d | NH-3,4-Cl₂-Phenyl | CO | CN | | | |
| 430. | a-d | NH-3,5-Cl₂-Phenyl | CO | CN | | | |
| 431. | a-d | NH-2,4,6-Cl₃-Phenyl | CO | CN | | | |
| 432. | a-d | NH-2,3,4-Cl₃-Phenyl | CO | CN | | | |
| 433. | a-d | NH-3,4,5-Cl₃-Phenyl | CO | CN | | | |
| 434. | a-d | NH-2-F-4-Cl-Phenyl | CO | CN | | | |
| 435. | a-d | NH-2-Cl-4-F-Phenyl | CO | CN | | | |
| 436. | a-d | NH-2-F-3-Cl-Phenyl | CO | CN | | | |
| 437. | a-d | NH-2-Cl-3-F-Phenyl | CO | CN | | | |
| 438. | a-d | NH-2-F-5-Cl-Phenyl | CO | CN | | | |
| 439. | a-d | NH-2-Cl-5-F-Phenyl | CO | CN | | | |
| 440. | a-d | NH-2-Cl-6-F-Phenyl | CO | CN | | | |
| 441. | a-d | NH-2-Br-Phenyl | CO | CN | | | |
| 442. | a-d | NH-3-Br-Phenyl | CO | CN | | | |
| 443. | a-d | NH-4-Br-Phenyl | CO | CN | | | |
| 444. | a-d | NH-2,3-Br₂-Phenyl | CO | CN | | | |
| 445. | a-d | NH-2,4-Br₂-Phenyl | CO | CN | | | |
| 446. | a-d | NH-2,5-Br₂-Phenyl | CO | CN | | | |
| 447. | a-d | NH-2-I-Phenyl | CO | CN | | | |
| 448. | a-d | NH-3-I-Phenyl | CO | CN | | | |
| 449. | a-d | NH-4-I-Phenyl | CO | CN | | | |
| 450. | a-d | NH-2-F-4-MeO-Phenyl | CO | CN | | | |
| 451. | a-d | NH-2-F-5-MeO-Phenyl | CO | CN | | | |
| 452. | a-d | NH-2-MeO-Phenyl | CO | CN | | | |
| 453. | a-d | NH-3-MeO-Phenyl | CO | CN | | | |
| 454. | a-d | NH-4-MeO-Phenyl | CO | CN | | | |
| 455. | a-d | NH-2,4-(MeO)₂-Phenyl | CO | CN | | | |
| 456. | a-d | NH-2,3-(MeO)₂-Phenyl | CO | CN | | | |
| 457. | a-d | NH-2,5-(MeO)₂-Phenyl | CO | CN | | | |
| 458. | a-d | NH-2-Me-Phenyl | CO | CN | | | |
| 459. | a-d | NH-3-Me-Phenyl | CO | CN | | | |
| 460. | a-d | NH-4-Me-Phenyl | CO | CN | | | |
| 461. | a-d | NH-2,4-(Me)₂-Phenyl | CO | CN | | | |
| 462. | a-d | NH-2,5-(Me)₂-Phenyl | CO | CN | | | |
| 463. | a-d | NH-2-CF₃-Phenyl | CO | CN | | | |
| 464. | a-d | NH-3-CF₃-Phenyl | CO | CN | | | |
| 465. | a-d | NH-4-CF₃-Phenyl | CO | CN | | | |
| 466. | a-d | NH-2,4-(CF₃)₂-Phenyl | CO | CN | | | |
| 467. | a-d | NH-2,6-Cl₂-4-(CF₃)₂-Phenyl | CO | CN | | | |
| 468. | a-d | NH-2-CF₃O-Phenyl | CO | CN | | | |
| 469. | a-d | NH-3-CF₃O-Phenyl | CO | CN | | | |
| 470. | a-d | NH-4-CF₃O-Phenyl | CO | CN | | | |
| 471. | a-d | NH-5-F-Pyridin-2-yl | CO | CN | | | |
| 472. | a-d | NH-5-Cl-Pyridin-2-yl | CO | CN | | | |
| 473. | a-d | NH-5-F-Pyridin-4-yl | CO | CN | | | |
| 474. | a-d | NH-5-Cl-Pyridin-4-yl | CO | CN | | | |
| 475. | a-d | NH-2-CN-Phenyl | CO | CN | | | |
| 476. | a-d | NH-3-CN-Phenyl | CO | CN | | | |
| 477. | a-d | NH-4-CN-Phenyl | CO | CN | | | |
| 478. | a-d | NH-3,5-(CN)₂-Phenyl | CO | CN | | | |
| 479. | a-d | NH-2-CN-4-F-Phenyl | CO | CN | | | |
| 480. | a-d | NH-4-CN-2-F-Phenyl | CO | CN | | | |
| 481. | a-d | NH-Phenyl | CO | Me | | | |
| 482. | a-d | NH-Napht-1-yl | CO | Me | | | |
| 483. | a-d | NH-Pyridin-2-yl | CO | Me | | | |
| 484. | a-d | NH-Pyridin-3-yl | CO | Me | | | |
| 485. | a-d | NH-Pyridin-4-yl | CO | Me | | | |
| 486. | a-d | NH-2-F-Phenyl | CO | Me | | | |
| 487. | a-d | NH-3-F-Phenyl | CO | Me | | | |
| 488. | a-d | NH-4-F-Phenyl | CO | Me | | | |
| 489. | a-d | NH-2,3-F₂-Phenyl | CO | Me | | | |
| 490. | a-d | NH-2,4-F₂-Phenyl | CO | Me | | | |
| 491. | a-d | NH-2,5-F₂-Phenyl | CO | Me | | | |
| 492. | a-d | NH-2,6-F₂-Phenyl | CO | Me | | | |
| 493. | a-d | NH-3,4-F₂-Phenyl | CO | Me | | | |
| 494. | a-d | NH-3,5-F₂-Phenyl | CO | Me | | | |
| 495. | a-d | NH-2,4,6-F₃-Phenyl | CO | Me | | | |
| 496. | a-d | NH-2,3,4-F₃-Phenyl | CO | Me | | | |
| 497. | a-d | NH-2-Cl-Phenyl | CO | Me | | | |
| 498. | a-d | NH-3-Cl-Phenyl | CO | Me | | | |
| 499. | a-d | NH-4-Cl-Phenyl | CO | Me | | | |
| 500. | a-d | NH-2,3-Cl₂-Phenyl | CO | Me | | | |
| 501. | a-d | NH-2,4- Cl₂-Phenyl | CO | Me | | | |
| 502. | a-d | NH-2,5- Cl₂-Phenyl | CO | Me | | | |
| 503. | a-d | NH-2,6- Cl₂-Phenyl | CO | Me | | | |
| 504. | a-d | NH-3,4- Cl₂-Phenyl | CO | Me | | | |
| 505. | a-d | NH-3,5- Cl₂-Phenyl | CO | Me | | | |
| 506. | a-d | NH-2,4,6-Cl₃-Phenyl | CO | Me | | | |
| 507. | a-d | NH-2,3,4- Cl₃-Phenyl | CO | Me | | | |
| 508. | a-d | NH-3,4,5- Cl₃-Phenyl | CO | Me | | | |
| 509. | a-d | NH-2-F-4-Cl-Phenyl | CO | Me | | | |
| 510. | a-d | NH-2-Cl-4-F-Phenyl | CO | Me | | | |
| 511. | a-d | NH-2-F-3-Cl-Phenyl | CO | Me | | | |
| 512. | a-d | NH-2-Cl-3-F-Phenyl | CO | Me | | | |
| 513. | a-d | NH-2-F-5-Cl-Phenyl | CO | Me | | | |
| 514. | a-d | NH-2-Cl-5-F-Phenyl | CO | Me | | | |
| 515. | a-d | NH-2-Cl-6-F-Phenyl | CO | Me | | | |
| 516. | a-d | NH-2-Br-Phenyl | CO | Me | | | |
| 517. | a-d | NH-3-Br-Phenyl | CO | Me | | | |
| 518. | a-d | NH-4-Br-Phenyl | CO | Me | | | |
| 519. | a-d | NH-2,3-Br₂-Phenyl | CO | Me | | | |
| 520. | a-d | NH-2,4-Br₂-Phenyl | CO | Me | | | |
| 521. | a-d | NH-2,5-Br₂-Phenyl | CO | Me | | | |
| 522. | a-d | NH-2-I-Phenyl | CO | Me | | | |
| 523. | a-d | NH-3-I-Phenyl | CO | Me | | | |
| 524. | a-d | NH-4-I-Phenyl | CO | Me | | | |
| 525. | a-d | NH-2-F-4-MeO-Phenyl | CO | Me | | | |
| 526. | a-d | NH-2-F-5-MeO-Phenyl | CO | Me | | | |
| 527. | a-d | NH-2-MeO-Phenyl | CO | Me | | | |
| 528. | a-d | NH-3-MeO-Phenyl | CO | Me | | | |
| 529. | a-d | NH-4-MeO-Phenyl | CO | Me | | | |
| 530. | a-d | NH-2,4-(MeO)₂-Phenyl | CO | Me | | | |
| 531. | a-d | NH-2,3-(MeO)₂-Phenyl | CO | Me | | | |
| 532. | a-d | NH-2,5-(MeO)₂-Phenyl | CO | Me | | | |
| 533. | a-d | NH-2-Me-Phenyl | CO | Me | | | |
| 534. | a-d | NH-3-Me-Phenyl | CO | Me | | | |
| 535. | a-d | NH-4-Me-Phenyl | CO | Me | | | |
| 536. | a-d | NH-2,4-(Me)₂-Phenyl | CO | Me | | | |
| 537. | a-d | NH-2,5-(Me)₂-Phenyl | CO | Me | | | |
| 538. | a-d | NH-2-CF₃-Phenyl | CO | Me | | | |
| 539. | a-d | NH-3-CF₃-Phenyl | CO | Me | | | |
| 540. | a-d | NH-4-CF₃-Phenyl | CO | Me | | | |
| 541. | a-d | NH-2,4-(CF₃)₂-Phenyl | CO | Me | | | |
| 542. | a-d | NH-2,6-Cl₂-4-(CF₃)₂-Phenyl | CO | Me | | | |
| 543. | a-d | NH-2-CF₃O-Phenyl | CO | Me | | | |
| 544. | a-d | NH-3-CF₃O-Phenyl | CO | Me | | | |
| 545. | a-d | NH-4-CF₃O-Phenyl | CO | Me | | | |
| 546. | a-d | NH-5-F-Pyridin-2-yl | CO | Me | | | |
| 547. | a-d | NH-5-Cl-Pyridin-2-yl | CO | Me | | | |
| 548. | a-d | NH-5-F-Pyridin-4-yl | CO | Me | | | |
| 549. | a-d | NH-5-Cl-Pyridin-4-yl | CO | Me | | | |
| 550. | a-d | NH-2-CN-Phenyl | CO | Me | | | |
| 551. | a-d | NH-3-CN-Phenyl | CO | Me | | | |
| 552. | a-d | NH-4-CN-Phenyl | CO | Me | | | |
| 553. | a-d | NH-3,5-(CN)₂-Phenyl | CO | Me | | | |
| 554. | a-d | NH-2-CN-4-F-Phenyl | CO | Me | | | |
| 555. | a-d | NH-4-CN-2-F-Phenyl | CO | Me | | | |
| 556. | a-d | NH-Phenyl | CO | MeO | | | |
| 557. | a-d | NH-Napht-1-yl | CO | MeO | | | |
| 558. | a-d | NH-Pyridin-2-yl | CO | MeO | | | |
| 559. | a-d | NH-Pyridin-3-yl | CO | MeO | | | |
| 560. | a-d | NH-Pyridin-4-yl | CO | MeO | | | |
| 561. | a-d | NH-2-F-Phenyl | CO | MeO | | | |
| 562. | a-d | NH-3-F-Phenyl | CO | MeO | | | |
| 563. | a-d | NH-4-F-Phenyl | CO | MeO | | | |
| 564. | a-d | NH-2,3-F₂-Phenyl | CO | MeO | | | |
| 565. | a-d | NH-2,4-F₂-Phenyl | CO | MeO | | | |
| 566. | a-d | NH-2,5-F₂-Phenyl | CO | MeO | | | |
| 567. | a-d | NH-2,6-F₂-Phenyl | CO | MeO | | | |
| 568. | a-d | NH-3,4-F₂-Phenyl | CO | MeO | | | |
| 569. | a-d | NH-3,5-F₂-Phenyl | CO | MeO | | | |
| 570. | a-d | NH-2,4,6-F₃-Phenyl | CO | MeO | | | |
| 571. | a-d | NH-2,3,4-F₃-Phenyl | CO | MeO | | | |
| 572. | a-d | NH-2-Cl-Phenyl | CO | MeO | | | |
| 573. | a-d | NH-3-Cl-Phenyl | CO | MeO | | | |
| 574. | a-d | NH-4-Cl-Phenyl | CO | MeO | | | |
| 575. | a-d | NH-2,3-Cl₂-Phenyl | CO | MeO | | | |
| 576. | a-d | NH-2,4-Cl₂-Phenyl | CO | MeO | | | |
| 577. | a-d | NH-2,5-Cl₂-Phenyl | CO | MeO | | | |
| 578. | a-d | NH-2,6-Cl₂-Phenyl | CO | MeO | | | |
| 579. | a-d | NH-3,4-Cl₂-Phenyl | CO | MeO | | | |
| 580. | a-d | NH-3,5-Cl₂-Phenyl | CO | MeO | | | |
| 581. | a-d | NH-2,4,6-Cl₃-Phenyl | CO | MeO | | | |
| 582. | a-d | NH-2,3,4-Cl₃-Phenyl | CO | MeO | | | |
| 583. | a-d | NH-3,4,5-Cl₃-Phenyl | CO | MeO | | | |
| 584. | a-d | NH-2-F-4-Cl-Phenyl | CO | MeO | | | |
| 585. | a-d | NH-2-Cl-4-F-Phenyl | CO | MeO | | | |
| 586. | a-d | NH-2-F-3-Cl-Phenyl | CO | MeO | | | |
| 587. | a-d | NH-2-Cl-3-F-Phenyl | CO | MeO | | | |
| 588. | a-d | NH-2-F-5-Cl-Phenyl | CO | MeO | | | |
| 589. | a-d | NH-2-Cl-5-F-Phenyl | CO | MeO | | | |
| 590. | a-d | NH-2-Cl-6-F-Phenyl | CO | MeO | | | |
| 591. | a-d | NH-2-Br-Phenyl | CO | MeO | | | |
| 592. | a-d | NH-3-Br-Phenyl | CO | MeO | | | |
| 593. | a-d | NH-4-Br-Phenyl | CO | MeO | | | |
| 594. | a-d | NH-2,3-Br₂-Phenyl | CO | MeO | | | |
| 595. | a-d | NH-2,4-Br₂-Phenyl | CO | MeO | | | |
| 596. | a-d | NH-2,5-Br₂-Phenyl | CO | MeO | | | |
| 597. | a-d | NH-2-I-Phenyl | CO | MeO | | | |
| 598. | a-d | NH-3-I-Phenyl | CO | MeO | | | |
| 599. | a-d | NH-4-I-Phenyl | CO | MeO | | | |
| 600. | a-d | NH-2-F-4-MeO-Phenyl | CO | MeO | | | |
| 601. | a-d | NH-2-F-5-MeO-Phenyl | CO | MeO | | | |
| 602. | a-d | NH-2-MeO-Phenyl | CO | MeO | | | |
| 603. | a-d | NH-3-MeO-Phenyl | CO | MeO | | | |
| 604. | a-d | NH-4-MeO-Phenyl | CO | MeO | | | |
| 605. | a-d | NH-2,4-(MeO)₂-Phenyl | CO | MeO | | | |
| 606. | a-d | NH-2,3-(MeO)₂-Phenyl | CO | MeO | | | |
| 607. | a-d | NH-2,5-(MeO)₂-Phenyl | CO | MeO | | | |
| 608. | a-d | NH-2-Me-Phenyl | CO | MeO | | | |
| 609. | a-d | NH-3-Me-Phenyl | CO | MeO | | | |
| 610. | a-d | NH-4-Me-Phenyl | CO | MeO | | | |
| 611. | a-d | NH-2,4-(Me)₂-Phenyl | CO | MeO | | | |
| 612. | a-d | NH-2,5-(Me)₂-Phenyl | CO | MeO | | | |
| 613. | a-d | NH-2-CF₃-Phenyl | CO | MeO | | | |
| 614. | a-d | NH-3-CF₃-Phenyl | CO | MeO | | | |
| 615. | a-d | NH-4-CF₃-Phenyl | CO | MeO | | | |
| 616. | a-d | NH-2,4-(CF₃)₂-Phenyl | CO | MeO | | | |
| 617. | a-d | NH-2,6-Cl₂-4-(CF₃)₂-Phenyl | CO | MeO | | | |
| 618. | a-d | NH-2-CF₃O-Phenyl | CO | MeO | | | |
| 619. | a-d | NH-3-CF₃O-Phenyl | CO | MeO | | | |
| 620. | a-d | NH-4-CF₃O-Phenyl | CO | MeO | | | |
| 621. | a-d | NH-5-F-Pyridin-2-yl | CO | MeO | | | |
| 622. | a-d | NH-5-Cl-Pyridin-2-yl | CO | MeO | | | |
| 623. | a-d | NH-5-F-Pyridin-4-yl | CO | MeO | | | |
| 624. | a-d | NH-5-Cl-Pyridin-4-yl | CO | MeO | | | |
| 625. | a-d | NH-2-CN-Phenyl | CO | MeO | | | |
| 626. | a-d | NH-3-CN-Phenyl | CO | MeO | | | |
| 627. | a-d | NH-4-CN-Phenyl | CO | MeO | | | |
| 628. | a-d | NH-3,5-(CN)₂-Phenyl | CO | MeO | | | |
| 629. | a-d | NH-2-CN-4-F-Phenyl | CO | MeO | | | |
| 630. | a-d | NH-4-CN-2-F-Phenyl | CO | MeO | | | |
| 631. | a-d | NH-Phenyl | CO | CHO | | | |
| 632. | a-d | NH-Napht-1-yl | CO | CHO | | | |
| 633. | a-d | NH-Pyridin-2-yl | CO | CHO | | | |
| 634. | a-d | NH-Pyridin-3-yl | CO | CHO | | | |
| 635. | a-d | NH-Pyridin-4-yl | CO | CHO | | | |
| 636. | a-d | NH-2-F-Phenyl | CO | CHO | | | |
| 637. | a-d | NH-3-F-Phenyl | CO | CHO | | | |
| 638. | a-d | NH-4-F-Phenyl | CO | CHO | | | |
| 639. | a-d | NH-2,3-F₂-Phenyl | CO | CHO | | | |
| 640. | a-d | NH-2,4-F₂-Phenyl | CO | CHO | | | |
| 641. | a-d | NH-2,5-F₂-Phenyl | CO | CHO | | | |
| 642. | a-d | NH-2,6-F₂-Phenyl | CO | CHO | | | |
| 643. | a-d | NH-3,4-F₂-Phenyl | CO | CHO | | | |
| 644. | a-d | NH-3,5-F₂-Phenyl | CO | CHO | | | |
| 645. | a-d | NH-2,4,6-F₃-Phenyl | CO | CHO | | | |
| 646. | a-d | NH-2,3,4-F₃-Phenyl | CO | CHO | | | |
| 647. | a-d | NH-2-Cl-Phenyl | CO | CHO | | | |
| 648. | a-d | NH-3-Cl-Phenyl | CO | CHO | | | |
| 649. | a-d | NH-4-Cl-Phenyl | CO | CHO | | | |
| 650. | a-d | NH-2,3-Cl₂-Phenyl | CO | CHO | | | |
| 651. | a-d | NH-2,4-Cl₂-Phenyl | CO | CHO | | | |
| 652. | a-d | NH-2,5-Cl₂-Phenyl | CO | CHO | | | |
| 653. | a-d | NH-2,6-Cl₂-Phenyl | CO | CHO | | | |
| 654. | a-d | NH-3,4-Cl₂-Phenyl | CO | CHO | | | |
| 655. | a-d | NH-3,5-Cl₂-Phenyl | CO | CHO | | | |
| 656. | a-d | NH-2,4,6-Cl₃-Phenyl | CO | CHO | | | |
| 657. | a-d | NH-2,3,4-Cl₃-Phenyl | CO | CHO | | | |
| 658. | a-d | NH-3,4,5-Cl₃-Phenyl | CO | CHO | | | |
| 659. | a-d | NH-2-F-4-Cl-Phenyl | CO | CHO | | | |
| 660. | a-d | NH-2-Cl-4-F-Phenyl | CO | CHO | | | |
| 661. | a-d | NH-2-F-3-Cl-Phenyl | CO | CHO | | | |
| 662. | a-d | NH-2-Cl-3-F-Phenyl | CO | CHO | | | |
| 663. | a-d | NH-2-F-5-Cl-Phenyl | CO | CHO | | | |
| 664. | a-d | NH-2-Cl-5-F-Phenyl | CO | CHO | | | |
| 665. | a-d | NH-2-Cl-6-F-Phenyl | CO | CHO | | | |
| 666. | a-d | NH-2-Br-Phenyl | CO | CHO | | | |
| 667. | a-d | NH-3-Br-Phenyl | CO | CHO | | | |
| 668. | a-d | NH-4-Br-Phenyl | CO | CHO | | | |
| 669. | a-d | NH-2,3-Br₂-Phenyl | CO | CHO | | | |
| 670. | a-d | NH-2,4-Br₂-Phenyl | CO | CHO | | | |
| 671. | a-d | NH-2,5-Br₂-Phenyl | CO | CHO | | | |
| 672. | a-d | NH-2-I-Phenyl | CO | CHO | | | |
| 673. | a-d | NH-3-I-Phenyl | CO | CHO | | | |
| 674. | a-d | NH-4-I-Phenyl | CO | CHO | | | |
| 675. | a-d | NH-2-F-4-MeO-Phenyl | CO | CHO | | | |
| 676. | a-d | NH-2-F-5-MeO-Phenyl | CO | CHO | | | |
| 677. | a-d | NH-2-MeO-Phenyl | CO | CHO | | | |
| 678. | a-d | NH-3-MeO-Phenyl | CO | CHO | | | |
| 679. | a-d | NH-4-MeO-Phenyl | CO | CHO | | | |
| 680. | a-d | NH-2,4-(MeO)₂-Phenyl | CO | CHO | | | |
| 681. | a-d | NH-2,3-(MeO)₂-Phenyl | CO | CHO | | | |
| 682. | a-d | NH-2,5-(MeO)₂-Phenyl | CO | CHO | | | |
| 683. | a-d | NH-2-Me-Phenyl | CO | CHO | | | |
| 684. | a-d | NH-3-Me-Phenyl | CO | CHO | | | |
| 685. | a-d | NH-4-Me-Phenyl | CO | CHO | | | |
| 686. | a-d | NH-2,4-(Me)₂-Phenyl | CO | CHO | | | |
| 687. | a-d | NH-2,5-(Me)₂-Phenyl | CO | CHO | | | |
| 688. | a-d | NH-2-CF₃-Phenyl | CO | CHO | | | |
| 689. | a-d | NH-3-CF₃-Phenyl | CO | CHO | | | |
| 690. | a-d | NH-4-CF₃-Phenyl | CO | CHO | | | |
| 691. | a-d | NH-2,4-(CF₃)₂-Phenyl | CO | CHO | | | |
| 692. | a-d | NH-2,6-Cl₂-4-(CF₃)₂-Phenyl | CO | CHO | | | |
| 693. | a-d | NH-2-CF₃O-Phenyl | CO | CHO | | | |
| 694. | a-d | NH-3-CF₃O-Phenyl | CO | CHO | | | |
| 695. | a-d | NH-4-CF₃O-Phenyl | CO | CHO | | | |
| 696. | a-d | NH-5-F-Pyridin-2-yl | CO | CHO | | | |
| 697. | a-d | NH-5-Cl-Pyridin-2-yl | CO | CHO | | | |
| 698. | a-d | NH-5-F-Pyridin-4-yl | CO | CHO | | | |
| 699. | a-d | NH-5-Cl-Pyridin-4-yl | CO | CHO | | | |
| 700. | a-d | NH-2-CN-Phenyl | CO | CHO | | | |
| 701. | a-d | NH-3-CN-Phenyl | CO | CHO | | | |
| 702. | a-d | NH-4-CN-Phenyl | CO | CHO | | | |
| 703. | a-d | NH-3,5-(CN)₂-Phenyl | CO | CHO | | | |
| 704. | a-d | NH-2-CN-4-F-Phenyl | CO | CHO | | | |
| 705. | a-d | NH-4-CN-2-F-Phenyl | CO | CHO | | | |
| 706. | a-d | NH-Phenyl | CO | NO₂ | | | |
| 707. | a-d | NH-Napht-1-yl | CO | NO₂ | | | |
| 708. | a-d | NH-Pyridin-2-yl | CO | NO₂ | | | |
| 709. | a-d | NH-Pyridin-3-yl | CO | NO₂ | | | |
| 710. | a-d | NH-Pyridin-4-yl | CO | NO₂ | | | |
| 711. | a-d | NH-2-F-Phenyl | CO | NO₂ | | | |
| 712. | a-d | NH-3-F-Phenyl | CO | NO₂ | | | |
| 713. | a-d | NH-4-F-Phenyl | CO | NO₂ | | | |
| 714. | a-d | NH-2,3-F₂-Phenyl | CO | NO₂ | | | |
| 715. | a-d | NH-2,4-F₂-Phenyl | CO | NO₂ | | | |
| 716. | a-d | NH-2,5-F₂-Phenyl | CO | NO₂ | | | |
| 717. | a-d | NH-2,6-F₂-Phenyl | CO | NO₂ | | | |
| 718. | a-d | NH-3,4-F₂-Phenyl | CO | NO₂ | | | |
| 719. | a-d | NH-3,5-F₂-Phenyl | CO | NO₂ | | | |
| 720. | a-d | NH-2,4,6-F₃-Phenyl | CO | NO₂ | | | |
| 721. | a-d | NH-2,3,4-F₃-Phenyl | CO | NO₂ | | | |
| 722. | a-d | NH-2-Cl-Phenyl | CO | NO₂ | | | |
| 723. | a-d | NH-3-Cl-Phenyl | CO | NO₂ | | | |
| 724. | a-d | NH-4- Cl-Phenyl | CO | NO₂ | | | |
| 725. | a-d | NH-2,3-Cl₂-Phenyl | CO | NO₂ | | | |
| 726. | a-d | NH-2,4- Cl₂-Phenyl | CO | NO₂ | | | |
| 727. | a-d | NH-2,5- Cl₂-Phenyl | CO | NO₂ | | | |
| 728. | a-d | NH-2,6- Cl₂-Phenyl | CO | NO₂ | | | |
| 729. | a-d | NH-3,4- Cl₂-Phenyl | CO | NO₂ | | | |
| 730. | a-d | NH-3,5- Cl₂-Phenyl | CO | NO₂ | | | |
| 731. | a-d | NH-2,4,6- Cl₃-Phenyl | CO | NO₂ | | | |
| 732. | a-d | NH-2,3,4- Cl₃-Phenyl | CO | NO₂ | | | |
| 733. | a-d | NH-3,4,5- Cl₃-Phenyl | CO | NO₂ | | | |
| 734. | a-d | NH-2-F-4-Cl-Phenyl | CO | NO₂ | | | |
| 735. | a-d | NH-2-Cl-4-F-Phenyl | CO | NO₂ | | | |
| 736. | a-d | NH-2-F-3-Cl-Phenyl | CO | NO₂ | | | |
| 737. | a-d | NH-2-Cl-3-F-Phenyl | CO | NO₂ | | | |
| 738. | a-d | NH-2-F-5-Cl-Phenyl | CO | NO₂ | | | |
| 739. | a-d | NH-2-Cl-5-F-Phenyl | CO | NO₂ | | | |
| 740. | a-d | NH-2-Cl-6-F-Phenyl | CO | NO₂ | | | |
| 741. | a-d | NH-2-Br-Phenyl | CO | NO₂ | | | |
| 742. | a-d | NH-3-Br-Phenyl | CO | NO₂ | | | |
| 743. | a-d | NH-4-Br-Phenyl | CO | NO₂ | | | |
| 744. | a-d | NH-2,3-Br₂-Phenyl | CO | NO₂ | | | |
| 745. | a-d | NH-2,4-Br₂-Phenyl | CO | NO₂ | | | |
| 746. | a-d | NH-2,5-Br₂-Phenyl | CO | NO₂ | | | |
| 747. | a-d | NH-2-I-Phenyl | CO | NO₂ | | | |
| 748. | a-d | NH-3-I-Phenyl | CO | NO₂ | | | |
| 749. | a-d | NH-4-I-Phenyl | CO | NO₂ | | | |
| 750. | a-d | NH-2-F-4-MeO-Phenyl | CO | NO₂ | | | |
| 751. | a-d | NH-2-F-5-MeO-Phenyl | CO | NO₂ | | | |
| 752. | a-d | NH-2-MeO-Phenyl | CO | NO₂ | | | |
| 753. | a-d | NH-3-MeO-Phenyl | CO | NO₂ | | | |
| 754. | a-d | NH-4-MeO-Phenyl | CO | NO₂ | | | |
| 755. | a-d | NH-2,4-(MeO)₂-Phenyl | CO | NO₂ | | | |
| 756. | a-d | NH-2,3-(MeO)₂-Phenyl | CO | NO₂ | | | |
| 757. | a-d | NH-2,5-(MeO)₂-Phenyl | CO | NO₂ | | | |
| 758. | a-d | NH-2-Me-Phenyl | CO | NO₂ | | | |
| 759. | a-d | NH-3-Me-Phenyl | CO | NO₂ | | | |
| 760. | a-d | NH-4-Me-Phenyl | CO | NO₂ | | | |
| 761. | a-d | NH-2,4-(Me)₂-Phenyl | CO | NO₂ | | | |
| 762. | a-d | NH-2,5-(Me)₂-Phenyl | CO | NO₂ | | | |
| 763. | a-d | NH-2-CF₃-Phenyl | CO | NO₂ | | | |
| 764. | a-d | NH-3-CF₃-Phenyl | CO | NO₂ | | | |
| 765. | a-d | NH-4-CF₃-Phenyl | CO | NO₂ | | | |
| 766. | a-d | NH-2,4-(CF₃)₂-Phenyl | CO | NO₂ | | | |
| 767. | a-d | NH-2,6-Cl₂-4-(CF₃)₂-Phenyl | CO | NO₂ | | | |
| 768. | a-d | NH-2-CF₃O-Phenyl | CO | NO₂ | | | |
| 769. | a-d | NH-3-CF₃O-Phenyl | CO | NO₂ | | | |
| 770. | a-d | NH-4-CF₃O-Phenyl | CO | NO₂ | | | |
| 771. | a-d | NH-5-F-Pyridin-2-yl | CO | NO₂ | | | |
| 772. | a-d | NH-5-Cl-Pyridin-2-yl | CO | NO₂ | | | |
| 773. | a-d | NH-5-F-Pyridin-4-yl | CO | NO₂ | | | |
| 774. | a-d | NH-5-Cl-Pyridin-4-yl | CO | NO₂ | | | |
| 775. | a-d | NH-2-CN-Phenyl | CO | NO₂ | | | |
| 776. | a-d | NH-3-CN-Phenyl | CO | NO₂ | | | |
| 777. | a-d | NH-4-CN-Phenyl | CO | NO₂ | | | |
| 778. | a-d | NH-3,5-(CN)₂-Phenyl | CO | NO₂ | | | |
| 779. | a-d | NH-2-CN-4-F-Phenyl | CO | NO₂ | | | |
| 780. | a-d | NH-4-CN-2-F-Phenyl | CO | NO₂ | | | |
| 781. | a-d | NH₂ | CH₂ | CN | | | |
| 782. | a-d | NH₂ | CH₂ | Me | | | |
| 783. | a-d | NH₂ | CH₂ | MeO | gelbes Harz | | |
| 784. | a-d | NH₂ | CH₂ | CHO | | | |
| 785. | a-d | NH₂ | CH₂ | NO₂ | | | |
| 786. | a-d | NH-COMe | CH₂ | CN | | | |
| 787. | a-d | NH-COEt | CH₂ | CN | | | |
| 788. | a-d | NH-CO*n*Pr | CH₂ | CN | | | |
| 789. | a-d | NH-CO*i*Pr | CH₂ | CN | | | |
| 790. | a-d | NH-CO*c*Pr | CH₂ | CN | | | |
| 791. | a-d | NH-CO*n*Bu | CH₂ | CN | | | |
| 792. | a-d | NH-CO*i*Bu | CH₂ | CN | | | |
| 793. | a-d | NH-CO*c*Bu | CH₂ | CN | | | |
| 794. | a-d | NH-CO*c*Pentyl | CH₂ | CN | | | |
| 795. | a-d | NH-CO*c*Hexyl | CH₂ | CN | | | |
| 796. | a-d | NH-COCF₃ | CH₂ | CN | | | |
| 797. | a-d | NH-COCHF₂ | CH₂ | CN | | | |
| 798. | a-d | NH-COCH₂F | CH₂ | CN | | | |
| 799. | a-d | NH-COCCl₃ | CH₂ | CN | | | |
| 800. | a-d | NH-COCHCl₂ | CH₂ | CN | | | |
| 801. | a-d | NH-COCH₂Cl | CH₂ | CN | | | |
| 802. | a-d | NH-COCH₂OMe | CH₂ | CN | | | |
| 803. | a-d | NH-COCH(OMe)₂ | CH₂ | CN | | | |
| 804. | a-d | NH-COCH₂OEt | CH₂ | CN | | | |
| 805. | a-d | NH-COCH(OEt)₂ | CH₂ | CN | | | |
| 806. | a-d | NH-COPh | CH₂ | CN | | | |
| 807. | a-d | NH-CO(2-F-Ph) | CH₂ | CN | | | |
| 808. | a-d | NH-CO(3-F-Ph) | CH₂ | CN | | | |
| 809. | a-d | NH-CO(4-F-Ph) | CH₂ | CN | | | |
| 810. | a-d | NH-CO(2,4-F₂-Ph) | CH₂ | CN | | | |
| 811. | a-d | NH-CO(2,4,6-F₃-Ph) | CH₂ | CN | | | |
| 812. | a-d | NH-CO(2-Cl-Ph) | CH₂ | CN | | | |
| 813. | a-d | NH-CO(3-Cl-Ph) | CH₂ | CN | | | |
| 814. | a-d | NH-CO(4-Cl-Ph) | CH₂ | CN | | | |
| 815. | a-d | NH-CO(2,4-Cl₂-Ph) | CH₂ | CN | | | |
| 816. | a-d | NH-CO(2,4,6-Cl₃-Ph) | CH₂ | CN | | | |
| 817. | a-d | NH-COBn | CH₂ | CN | | | |
| 818. | a-d | NH-CO(2-F-4-Cl-Ph) | CH₂ | CN | | | |
| 819. | a-d | NH-CO(2-Cl-4-F-Ph) | CH₂ | CN | | | |
| 820. | a-d | NH-CO(2-Me-Ph) | CH₂ | CN | | | |
| 821. | a-d | NH-CO(3-Me-Ph) | CH₂ | CN | | | |
| 822. | a-d | NH-CO(4-Me-Ph) | CH₂ | CN | | | |
| 823. | a-d | NH-CO(2-CF₃-Ph) | CH₂ | CN | | | |
| 824. | a-d | NH-CO(3-CF₃-Ph) | CH₂ | CN | | | |
| 825. | a-d | NH-CO(4-CF₃-Ph) | CH₂ | CN | | | |
| 826. | a-d | NH-COMe | CH₂ | Me | | | |
| 827. | a-d | NH-COEt | CH₂ | Me | | | |
| 828. | a-d | NH-CO*n*Pr | CH₂ | Me | | | |
| 829. | a-d | NH-CO*i*Pr | CH₂ | Me | | | |
| 830. | a-d | NH-CO*c*Pr | CH₂ | Me | | | |
| 831. | a-d | NH-CO*n*Bu | CH₂ | Me | | | |
| 832. | a-d | NH-CO*i*Bu | CH₂ | Me | | | |
| 833. | a-d | NH-CO*c*Bu | CH₂ | Me | | | |
| 834. | a-d | NH-CO*c*Pentyl | CH₂ | Me | | | |
| 835. | a-d | NH-CO*c*Hexyl | CH₂ | Me | | | |
| 836. | a-d | NH-COCF₃ | CH₂ | Me | | | |
| 837. | a-d | NH-COCHF₂ | CH₂ | Me | | | |
| 838. | a-d | NH-COCH₂F | CH₂ | Me | | | |
| 839. | a-d | NH-COCCl₃ | CH₂ | Me | | | |
| 840. | a-d | NH-COCHCl₂ | CH₂ | Me | | | |
| 841. | a-d | NH-COCH₂Cl | CH₂ | Me | | | |
| 842. | a-d | NH-COCH₂OMe | CH₂ | Me | | | |
| 843. | a-d | NH-COCH(OMe)₂ | CH₂ | Me | | | |
| 844. | a-d | NH-COCH₂OEt | CH₂ | Me | | | |
| 845. | a-d | NH-COCH(OEt)₂ | CH₂ | Me | | | |
| 846. | a-d | NH-COPh | CH₂ | Me | | | |
| 847. | a-d | NH-CO(2-F-Ph) | CH₂ | Me | | | |
| 848. | a-d | NH-CO(3-F-Ph) | CH₂ | Me | | | |
| 849. | a-d | NH-CO(4-F-Ph) | CH₂ | Me | | | |
| 850. | a-d | NH-CO(2,4-F₂-Ph) | CH₂ | Me | | | |
| 851. | a-d | NH-CO(2,4,6-F₃-Ph) | CH₂ | Me | | | |
| 852. | a-d | NH-CO(2-Cl-Ph) | CH₂ | Me | | | |
| 853. | a-d | NH-CO(3-Cl-Ph) | CH₂ | Me | | | |
| 854. | a-d | NH-CO(4-Cl-Ph) | CH₂ | Me | | | |
| 855. | a-d | NH-CO(2,4-Cl₂-Ph) | CH₂ | Me | | | |
| 856. | a-d | NH-CO(2,4,6-Cl₃-Ph) | CH₂ | Me | | | |
| 857. | a-d | NH-COBn | CH₂ | Me | | | |
| 858. | a-d | NH-CO(2-F-4-Cl-Ph) | CH₂ | Me | | | |
| 859. | a-d | NH-CO(2-Cl-4-F-Ph) | CH₂ | Me | | | |
| 860. | a-d | NH-CO(2-Me-Ph) | CH₂ | Me | | | |
| 861. | a-d | NH-CO(3-Me-Ph) | CH₂ | Me | | | |
| 862. | a-d | NH-CO(4-Me-Ph) | CH₂ | Me | | | |
| 863. | a-d | NH-CO(2-CF₃-Ph) | CH₂ | Me | | | |
| 864. | a-d | NH-CO(3-CF₃-Ph) | CH₂ | Me | | | |
| 865. | a-d | NH-CO(4-CF₃-Ph) | CH₂ | Me | | | |
| 866. | a-d | NH-COMe | CH₂ | MeO | | | |
| 867. | a-d | NH-COEt | CH₂ | MeO | | | |
| 868. | a-d | NH-CO*n*Pr | CH₂ | MeO | | | |
| 869. | a-d | NH-CO*i*Pr | CH₂ | MeO | 86 | | |
| 870. | a-d | NH-CO*c*Pr | CH₂ | MeO | 86 | | |
| 871. | a-d | NH-CO*n*Bu | CH₂ | MeO | | | |
| 872. | a-d | NH-CO*i*Bu | CH₂ | MeO | | | |
| 873. | a-d | NH-CO*c*Bu | CH₂ | MeO | | | |
| 874. | a-d | NH-CO*c*Pentyl | CH₂ | MeO | | | |
| 875. | a-d | NH-CO*c*Hexyl | CH₂ | MeO | | | |
| 876. | a-d | NH-COCF₃ | CH₂ | MeO | brauner semi-kristalliner Feststoff | | |
| 877. | a-d | NH-COCHF₂ | CH₂ | MeO | | | |
| 878. | a-d | NH-COCH₂F | CH₂ | MeO | | | |
| 879. | a-d | NH-COCCl₃ | CH₂ | MeO | | | |
| 880. | a-d | NH-COCHCl₂ | CH₂ | MeO | | | |
| 881. | a-d | NH-COCH₂Cl | CH₂ | MeO | | | |
| 882. | a-d | NH-COCH₂OMe | CH₂ | MeO | | | |
| 883. | a-d | NH-COCH(OMe)₂ | CH₂ | MeO | | | |
| 884. | a-d | NH-COCH₂OEt | CH₂ | MeO | | | |
| 885. | a-d | NH-COCH(OEt)₂ | CH₂ | MeO | | | |
| 886. | a-d | NH-COPh | CH₂ | MeO | | | |
| 887. | a-d | NH-CO(2-F-Ph) | CH₂ | MeO | | | |
| 888. | a-d | NH-CO(3-F-Ph) | CH₂ | MeO | | | |
| 889. | a-d | NH-CO(4-F-Ph) | CH₂ | MeO | | | |
| 890. | a-d | NH-CO(2,4-F₂-Ph) | CH₂ | MeO | | | |
| 891. | a-d | NH-CO(2,4,6-F₃-Ph) | CH₂ | MeO | | | |
| 892. | a-d | NH-CO(2-Cl-Ph) | CH₂ | MeO | | | |
| 893. | a-d | NH-CO(3-Cl-Ph) | CH₂ | MeO | | | |
| 894. | a-d | NH-CO(4-Cl-Ph) | CH₂ | MeO | | | |
| 895. | a-d | NH-CO(2,4-Cl₂-Ph) | CH₂ | MeO | | | |
| 896. | a-d | NH-CO(2,4,6-Cl₃-Ph) | CH₂ | MeO | | | |
| 897. | a-d | NH-COBn | CH₂ | MeO | | | |
| 898. | a-d | NH-CO(2-F-4-Cl-Ph) | CH₂ | MeO | | | |
| 899. | a-d | NH-CO(2-Cl-4-F-Ph) | CH₂ | MeO | | | |
| 900. | a-d | NH-CO(2-Me-Ph) | CH₂ | MeO | | | |
| 901. | a-d | NH-CO(3-Me-Ph) | CH₂ | MeO | | | |
| 902. | a-d | NH-CO(4-Me-Ph) | CH₂ | MeO | | | |
| 903. | a-d | NH-CO(2-CF₃-Ph) | CH₂ | MeO | | | |
| 904. | a-d | NH-CO(3-CF₃-Ph) | CH₂ | MeO | | | |
| 905. | a-d | NH-CO(4-CF₃-Ph-CO) | CH₂ | MeO | | | |
| 906. | a-d | NH-COMe | CH₂ | CHO | | | |
| 907. | a-d | NH-COEt | CH₂ | CHO | | | |
| 908. | a-d | NH-CO*n*Pr | CH₂ | CHO | | | |
| 909. | a-d | NH-CO*i*Pr | CH₂ | CHO | | | |
| 910. | a-d | NH-CO*c*Pr | CH₂ | CHO | | | |
| 911. | a-d | NH-CO*n*Bu | CH₂ | CHO | | | |
| 912. | a-d | NH-CO*i*Bu | CH₂ | CHO | | | |
| 913. | a-d | NH-CO*c*Bu | CH₂ | CHO | | | |
| 914. | a-d | NH-CO*c*Pentyl | CH₂ | CHO | | | |
| 915. | a-d | NH-CO*c*Hexyl | CH₂ | CHO | | | |
| 916. | a-d | NH-COCF₃ | CH₂ | CHO | | | |
| 917. | a-d | NH-COCHF₂ | CH₂ | CHO | | | |
| 918. | a-d | NH-COCH₂F | CH₂ | CHO | | | |
| 919. | a-d | NH-COCCl₃ | CH₂ | CHO | | | |
| 920. | a-d | NH-COCHCl₂ | CH₂ | CHO | | | |
| 921. | a-d | NH-COCH₂Cl | CH₂ | CHO | | | |
| 922. | a-d | NH-COCH₂OMe | CH₂ | CHO | | | |
| 923. | a-d | NH-COCH(OMe)₂ | CH₂ | CHO | | | |
| 924. | a-d | NH-COCH₂OEt | CH₂ | CHO | | | |
| 925. | a-d | NH-COCH(OEt)₂ | CH₂ | CHO | | | |
| 926. | a-d | NH-COPh | CH₂ | CHO | | | |
| 927. | a-d | NH-CO(2-F-Ph) | CH₂ | CHO | | | |
| 928. | a-d | NH-CO(3-F-Ph) | CH₂ | CHO | | | |
| 929. | a-d | NH-CO(4-F-Ph) | CH₂ | CHO | | | |
| 930. | a-d | NH-CO(2,4-F₂-Ph) | CH₂ | CHO | | | |
| 931. | a-d | NH-CO(2,4,6-F₃-Ph) | CH₂ | CHO | | | |
| 932. | a-d | NH-CO(2-Cl-Ph) | CH₂ | CHO | | | |
| 933. | a-d | NH-CO(3-Cl-Ph) | CH₂ | CHO | | | |
| 934. | a-d | NH-CO(4-Cl-Ph) | CH₂ | CHO | | | |
| 935. | a-d | NH-CO(2,4-Cl₂-Ph) | CH₂ | CHO | | | |
| 936. | a-d | NH-CO(2,4,6-Cl₃-Ph) | CH₂ | CHO | | | |
| 937. | a-d | NH-COBn | CH₂ | CHO | | | |
| 938. | a-d | NH-CO(2-F-4-Cl-Ph) | CH₂ | CHO | | | |
| 939. | a-d | NH-CO(2-Cl-4-F-Ph) | CH₂ | CHO | | | |
| 940. | a-d | NH-CO(2-Me-Ph) | CH₂ | CHO | | | |
| 941. | a-d | NH-CO(3-Me-Ph) | CH₂ | CHO | | | |
| 942. | a-d | NH-CO(4-Me-Ph) | CH₂ | CHO | | | |
| 943. | a-d | NH-CO(2-CF₃-Ph) | CH₂ | CHO | | | |
| 944. | a-d | NH-CO(3-CF₃-Ph) | CH₂ | CHO | | | |
| 945. | a-d | NH-CO(4-CF₃-Ph) | CH₂ | CHO | | | |
| 946. | a-d | NH-COMe | CH₂ | NO₂ | | | |
| 947. | a-d | NH-COEt | CH₂ | NO₂ | | | |
| 948. | a-d | NH-CO*n*Pr | CH₂ | NO₂ | | | |
| 949. | a-d | NH-CO*i*Pr | CH₂ | NO₂ | | | |
| 950. | a-d | NH-CO*c*Pr | CH₂ | NO₂ | | | |
| 951. | a-d | NH-CO*n*Bu | CH₂ | NO₂ | | | |
| 952. | a-d | NH-CO*i*Bu | CH₂ | NO₂ | | | |
| 953. | a-d | NH-CO*c*Bu | CH₂ | NO₂ | | | |
| 954. | a-d | NH-CO*c*Pentyl | CH₂ | NO₂ | | | |
| 955. | a-d | NH-CO*c*Hexyl | CH₂ | NO₂ | | | |
| 956. | a-d | NH-COCF₃ | CH₂ | NO₂ | | | |
| 957. | a-d | NH-COCHF₂ | CH₂ | NO₂ | | | |
| 958. | a-d | NH-COCH₂F | CH₂ | NO₂ | | | |
| 959. | a-d | NH-COCCl₃ | CH₂ | NO₂ | | | |
| 960. | a-d | NH-COCHCl₂ | CH₂ | NO₂ | | | |
| 961. | a-d | NH-COCH₂Cl | CH₂ | NO₂ | | | |
| 962. | a-d | NH-COCH₂OMe | CH₂ | NO₂ | | | |
| 963. | a-d | NH-COCH(OMe)₂ | CH₂ | NO₂ | | | |
| 964. | a-d | NH-COCH₂OEt | CH₂ | NO₂ | | | |
| 965. | a-d | NH-COCH(OEt)₂ | CH₂ | NO₂ | | | |
| 966. | a-d | NH-COPh | CH₂ | NO₂ | | | |
| 967. | a-d | NH-CO(2-F-Ph) | CH₂ | NO₂ | | | |
| 968. | a-d | NH-CO(3-F-Ph) | CH₂ | NO₂ | | | |
| 969. | a-d | NH-CO(4-F-Ph) | CH₂ | NO₂ | | | |
| 970. | a-d | NH-CO(2,4-F₂-Ph) | CH₂ | NO₂ | | | |
| 971. | a-d | NH-CO(2,4,6-F₃-Ph) | CH₂ | NO₂ | | | |
| 972. | a-d | NH-CO(2-Cl-Ph) | CH₂ | NO₂ | | | |
| 973. | a-d | NH-CO(3-Cl-Ph) | CH₂ | NO₂ | | | |
| 974. | a-d | NH-CO(4-Cl-Ph) | CH₂ | NO₂ | | | |
| 975. | a-d | NH-CO(2,4-Cl₂-Ph) | CH₂ | NO₂ | | | |
| 976. | a-d | NH-CO(2,4,6-Cl₃-Ph) | CH₂ | NO₂ | | | |
| 977. | a-d | NH-COBn | CH₂ | NO₂ | | | |
| 978. | a-d | NH-CO(2-F-4-Cl-Ph) | CH₂ | NO₂ | | | |
| 979. | a-d | NH-CO(2-Cl-4-F-Ph) | CH₂ | NO₂ | | | |
| 980. | a-d | NH-CO(2-Me-Ph) | CH₂ | NO₂ | | | |
| 981. | a-d | NH-CO(3-Me-Ph) | CH₂ | NO₂ | | | |
| 982. | a-d | NH-CO(4-Me-Ph) | CH₂ | NO₂ | | | |
| 983. | a-d | NH-CO(2-CF₃-Ph) | CH₂ | NO₂ | | | |
| 984. | a-d | NH-CO(3-CF₃-Ph) | CH₂ | NO₂ | | | |
| 985. | a-d | NH-CO(4-CF₃-Ph) | CH₂ | NO₂ | | | |
| 986. | a-d | NH-Me | CO | CN | | | |
| 987. | a-d | NH-Me | CO | Me | | | |
| 988. | a-d | NH-Me | CO | MeO | | | |
| 989. | a-d | NH-Me | CO | CHO | | | |
| 990. | a-d | NH-Me | CO | NO₂ | | | |
| 991. | a-d | NH-Et | CO | CN | | | |
| 992. | a-d | NH-Et | CO | Me | | | |
| 993. | a-d | NH-Et | CO | MeO | | | |
| 994. | a-d | NH-Et | CO | CHO | | | |
| 995. | a-d | NH-Et | CO | NO₂ | | | |
| 996. | a-d | NH-CH₂CHF₂ | CO | CN | | | |
| 997. | a-d | NH-CH₂CHF₂ | CO | Me | | | |
| 998. | a-d | NH-CH₂CHF₂ | CO | MeO | | | |
| 999. | a-d | NH-CH₂CHF₂ | CO | CHO | | | |
| 1000. | a-d | NH-CH₂CHF₂ | CO | NO₂ | | | |
| 1001. | a-d | NH-CH₂CH₂CF₃ | CO | CN | | | |
| 1002. | a-d | NH-CH₂CH₂CF₃ | CO | Me | | | |
| 1003. | a-d | NH-CH₂CH₂CF₃ | CO | MeO | | | |
| 1004. | a-d | NH-CH₂CH₂CF₃ | CO | CHO | | | |
| 1005. | a-d | NH-CH₂CH₂CF₃ | CO | NO₂ | | | |
| 1006. | a-d | NH-CH₂CF₃ | CO | CN | | | |
| 1007. | a-d | NH-CH₂CF₃ | CO | Me | | | |
| 1008. | a-d | NH-CH₂CF₃ | CO | MeO | | | |
| 1009. | a-d | NH-CH₂CF₃ | CO | CHO | | | |
| 1010. | a-d | NH-CH₂CF₃ | CO | NO₂ | | | |
| 1011. | a-d | NH-Bn | CO | CN | | | |
| 1012. | a-d | NH-Bn | CO | Me | | | |
| 1013. | a-d | NH-Bn | CO | MeO | | | |
| 1014. | a-d | NH-Bn | CO | CHO | | | |
| 1015. | a-d | NH-Bn | CO | NO₂ | | | |
| 1016. | a-d | NH-*c*Hexyl | CO | CN | | | |
| 1017. | a-d | NH-*c*Hexyl | CO | Me | | | |
| 1018. | a-d | NH-*c*Hexyl | CO | MeO | | | |
| 1019. | a-d | NH-*c*Hexyl | CO | CHO | | | |
| 1020. | a-d | NH-*c*Hexyl | CO | NO₂ | | | |
| 1021. | a-d | NH-*c*Pentyl | CO | CN | | | |
| 1022. | a-d | NH-*c*Pentyl | CO | Me | | | |
| 1023. | a-d | NH-*c*Pentyl | CO | MeO | | | |
| 1024. | a-d | NH-*c*Pentyl | CO | CHO | | | |
| 1025. | a-d | NH-*c*Pentyl | CO | NO₂ | | | |
| 1026. | a-d | NH-*c*Bu | CO | CN | | | |
| 1027. | a-d | NH-*c*Bu | CO | Me | | | |
| 1028. | a-d | NH-*c*Bu | CO | MeO | | | |
| 1029. | a-d | NH-*c*Bu | CO | CHO | | | |
| 1030. | a-d | NH-*c*Bu | CO | NO₂ | | | |
| 1031. | a-d | NH-*n*Bu | CO | CN | | | |
| 1032. | a-d | NH-*n*Bu | CO | Me | | | |
| 1033. | a-d | NH-*n*Bu | CO | MeO | | | |
| 1034. | a-d | NH-*n*Bu | CO | CHO | | | |
| 1035. | a-d | NH-*n*Bu | CO | NO₂ | | | |
| 1036. | a-d | NH-*n*Pr | CO | CN | | | |
| 1037. | a-d | NH-*n*Pr | CO | Me | | | |
| 1038. | a-d | NH-*n*Pr | CO | MeO | | | |
| 1039. | a-d | NH-*n*Pr | CO | CHO | | | |
| 1040. | a-d | NH-*n*Pr | CO | NO₂ | | | |
| 1041. | a-d | NH-*c*Pr | CO | CN | | | |
| 1042. | a-d | NH-*c*Pr | CO | Me | | | |
| 1043. | a-d | NH-*c*Pr | CO | MeO | | | |
| 1044. | a-d | NH-*c*Pr | CO | CHO | | | |
| 1045. | a-d | NH-*c*Pr | CO | NO₂ | | | |
| 1046. | a-d | NH-*i*Pr | CO | CN | | | |
| 1047. | a-d | NH-*i*Pr | CO | Me | | | |
| 1048. | a-d | NH-*i*Pr | CO | MeO | | | |
| 1049. | a-d | NH-*i*Pr | CO | CHO | | | |
| 1050. | a-d | NH-*i*Pr | CO | NO₂ | | | |
| 1051. | a-d | NH-CH₂CH=CH₂ | CO | CN | | | |
| 1052. | a-d | NH-CH₂CH=CH₂ | CO | Me | | | |
| 1053. | a-d | NH-CH₂CH=CH₂ | CO | MeO | | | |
| 1054. | a-d | NH-CH₂CH=CH₂ | CO | CHO | | | |
| 1055. | a-d | NH-CH₂CH=CH₂ | CO | NO₂ | | | |
| 1056. | a-d | CH₂CH=CH₂ | O | CN | gelbes Öl | | |
| 1057. | a-d | CH₂CH=CHMe | O | CN | | | |
| 1058. | a-d | CH₂CH=CMe₂ | O | CN | oranges Öl | | |
| 1059. | a-d | (E)-CH₂CH=CH-CF₃ | O | CN | 115 | | |
| 1060. | a-d | CH₂CCl=CH₂ | O | CN | 83 | | |
| 1061. | a-d | CH₂CBr=CHBr | O | CN | | | |
| 1062. | a-d | (Z)-CH₂CH=CCIMe | O | CN | semi-kristallin farblos | | |
| 1063. | a-d | CH₂CH=CH-CH₂OEt | O | CN | | | |
| 1064. | a-d | CH₂CH=CH-CF₂Br | O | CN | | | |
| 1065. | a-d | CH₂CH=CHBr | O | CN | | | |
| 1066. | a-d | CH₂CH=CHPh | O | CN | gelbes Harz | | |
| 1067. | a-d | CH₂CH=CHEt | O | CN | | | |
| 1068. | a-d | CH₂CMe=CH₂ | O | CN | braunes Harz | | |
| 1069. | a-d | CH₂CCl=CH-CF₃ | O | CN | | | |
| 1070. | a-d | (Z)-CH₂CH=CCl-CF₃ | O | CN | farbloses Harz | | |
| 1071. | a-d | CH₂CH=CCl₂ | O | CN | hell-braunes Harz | semi-kristallin braun | |
| 1072. | a-d | CH₂CH=CH₂ | O | Me | | | |
| 1073. | a-d | (E)-CH₂CH=CHMe | O | Me | farbloses Öl | | |
| 1074. | a-d | CH₂CH=CMe₂ | O | Me | | | |
| 1075. | a-d | CH₂CH=CH-CF₃ | O | Me | | | |
| 1076. | a-d | CH₂CCl=CH₂ | O | Me | | | |
| 1077. | a-d | CH₂CBr=CHBr | O | Me | | | |
| 1078. | a-d | CH₂CH=CClMe | O | Me | | | |
| 1079. | a-d | CH₂CH=CH-CH₂OEt | O | Me | | | |
| 1080. | a-d | CH₂CH=CH-CF₂Br | O | Me | | | |
| 1081. | a-d | CH₂CH=CHBr | O | Me | | | |
| 1082. | a-d | CH₂CH=CHPh | O | Me | | | |
| 1083. | a-d | CH₂CH=CHEt | O | Me | | | |
| 1084. | a-d | CH₂CMe=CH₂ | O | Me | | | |
| 1085. | a-d | CH₂Cl=CH-CF₃ | O | Me | | | |
| 1086. | a-d | CH₂CH=CCl-CF₃ | O | Me | | | |
| 1087. | a-d | CH₂CH=CCl₂ | O | Me | | | |
| 1088. | a-d | CH₂CH=CH₂ | O | MeO | | | |
| 1089. | a-d | CH₂CH=CHMe | O | MeO | | | |
| 1090. | a-d | CH₂CH=CMe₂ | O | MeO | | | |
| 1091. | a-d | CH₂CH=CH-CF₃ | O | MeO | | | |
| 1092. | a-d | CH₂CCl=CH₂ | O | MeO | | | |
| 1093. | a-d | CH₂CBr=CHBr | O | MeO | | | |
| 1094. | a-d | CH₂CH=CCIMe | O | MeO | | | |
| 1095. | a-d | CH₂CH=CH-CH₂OEt | O | MeO | | | |
| 1096. | a-d | CH₂CH=CH-CF₂Br | O | MeO | | | |
| 1097. | a-d | CH₂CH=CHBr | O | MeO | | | |
| 1098. | a-d | CH₂CH=CHPh | O | MeO | | | |
| 1099. | a-d | CH₂CH=CHEt | O | MeO | | | |
| 1100. | a-d | CH₂CMe=CH₂ | O | MeO | | | |
| 1101. | a-d | CH₂Cl=CH-CF₃ | O | MeO | | | |
| 1102. | a-d | CH₂CH=CCl-CF₃ | O | MeO | | | |
| 1103. | a-d | CH₂CH=CCl₂ | O | MeO | | | |
| 1104. | a-d | CH₂CH=CH₂ | O | CHO | | | |
| 1105. | a-d | CH₂CH=CHMe | O | CHO | | | |
| 1106. | a-d | CH₂CH=CMe₂ | O | CHO | | | |
| 1107. | a-d | CH₂CH=CH-CF₃ | O | CHO | | | |
| 1108. | a-d | CH₂CCl=CH₂ | O | CHO | | | |
| 1109. | a-d | CH₂CBr=CHBr | O | CHO | | | |
| 1110. | a-d | CH₂CH=CClMe | O | CHO | | | |
| 1111. | a-d | CH₂CH=CH-CH₂OEt | O | CHO | | | |
| 1112. | a-d | CH₂CH=CH-CF₂Br | O | CHO | | | |
| 1113. | a-d | CH₂CH=CHBr | O | CHO | | | |
| 1114. | a-d | CH₂CH=CHPh | O | CHO | | | |
| 1115. | a-d | CH₂CH=CHEt | O | CHO | | | |
| 1116. | a-d | CH₂CMe=CH₂ | O | CHO | | | |
| 1117. | a-d | CH₂Cl=CH-CF₃ | O | CHO | | | |
| 1118. | a-d | CH₂CH=CCl-CF₃ | O | CHO | | | |
| 1119. | a-d | CH₂CH=CCl₂ | O | CHO | | | |
| 1120. | a-d | CH₂CH=CH₂ | O | NO₂ | | | |
| 1121. | a-d | CH₂CH=CHMe | O | NO₂ | | | |
| 1122. | a-d | CH₂CH=CMe₂ | O | NO₂ | | | |
| 1123. | a-d | CH₂CH=CH-CF₃ | O | NO₂ | | | |
| 1124. | a-d | CH₂CCl=CH₂ | O | NO₂ | | | |
| 1125. | a-d | CH₂CBr=CHBr | O | NO₂ | | | |
| 1126. | a-d | CH₂CH=CClMe | O | NO₂ | | | |
| 1127. | a-d | CH₂CH=CH-CH₂OEt | O | NO₂ | | | |
| 1128. | a-d | CH₂CH=CH-CF₂Br | O | NO₂ | | | |
| 1129. | a-d | CH₂CH=CHBr | O | NO₂ | | | |
| 1130. | a-d | CH₂CH=CHPh | O | NO₂ | | | |
| 1131. | a-d | CH₂CH=CHEt | O | NO₂ | | | |
| 1132. | a-d | CH₂CMe=CH₂ | O | NO₂ | | | |
| 1133. | a-d | CH₂Cl=CH-CF₃ | O | NO₂ | | | |
| 1134. | a-d | CH₂CH=CCl-CF₃ | O | NO₂ | | | |
| 1135. | a-d | CH₂CH=CCl₂ | O | NO₂ | | | |
| 1136. | a-d | F | Bindung | CN | siehe Bsp. 2 | gelbes Öl | |
| 1137. | a-d | Cl | Bindung | CN | | | |
| 1138. | a-d | Br | Bindung | CN | | | |
| 1139. | a-d | I | Bindung | CN | | | |
| 1140. | a-d | CN | Bindung | CN | siehe Bsp. 6 | braunes Wachs | 131 |
| 1141. | a-d | F | Bindung | Me | | | |
| 1142. | a-d | Cl | Bindung | Me | | | |
| 1143. | a-d | Br | Bindung | Me | | | |
| 1144. | a-d | I | Bindung | Me | | | |
| 1145. | a-d | CN | Bindung | Me | 92 | | |
| 1146. | a-d | F | Bindung | MeO | | | |
| 1147. | a-d | Cl | Bindung | MeO | | | |
| 1148. | a-d | Br | Bindung | MeO | | | |
| 1149. | a-d | I | Bindung | MeO | | | |
| 1150. | a-d | CN | Bindung | MeO | | | |
| 1151. | a-d | F | Bindung | CHO | | | |
| 1152. | a-d | Cl | Bindung | CHO | | | |
| 1153. | a-d | Br | Bindung | CHO | | | |
| 1154. | a-d | I | Bindung | CHO | | | |
| 1155. | a-d | CN | Bindung | CHO | | | |
| 1156. | a-d | F | Bindung | NO₂ | 78 | | |
| 1157. | a-d | Cl | Bindung | NO₂ | | | |
| 1158. | a-d | Br | Bindung | NO₂ | | | |
| 1159. | a-d | I | Bindung | NO₂ | | | |
| 1160. | a-d | CN | Bindung | NO₂ | | | |
| 1161. | a-d | 3-CF₃-Pyrazol-1-yl | Bindung | CN | siehe Bsp. 5 | 110 | |
| 1162. | a-d | 3-CF₃-Pyrazol-1-yl | Bindung | Me | | | |
| 1163. | a-d | 3-CF₃-Pyrazol-1-yl | Bindung | MeO | | | |
| 1164. | a-d | 3-CF₃-Pyrazol-1-yl | Bindung | CHO | | | |
| 1165. | a-d | 3-CF₃-Pyrazol-1-yl | Bindung | NO₂ | 128 | | |
| 1166. | a-d | CH₂CH₂OMe | O | CN | 72 | | |
| 1167. | a-d | CH₂CH₂OEt | O | CN | farbloses Öl | | |
| 1168. | a-d | CH₂CH₂O*n*Pr | O | CN | | | |
| 1169. | a-d | CH₂CH₂O*i*Pr | O | CN | farbloses Wachs | 46 | |
| 1170. | a-d | CH₂CH₂OCH₂CH₂O Me | O | CN | | | |
| 1171. | a-d | CH₂CH₂OCH₂CH₂O Et | O | CN | farbloses Öl | | |
| 1172. | a-d | CH₂CH₂SCF₃ | O | CN | farbloses Öl | | |
| 1173. | a-d | CH₂CH₂CH₂SCF₃ | O | CN | | | |
| 1174. | a-d | CH₂CH(OEt)₂ | O | CN | gelbes Öl | hellgelbes Harz | |
| 1175. | a-d | CH₂CH(OMe)₂ | O | CN | 84 | | |
| 1176. | a-d | CH₂CH₂OCF₃ | O | CN | | | |
| 1177. | a-d | CH₂CH₂CH₂OMe | O | CN | | | |
| 1178. | a-d | CH₂CH₂CH₂OEt | O | CN | | | |
| 1179. | a-d | CH₂CH₂CH₂OCF₃ | O | CN | | | |
| 1180. | a-d | tetrahydrofur-2-yl | O-CH₂ | CN | farbloses Öl | | |
| 1181. | a-d | tetrahydropyran-2-yl | O-CH₂ | CN | farbloses Öl | | |
| 1182. | a-d | 2,2-dimethyl-1,3-dioxolan-4-yl | O-CH₂ | CN | farbloses Öl | 88 | |
| 1183. | a-d | CH₂CH₂OMe | O | Me | | | |
| 1184. | a-d | CH₂CH₂OEt | O | Me | | | |
| 1185. | a-d | CH₂CH₂O*n*Pr | O | Me | | | |
| 1186. | a-d | CH₂CH₂O*i*Pr | O | Me | | | |
| 1187. | a-d | CH₂CH₂OCH₂CH₂O Me | O | Me | | | |
| 1188. | a-d | CH₂CH₂OCH₂CH₂O Et | O | Me | | | |
| 1189. | a-d | CH₂CH₂SCF₃ | O | Me | | | |
| 1190. | a-d | CH₂CH₂CH₂SCF₃ | O | Me | | | |
| 1191. | a-d | CH₂CH(OEt)₂ | O | Me | | | |
| 1192. | a-d | CH₂CH(OMe)₂ | O | Me | | | |
| 1193. | a-d | CH₂CH₂OCF₃ | O | Me | | | |
| 1194. | a-d | CH₂CH₂CH₂OMe | O | Me | | | |
| 1195. | a-d | CH₂CH₂CH₂OEt | O | Me | | | |
| 1196. | a-d | CH₂CH₂CH₂OCF₃ | O | Me | | | |
| 1197. | a-d | tetrahydrofur-2-yl | O-CH₂ | Me | | | |
| 1198. | a-d | tetrahydropyran-2-yl | O-CH₂ | Me | | | |
| 1199. | a-d | 2,2-dimethyl-1,3-dioxolan-4-yl | O-CH₂ | Me | | | |
| 1200. | a-d | CH₂CH₂OMe | O | MeO | | | |
| 1201. | a-d | CH₂CH₂OEt | O | MeO | | | |
| 1202. | a-d | CH₂CH₂O*n*Pr | O | MeO | | | |
| 1203. | a-d | CH₂CH₂O*i*Pr | O | MeO | | | |
| 1204. | a-d | CH₂CH₂OCH₂CH₂O Me | O | MeO | | | |
| 1205. | a-d | CH₂CH₂OCH₂CH₂O Et | O | MeO | | | |
| 1206. | a-d | CH₂CH₂SCF₃ | O | MeO | | | |
| 1207. | a-d | CH₂CH₂CH₂SCF₃ | O | MeO | | | |
| 1208. | a-d | CH₂CH(OEt)₂ | O | MeO | | | |
| 1209. | a-d | CH₂CH(OMe)₂ | O | MeO | | | |
| 1210. | a-d | CH₂CH₂OCF₃ | O | MeO | | | |
| 1211. | a-d | CH₂CH₂CH₂OMe | O | MeO | | | |
| 1212. | a-d | CH₂CH₂CH₂OEt | O | MeO | | | |
| 1213. | a-d | CH₂CH₂CH₂OCF₃ | O | MeO | | | |
| 1214. | a-d | tetrahydrofur-2-yl | O-CH₂ | MeO | | | |
| 1215. | a-d | tetrahydropyran-2-yl | O-CH₂ | MeO | | | |
| 1216. . | a-d | 2,2-dimethyl-1,3- dioxolan-4-yl | O-CH₂ | MeO | | | |
| 1217. | a-d | CH₂CH₂OMe | O | CHO | | | |
| 1218. | a-d | CH₂CH₂OEt | O | CHO | | | |
| 1219. | a-d | CH₂CH₂O*n*Pr | O | CHO | | | |
| 1220. | a-d | CH₂CH₂O*i*Pr | O | CHO | | | |
| 1221. | a-d | CH₂CH₂OCH₂CH₂O Me | O | CHO | | | |
| 1222. | a-d | CH₂CH₂OCH₂CH₂O Et | O | CHO | | | |
| 1223. | a-d | CH₂CH₂SCF₃ | O | CHO | | | |
| 1224. | a-d | CH₂CH₂CH₂SCF₃ | O | CHO | | | |
| 1225. | a-d | CH₂CH(OEt)₂ | O | CHO | | | |
| 1226. | a-d | CH₂CH(OMe)₂ | O | CHO | | | |
| 1227. | a-d | CH₂CH₂OCF₃ | O | CHO | | | |
| 1228. | a-d | CH₂CH₂CH₂OMe | O | CHO | | | |
| 1229. | a-d | CH₂CH₂CH₂OEt | O | CHO | | | |
| 1230. | a-d | CH₂CH₂CH₂OCF₃ | O | CHO | | | |
| 1231. | a-d | tetrahydrofur-2-yl | O-CH₂ | CHO | | | |
| 1232. | a-d | tetrahydropyran-2-yl | O-CH₂ | CHO | | | |
| 1233. | a-d | 2,2-dimethyl-1,3-dioxolan-4-yl | O-CH₂ | CHO | | | |
| 1234. | a-d | CH₂CH₂OMe | O | NO₂ | | | |
| 1235. | a-d | CH₂CH₂OEt | O | NO₂ | | | |
| 1236. | a-d | CH₂CH₂O*n*Pr | O | NO₂ | | | |
| 1237. | a-d | CH₂CH₂O*i*Pr | O | NO₂ | hellgelbes Öl | | |
| 1238. | a-d | CH₂CH₂OCH₂CH₂O Me | O | NO₂ | | | |
| 1239. | a-d | CH₂CH₂OCH₂CH₂O Et | O | NO₂ | | | |
| 1240. | a-d | CH₂CH₂SCF₃ | O | NO₂ | | | |
| 1241. | a-d | CH₂CH₂CH₂SCF₃ | O | NO₂ | | | |
| 1242. | a-d | CH₂CH(OEt)₂ | O | NO₂ | hellgelbes Öl | | |
| 1243. | a-d | CH₂CH(OMe)₂ | O | NO₂ | hellgelbes Öl | | |
| 1244. | a-d | CH₂CH₂OCF₃ | O | NO₂ | | | |
| 1245. | a-d | CH₂CH₂CH₂OMe | O | NO₂ | | | |
| 1246. | a-d | CH₂CH₂CH₂OEt | O | NO₂ | | | |
| 1247. | a-d | CH₂CH₂CH₂OCF₃ | O | NO₂ | | | |
| 1248. | a-d | tetrahydrofur-2-yl | O-CH₂ | NO₂ | 99 | | |
| 1249. | a-d | tetrahydropyran-2-yl | O-CH₂ | NO₂ | | | |
| 1250. | a-d | 2,2-dimethyl-1,3-dioxolan-4-yl | O-CH₂ | NO₂ | hellgelbes Harz | | |
| 1251. | a-d | NH₂ | CO | CONH₂ | 271 - 272 | | 291 |
| 1252. | a-d | Me | O | CN | 60 | oranges Öl | |
| 1253. | a-d | Me | O | Me | | | |
| 1254. | a-d | Me | O | MeO | | | |
| 1255. | a-d | Me | O | CHO | | | |
| 1256. | a-d | Me | O | NO₂ | | | |
| 1257. | a-d | Et | O | CN | weißes Harz | | |
| 1258. | a-d | Et | O | Me | | | |
| 1259. | a-d | Et | O | MeO | | | |
| 1260. | a-d | Et | O | CHO | | | |
| 1261. | a-d | Et | O | NO₂ | | | |
| 1262. | a-d | *i*Pr | O | CN | | | |
| 1263. | a-d | *i*Pr | O | Me | | | |
| 1264. | a-d | *i*Pr | O | MeO | | | |
| 1265. | a-d | *i*Pr | O | CHO | | | |
| 1266. | a-d | *i*Pr | O | NO₂ | | | |
| 1267. | a-d | *n*Pr | O | CN | | | |
| 1268. | a-d | *n*Pr | O | Me | | | |
| 1269. | a-d | *n*Pr | O | MeO | | | |
| 1270. | a-d | *n*Pr | O | CHO | | | |
| 1271. | a-d | *n*Pr | O | NO₂ | | | |
| 1272. | a-d | *n*Bu | O | CN | | | |
| 1273. | a-d | *n*Bu | O | Me | | | |
| 1274. | a-d | *n*Bu | O | MeO | | | |
| 1275. | a-d | *n*Bu | O | CHO | | | |
| 1276. | a-d | *n*Bu | O | NO₂ | | | |
| 1277. | a-d | CH₂CF₃ | O | CN | | | |
| 1278. | a-d | CH₂CF₃ | O | Me | | | |
| 1279. | a-d | CH₂CF₃ | O | MeO | | | |
| 1280. | a-d | CH₂CF₃ | O | CHO | | | |
| 1281. | a-d | CH₂CF₃ | O | NO₂ | | | |
| 1282. | a-d | CH₂ CF₂ CF₃ | O | CN | 78 | | |
| 1283. | a-d | CH₂ CF₂ CF₃ | O | Me | | | |
| 1284. | a-d | CH₂ CF₂ CF₃ | O | MeO | | | |
| 1285. | a-d | CH₂ CF₂ CF₃ | O | CHO | | | |
| 1286. | a-d | CH₂ CF₂ CF₃ | O | NO₂ | | | |
| 1287. | a-d | CH₂ CH₂ CF₃ | O | CN | farbloses Harz | | |
| 1288. | a-d | CH₂ CH₂ CF₃ | O | Me | | | |
| 1289. | a-d | CH₂ CH₂ CF₃ | O | MeO | | | |
| 1290. | a-d | CH₂ CH₂ CF₃ | O | CHO | | | |
| 1291. | a-d | CH₂ CH₂ CF₃ | O | NO₂ | | | |
| 1292. | a-d | CH₂CH₂Cl | O | CN | | | |
| 1293. | a-d | CH₂CH₂Cl | O | Me | | | |
| 1294. | a-d | CH₂CH₂Cl | O | MeO | | | |
| 1295. | a-d | CH₂CH₂Cl | O | CHO | | | |
| 1296. | a-d | CH₂CH₂Cl | O | NO₂ | | | |
| 1297. | a-d | CH₂CH₂SMe | O | CN | | | |
| 1298. | a-d | CH₂CH₂SMe | O | Me | | | |
| 1299. | a-d | CH₂CH₂SMe | O | MeO | | | |
| 1300. | a-d | CH₂CH₂SMe | O | CHO | | | |
| 1301. | a-d | CH₂CH₂SMe | O | NO₂ | | | |
| 1302. | a-d | CH₂ CH₂ CH₂ Cl | O | CN | | | |
| 1303. | a-d | CH₂ CH₂CH₂Cl | O | Me | | | |
| 1304. | a-d | CH₂ CH₂CH₂Cl | O | MeO | | | |
| 1305. | a-d | CH₂ CH₂ CH₂ Cl | O | CHO | | | |
| 1306. | a-d | CH₂ CH₂ CH₂ Cl | O | NO₂ | | | |
| 1307. | a-d | CH₂ CH=CH₂ | CO | CN | | | |
| 1308. | a-d | CH₂CH=CH₂ | CO | Me | | | |
| 1309. | a-d | CH₂CH=CH₂ | CO | MeO | | | |
| 1310. | a-d | CH₂ CH=CH₂ | CO | CHO | | | |
| 1311. | a-d | CH₂ CH=CH₂ | CO | NO₂ | | | |
| 1312. | a-d | Me | CO | CN | | | |
| 1313. | a-d | Me | CO | Me | | | |
| 1314. | a-d | Me | CO | MeO | | | |
| 1315. | a-d | Me | CO | CHO | | | |
| 1316. | a-d | Me | CO | NO₂ | | | |
| 1317. | a-d | Et | CO | CN | | | |
| 1318. | a-d | Et | CO | Me | | | |
| 1319. | a-d | Et | CO | MeO | | | |
| 1320. | a-d | Et | CO | CHO | | | |
| 1321. | a-d | Et | CO | NO₂ | | | |
| 1322. | a-d | CH₂ CH₂CHMe₂ | CO | CN | | | |
| 1323. | a-d | CH₂ CH₂CHMe₂ | CO | Me | | | |
| 1324. | a-d | CH₂ CH₂CHMe₂ | CO | MeO | | | |
| 1325. | a-d | CH₂ CH₂CHMe₂ | CO | CHO | | | |
| 1326. | a-d | CH₂ CH₂CHMe₂ | CO | NO₂ | | | |
| 1327. | a-d | Ph | CO-CH₂ | CN | | | |
| 1328. | a-d | Ph | CO-CH₂ | Me | | | |
| 1329. | a-d | Ph | CO-CH₂ | MeO | | | |
| 1330. | a-d | Ph | CO-CH₂ | CHO | | | |
| 1331. | a-d | Ph | CO-CH₂ | NO₂ | | | |
| 1332. | a-d | c-Pentyl | CO | CN | farbloses Harz | | |
| 1333. | a-d | c-Pentyl | CO | Me | | | |
| 1334. | a-d | c-Pentyl | CO | MeO | | | |
| 1335. | a-d | c-Pentyl | CO | CHO | | | |
| 1336. | a-d | c-Pentyl | CO | NO₂ | | | |
| 1337. | a-d | iPr | CO | CN | farbloses Harz | | |
| 1338. | a-d | iPr | CO | Me | | | |
| 1339. | a-d | iPr | CO | MeO | | | |
| 1340. | a-d | iPr | CO | CHO | | | |
| 1341. | a-d | iPr | CO | NO₂ | | | |
| 1342. | a-d | cPr | CO | CN | | | |
| 1343. | a-d | cPr | CO | Me | | | |
| 1344. | a-d | cPr | CO | MeO | | | |
| 1345. | a-d | cPr | CO | CHO | | | |
| 1346. | a-d | cPr | CO | NO₂ | | | |
| 1347. | a-d | cBu | CO | CN | | | |
| 1348. | a-d | cBu | CO | Me | | | |
| 1349. | a-d | cBu | CO | MeO | | | |
| 1350. | a-d | cBu | CO | CHO | | | |
| 1351. | a-d | cBu | CO | NO₂ | | | |
| 1352. | a-d | nPr | CO | CN | | | |
| 1353. | a-d | nPr | CO | Me | | | |
| 1354. | a-d | nPr | CO | MeO | | | |
| 1355. | a-d | nPr | CO | CHO | | | |
| 1356. | a-d | nPr | CO | NO₂ | | | |
| 1357. | a-d | nBu | CO | CN | | | |
| 1358. | a-d | nBu | CO | Me | | | |
| 1359. | a-d | nBu | CO | MeO | | | |
| 1360. | a-d | nBu | CO | CHO | | | |
| 1361. | a-d | nBu | CO | NO₂ | | | |
| 1362. | a-d | Ph | CO | CN | | | |
| 1363. | a-d | Ph | CO | Me | | | |
| 1364. | a-d | Ph | CO | MeO | | | |
| 1365. | a-d | Ph | CO | CHO | | | |
| 1366. | a-d | Ph | CO | NO₂ | | | |
| 1367. | a-d | 1-CH₃-3-CF₃-pyrazol-5-yl | O | COOMe | farbloses Wachs | | |
| 1368. | a-d | Ph | O-CH₂ | CN | hellgelbes Harz | | |
| 1369. | a-d | Ph | O-CH₂ | Me | | | |
| 1370. | a-d | Ph | O-CH₂ | MeO | | | |
| 1371. | a-d | Ph | O-CH₂ | CHO | | | |
| 1372. | a-d | Ph | O-CH₂ | NO₂ | | | |
| 1373. | a-d | 4-F-Ph | O-CH₂ | CN | farbloses Öl | | |
| 1374. | a-d | 4-F-Ph | O-CH₂ | Me | | | |
| 1375. | a-d | 4-F-Ph | O-CH₂ | MeO | | | |
| 1376. | a-d | 4-F-Ph | O-CH₂ | CHO | | | |
| 1377. | a-d | 4-F-Ph | O-CH₂ | NO₂ | | | |
| 1378. | a-d | 2,4-F₂-Ph | O-CH₂ | CN | gelbes | | |
| 1379. | a-d | 2,4-F₂-Ph | O-CH₂ | Me | | | |
| 1380. | a-d | 2,4-F₂-Ph | O-CH₂ | MeO | | | |
| 1381. | a-d | 2,4-F₂-Ph | O-CH₂ | CHO | | | |
| 1382. | a-d | 2,4-F₂-Ph | O-CH₂ | NO₂ | | | |
| 1383. | a-d | 3,4-F₂-Ph | O-CH₂ | CN | farbloses Öl | | |
| 1384. | a-d | 3,4-F₂-Ph | O-CH₂ | Me | | | |
| 1385. | a-d | 3,4-F₂-Ph | O-CH₂ | MeO | | | |
| 1386. | a-d | 3,4-F₂-Ph | O-CH₂ | CHO | | | |
| 1387. | a-d | 3,4-F₂-Ph | O-CH₂ | NO₂ | | | |
| 1388. | a-d | 2-Me-Ph | O-CH₂ | CN | 113 | | |
| 1389. | a-d | 2-Me-Ph | O-CH₂ | Me | | | |
| 1390. | a-d | 2-Me-Ph | O-CH₂ | MeO | | | |
| 1391. | a-d | 2-Me-Ph | O-CH₂ | CHO | | | |
| 1392. | a-d | 2-Me-Ph | O-CH₂ | NO₂ | | | |
| 1393. | a-d | 3-CF₃-Ph | O-CH₂ | CN | farbloses Öl | | |
| 1394. | a-d | 3-CF₃-Ph | O-CH₂ | Me | | | |
| 1395. | a-d | 3-CF₃-Ph | O-CH₂ | MeO | | | |
| 1396. | a-d | 3-CF₃-Ph | O-CH₂ | CHO | | | |
| 1397. | a-d | 3-CF₃-Ph | O-CH₂ | NO₂ | | | |
| 1398. | a-d | 3-CF₃-Phenyl | O | CONH₂ | 155 | | |
| 1399. | a-d | 3-Cl-4-F-Phenyl | O | CN | gelbes Harz | | |
| 1400. | a-d | 3-CF₃-4-F-Phenyl | O | CN | gelbes Harz | | |
| 1401. | a-d | 3-CF₃-4-Cl-Phenyl | O | CN | gelber Wachsartiger Feststoff | | |
| 1402. | a-d | 3,4-Me₂-Phenyl | O | CN | gelbes Harz | | |
| 1403. | a-d | 3,4,5-Me₃-Phenyl | O | CN | gelbes Harz | | |
| 1404. | a-d | 1-CH₃-3-CHF₂-pyrazol-5-yl | O | CN | hellgelbes Harz | | |
| 1405. | a-d | 1-CH₃-3-CF₃-pyrazol-5-yl | O | COOMe | farbloses Öl | | |
| 1406. | a-d | 1-CH₃-3-CF₃-pyrazol-5-yl | O | Cl | hellgelbes Öl | | |
| 1407. | a-d | 1-CH₂-3-CF₃-pyrazol-5-yl | O | NH₂ | 117 | | |
| 1408. | a-d | 4,5-Cl₂-imidazol-1-yl | Bindung | CN | farbloses Öl | | |
| 1409. | a-d | 1-CH₃-3-CF₃-pyrazol-5-yl | O | COOH | gelbes Harz | | |
| 1410. | a-d | 1-CH₃-3-CF₃-pyrazol-5-yl | O | COMe | 116 | | |
| 1411. | a-d | 1-CH₃-3-CF₃-pyrazol-5-yl | O | F | 73 | | |
| 1412. | a-d | 1-CH₃-3-CF₃-pyrazol-5-yl | O | C(=CH₂) Me | semi-kristallin weiß | | |
| 1413. | a-d | 1-CH₃-3-CF₃-pyrazol-5-yl | O | CSNH₂ | 159 | | |
| 1414. | a-d | 1-CH₃-3-t-Bupyrazol-5-yl | O | CN | farbloses Öl | | |
| 1415. | a-d | NH-4-F-Ph | CO | F | weiße Kristalle | | |
| 1416. | a-d | NH-2,4-F₂-Phenyl | CO | F | weiße Kristalle | | |
| 1417. | a-d | NH₂ | CH₂ | F | gelbes Harz | | |
| 1418. | a-d | NH₂ | CH₂ | CF₃ | hellgelbes Harz | | |
| 1419. | a-d | 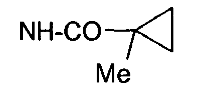 | CH₂ | F | farbloses Öl | | |
| 1420. | a-d | NH-CO-tBu | CH₂ | F | gelbes Öl | | |
| 1421. | a-d | NH-CO-Et | CH₂ | F | 75 | | |
| 1422. | a-d | NH-COOMe | CH₂ | F | gelbes Öl | | |
| 1423. | a-d | NH-CO-CF₃ | CH₂ | F | gelbes Öl | | |
| 1424. | a-d | NH-CO-iPr | CH₂ | F | 102 | | |
| 1425. | a-d | NH-CO-cPr | CH₂ | F | 104 | | |
| 1426. | a-d | NH-COOMe | CH₂ | CF₃ | 78 | | |
| 1427. | a-d | NH-CO-CF₃ | CH₂ | CF₃ | gelbes Harz | | |
| 1428. | a-d | 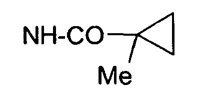 | CH₂ | CF₃ | 137 | | |
| 1429. | a-d | NH-COiPr | CH₂ | CF₃ | 123 | | |
| 1430. | a-d | NH-COcPr | CH₂ | CF₃ | 118 | | |
| 1431. | a-d | NH-COOMe | CH₂ | OMe | farbloses Harz | | |
| 1432. | a-d | 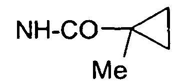 | CH₂ | OMe | 89 | | |
| 1433. | a-d | NH-CH₂CH₂SO₂Me | CO | F | 132 | | |
| 1434. | a-d | NH₂ | CO | CN | 240 | 135 | |
| 1435. | a-d | NH₂ | CO | F | 186 | | |
| 1436. | a-d | (E)-CH₂CH=CH-CH₂Cl | O | CN | farbloses Öl | | |
| 1437. | a-d | (Z)-CH₂CH=CH-CH₂Cl | O | CN | semi-kristallin, farblos | | |
| 1438. | a-d | (E)-CH₂CH=CHCl | O | CN | | braunes Harz | |
| 1439. | a-d | CH₂-C(OCH₂OMe)= CH₂ | O | CN | farbloses Öl | | |
| 1440. | a-d | H | O | CN | 155 | | |
| 1441. | a-d | SO₂CF₃ | O | CN | hellgelbes Öl | | |
| 1442. | a-d | (E)-CH₂CH=CHCl | O | CN | gelbes Harz | | |
| 1443. | a-d | (Z)-CH₂CH=CHCl | O | CN | gelbes Harz | | |
| 1444. | a-d | (E)-CH₂CH=CCIMe | O | CN | farbloses Harz | | |
| 1445. | a-d | (Z)-CH₂CCl=CHCl | O | CN | beiges Harz | | |
| 1446. | a-d | (E)-CH₂CCl=CHCl | O | CN | braunes Harz | | |
| 1447. | a-d | CF₃ | Bindung | CN | 86 | | |
| 1448. | a-d | F | Bindung | Cl | hellgelbes Öl | | |
| 1449. | a-d | F | Bindung | Br | gelbes Öl | | |
| 1450. | a-d | F | Bindung | F | farbloses Öl | | |
| 1451. | a-d | CH₂CH₂C(Me)₂OMe | O | CN | farbloses Harz | | |
| 1452. | a-d | CH₂Ph | O | CN | 88 | | |
| 1453. | a-d | Cl-NO₂-imidazol-1-yl | Bindung | CN | 220 | | |
| 1454. | a-d | 4-CF₃-imidazol-1-yl | Bindung | CN | farbloses Öl | | |
| 1455. | a-d | 4-CF₃-Pyrazol-1-yl | Bindung | CN | 130 | 112 | |
| 1456. | a-d | 4-CF₃-Pyrazol-1-yl | Bindung | CONH₂ | farbloses | | |
| 1457. | a-d | pyholyl | Bindung | CN | oranges Öl | | |
| 1458. | a-d | Imidazolyl | Bindung | CN | farbloses Öl | | |
| 1459. | a-d | 4-Me-Imidazol-1-yl | Bindung | CN | farbloses Öl | | |
| 1460. | a-d | 4-Br-pyrazol-1-yl | Bindung | CN | semi-kristallin, weiß | | |
| 1461. | a-d | Pyrazol-1-yl | Bindung | CN | farbloses Öl | | |
| 1462. | a-d | 1,2,4-Triazol-1-yl | Bindung | CN | 161 | | |
| 1463. | a-d | 4-Br-3,5-dimethyl-pyrazol-1-yl | Bindung | CN | 90 | | |
| 1464. | a-d | 3-MeOOC-midazol-1-yl | Bindung | CN | 214 | | |
| 1465. | a-d | CH₂CH₂OPh | O | CN | farbloses Harz | | |
| 1466. | a-d | CH₂CH₂CH(Me) (OMe) | O | CN | hellgelbes Öl | | |
| 1467. | a-d | CH₂CH₂CH(OEt)₂ | O | CN | farbloses Harz | | |
| 1468. | a-d | CH₂CH₂NMe₂ | O | CN | hellbraunes Öl | | |
| 1469. | a-d | CH₂CH₂SMe | O | CN | farbloses Harz | | |
| 1470. | a-d | 1,3-dioxolan-4-yl | O-CH₂ | CN | braunes Harz | | |
| 1471. | a-d | CH₂CH(OEt)₂ | O | COMe | braunes Öl | | |
| 1472. | a-d | Me | S | CN | 100 | | |
| 1473. | a-d | Me | SO | CN | 116 | | |
| 1474. | a-d | Me | SO₂ | CN | 140 | | |
| 1475. | a-d | CH₂CHO | O | CN | gelbes Öl | | |
| 1476. | a-d | 1,3-dioxolan-2-yl | O-CH₂ | CN | farbloses Harz | | |
| 1477. | a-d | 4-Ethyl-1,3-dioxolan-2-yl | O-CH₂ | CN | 88 | | |
| 1478. | a-d | 1,3-dioxan-2-yl | O-CH₂ | CN | 137 | | |
| 1479. | a-d | trans-5-Methoxy-1,3-dioxan-2-yl | O-CH₂ | CN | 165 | | |
| 1480. | a-d | cis-5-Methoxy-1,3-dioxan-2-yl | O-CH₂ | CN | 109 | | |
| 1481. | a-d | 4-Fluormethyl-1,3-dioxolan-2-yl | O-CH₂ | CN | weißes Harz | | |
| 1482. | a-d | 1,3-Dioxopen-2-yl | O-CH₂ | CN | farbloses Harz | | |
| 1483. | a-d | cis-4,6-Dimethyl-1,3-dioxan-2-yl | O-CH₂ | CN | 124 | | |
| 1484. | a-d | trans-4,6-Dimethyl-1,3-dioxan-2-yl | O-CH₂ | CN | 118 | | |
| 1485. | a-d | 5,5-Dimethyl-1,3-dioxan-2-yl | O-CH₂ | CN | 122 | | |
| 1486. | a-d | CH₂CH₂CH₂CF₃ | O | CN | farbloses Harz | | |
| 1487. | a-d | CH₂CH(OMe)₂ | O | F | hellgelbes Öl | | |
| 1488. | a-d | tetrahydrofur-2-yl | O-CH₂ | F | gelbes Harz | | |
| 1489. | a-d | 2,2-dimethyl-1,3-dioxolan-4-yl | O-CH₂ | F | farbloses Öl | | |
| 1490. | a-d | CH₂CH₂CH₂CF₃ | O | NO₂ | hellgelbes | | |
| 1491. | a-d | CH₂CH₂CH₂CF₃ | O | F | hellgelbes Öl | | |
| 1492. | a-d | CH₂CH₂-cPr | O | CN | semi-kristallin farblos | | |
| 1493. | a-d | Me | CO | F | 60 | | |
| 1494. | a-d | CH₂CH(OEt)₂ | O | CSNH₂ | 92 | | |
| 1495. | a-d | Me | O | CSNH₂ | 121 | | |
| 1496. | a-d | 3-CF₃-Pyrazol | Bindung | CSNH₂ | 101 | | |
| 1497. | a-d | (E)-CH₂CH=CCIMe | O | CSNH₂ | 123 | | |
| 1498. | a-d | (E)-CH₂CH=CCl (CF₃) | O | CSNH₂ | 109 | | |
| 1499. | a-d | (E)-CH₂CH=CHCF₃ | O | CSNH₂ | 109 | | |
| 1500. | a-d | 3-Me-4-F-Phenyl | Bindung | CN | 123 | | |
| 1501. | a-d | 4-CF₃O-Phenyl | Bindung | CN | 74 | | |
| 1502. | a-d | 4-MeO-Phenyl | Bindung | CN | 131 | | |
| 1503. | a-d | 3,4-(MeO)₂Phenyl | Bindung | CN | 132 | | |
| 1504. | a-d | CH=CH-(4-F-Phenyl) | Bindung | CN | 124 | | |
| 1505. | a-d | 4-CH₃-Phenyl | Bindung | CN | 82 | | |
| 1506. | a-d | 4-CN-Phenyl | Bindung | CN | 131 | | |
| 1507. | a-d | 4-F-Phenyl | Bindung | CN | 96 | | |
| 1508. | a-d | 4-Cl-Phenyl | Bindung | CN | 104 | | |
| 1509. | a-d | 3,4-F₂-Phenyl | Bindung | CN | 109 | | |
| 1510. | a-d | 3-Cl,4-F-Phenyl | Bindung | CN | 120 | | |
| 1511. | a-d | 3,4-Cl₂-Phenyl | Bindung | CN | semi-kristallin bräunlich | | |
| 1512. | a-d | 4-CF₃-Phenyl | Bindung | CN | farbloses | | |

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I),
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I),
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen Nr. 62a, 73d, 74c, 75a, 76a, 77a, 397a, 1136a, 1136c, 1140a,1140c, 1140d, 1156a, 1161 a, 1169a, 1171a,1177a,1180a,1182a und andere Verbindungen aus Tabelle 1 sehr gute herbizide Wirkung gegen Schadpflanzen wie Sinapis alba, Echinochloa crus-galli, Lolium multiflorum, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 2 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen Nr. 62a, 73d, 74c, 75a, 76a, 77a, 397a, 1136a, 1136c, 1140a,1140c, 1140d, 1156a, 1161 a, 1169a, 1171a, 1177a, 1180a, 1182a und andere Verbindungen aus Tabelle 1 sehr gute herbizide Wirkung gegen Schadpflanzen wie Sinapis alba, Echinochloa crus-galli, Lolium multiflorum, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Nachlaufverfahren bei einer Aufwandmenge von 2 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Verbindungen in unterschiedlichen Dosierungen besprüht. Vier bis fünf Wochen nach der Applikation und Standzeit im Gewächshaus wurde mittels optischer Bonitur festgestellt, daß erfindungsgemäße Verbindungen zweikeimblättrige Kulturen wie Soja, Baumwolle, Raps, Zuckerrüben oder Kartoffeln im Vor- und Nachauflaufverfahren selbst bei hohen Wirkstoffdosierungen ungeschädigt ließen. Einige Substanzen schonten darüber hinaus auch Gramineen-Kulturen wie Gerste, Weizen, Roggen, Hirsen, Mais oder Reis. Die erfindungsgemäßen Verbindungen zeigen teilweise eine hohe Selektivität und eignen sich deshalb zur Bekämpfung von unerwünschten Pflanzenwuchs in landwirtschaftlichen Kulturen.

## Patentansprüche

1. Verbindung der Formel (I) und/oder deren Salze 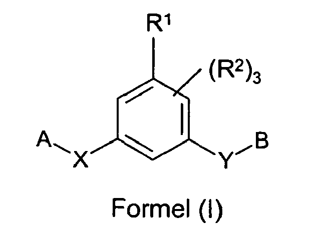 worin A einen substituierten Pyrazolyl-, Thienyl- oder Pyridylrest der Formeln (la), (Ic) oder (Id) darstellt, 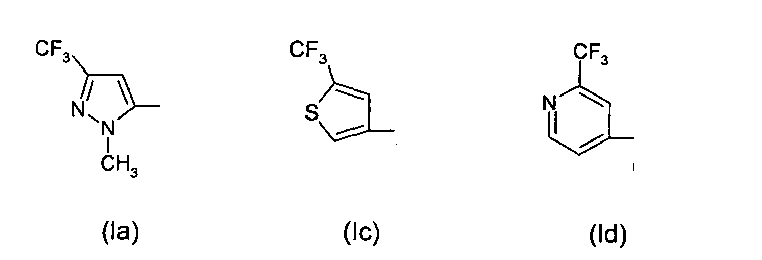 worin
X O, S oder CH₂ bedeutet,
R¹ Hydroxy, Halogen, CN, NC, CHO, CO(C₁-C₈)Alkyl, wobei die Alkylgruppe unsubstituiert oder substituiert ist, CONH₂, CSNH₂, Nitro, SF₅, (C₁-C₈)Alkyl, (C₂-C₈)Alkenyl, (C₂-C₈)Alkinyl, (C₁-C₈)Alkoxy, [(C₁-C₈)Alkyl]-carbonyl oder (C₁-C₈)Alkylsulfonyl ist, wobei jeder der letztgenannten sechs Reste unsubstituiert oder substituiert ist, oder
S(O)ₚ-R³ ist, wobei
p = 1 oder 2 und
R³ (C₁-C₈)Alkyl, (C₁-C₈)Haloalkyl oder NR⁴R⁵ bedeutet, wobei R⁴,R⁵ unabhängig voneinander gleich oder verschieden H, (C₁-C₈)Alkyl, (C₂-C₈)Alkenyl, (C₇-C₁₀)Arylalkyl, (C₇-C₁₀)Alkylaryl oder (C₆-C₁₀)Aryl sind, wobei jeder der letztgenannten fünf Reste unsubstituiert oder substituiert ist,
oder NR⁴R⁵ bedeutet, wobei R⁴,R⁵ unabhängig voneinander gleich oder verschieden H, (C₁-C₈)Alkyl, (C₂-C₈)Alkenyl, (C₇-C₁₀)Arylalkyl, (C₇-C₁₀)Alkylaryl oder (C₆-C₁₀)Aryl sind, wobei jeder der letztgenannten fünf Reste unsubstituiert oder substituiert ist,
oder R¹ eine Gruppe der Formel 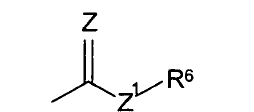 ist, wobei R⁶ (C₁-C₈)Alkyl ist, welches unsubstituiert oder substituiert ist,
Z = O oder S bedeutet, und
Z¹ = O oder S bedeutet,
R² gleich oder verschieden H, Halogen, CN oder (C₁-C₈)Alkyl sind, welche unsubstituiert oder substituiert sind,
Y O-(CR⁸R⁹)_{q}, S(O)_{q}, NH, CO(CR⁸R⁹)_{q} oder CR⁸R⁹ bedeutet und Y für den Fall, daß B ein optional substituierter Arylrest, ein optional substituierter Heterocyclylrest, Halogen oder CN ist, auch eine Bindung sein kann,
wobei R⁸ und R⁹ gleich oder verschieden H, Hydroxy, Halogen, CN, (C₁-C₈)Alkoxy oder (C₁-C₈)Alkyl bedeuten, wobei jeder der beiden letztgenannten Reste unsubstituiert oder substituiert ist, und
q = 0, 1 oder 2 ist, und
B ein optional substituierter Arylrest, ein optional substituierter heterocyclischer est, H, OH, Halogen, CN, Nitro, SF₅, (C₁-C₈)Alkyl, (C₂-C₈)Alkenyl oder (C₂-C₈)Alkinyl ist, wobei die letztgenannten 3 Reste unsubstituiert oder substituiert sind, oder ein Acylrest oder
NR¹¹R¹² ist, wobei
R¹¹,R¹² unabhängig voneinander gleich oder verschieden H, (C₁-C₈)Alkyl, (C₂-C₈)Alkenyl, (C₇-C₁₀)Arylalkyl, (C₇-C₁₀)Alkylaryl, (C₆-C₁₀)Aryl oder Heteroaryl sind, wobei jeder der letztgenannten sechs Reste unsubstituiert oder substituiert ist, oder ein Acylrest sind, oder
B ist eine Gruppe der Formel 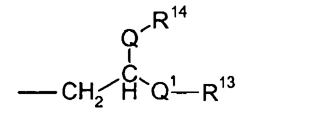 , wobei R¹³ (C₁-C₈)Alkyl ist, welches unsubstituiert oder substituiert ist, R¹⁴ (C₁-C₈)Alkyl ist, welches unsubstituiert oder substituiert ist,
oder R¹³ und R¹⁴ zusammen einen Ring bilden,
Q = O oder S bedeutet, und
Q¹ = O oder S bedeutet.

2. Herbizides oder pflanzenwachstumsregulierendes Mittel, enthaltend a) mindestens eine Verbindung der Formel (I) und/oder deren Salze, gemäß Anspruch 1, und b) im Pflanzenschutz übliche Hilfsmittel.

3. Verfahren zur Bekämpfung von Schadpflanzen oder zur Wachstumsregulierung von Kulturpflanzen, wobei eine wirksame Menge von mindestens einer Verbindung der Formel (I) und/oder deren Salze, gemäß Anspruch 1 oder 2 auf die Pflanzen, Pflanzensamen oder die Fläche, auf der sie wachsen, appliziert wird.

4. Verwendung mindestens einer Verbindung der Formel (I) und/oder deren Salze, gemäß Anspruch 1 als Herbizide oder Pflanzenwachstumsregulatoren.

5. Verwendung nach Anspruch 4, wobei die Verbindung der Formel (I) und/oder deren Salze zur Bekämpfung von Schadpflanzen oder zur Wachstumsregulierung von Kulturpflanzen eingesetzt werden.

6. Verwendung nach Anspruch 5, wobei die Kulturpflanzen transgene Kulturpflanzen sind.

7. Verfahren zur Herstellung einer Verbindung der Formel (I) und/oder deren Salze, gemäß Anspruch 1, worin
a) eine Verbindung der Formel (II) 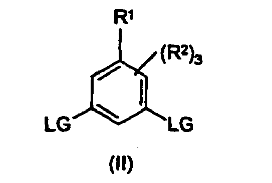 , worin R¹ und R² wie in Formel (I) Anspruch 1 definiert sind und LG gleiche oder voneinander verschiedene Abgangsgruppen sind, mit Nucleophilen der Formel A-X-H und B-Y-H umgesetzt wird, worin A, B, X und Y wie in Formel (I) gemäß Anspruch 1 definiert sind; oder
b) eine Verbindung der Formel (III) mit einer Verbindung der Formel (IV) umgesetzt wird, 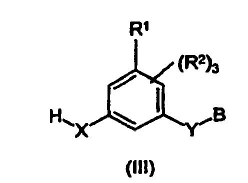
A-Bor(OH)₂ (IV)
oder eine Verbindung der Formel (III') mit einer Verbindung der Formel (IV') umgesetzt wird, 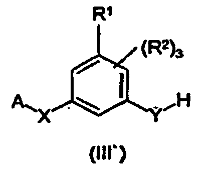
B-Bor(OH)₂ (IV')
wobei R¹, R², A, B, X und Y in den Formeln (III), (III'), (IV) und (IV') wie in Formel (I), gemäß Anspruch 1 definiert sind; oder
c) eine Verbindung der Formel (V) mit einer Verbindung der Formel A-X-H umgesetzt wird, 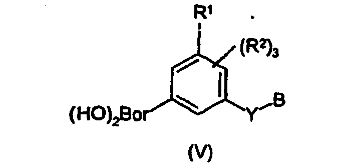 oder eine Verbindung der Formel (V') mit einer Verbindung der Formel B-Y-H umgesetzt wird, 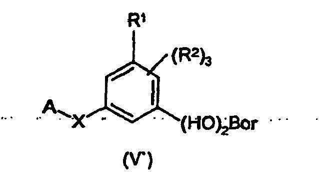 wobei R¹, R², A, B, X und Y in den Formeln (V), (V'), A-X-H und B-Y-H wie in Formel (I), gemäß Anspruch 1 definiert sind; oder
d) eine Verbindung der Formel (VI) reduziert und acyliert wird, 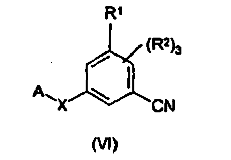 wobei R¹, R², A und X in Formel (VI) wie in Formel (I), gemäß Anspruch 1 definiert sind; oder
e) eine Verbindung der Formel (VI) verseift und mit einem Amin der Formel NH₂-R¹² umgesetzt wird, 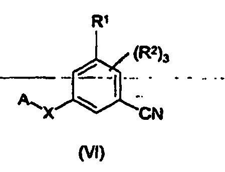 wobei R¹, R², R¹², A und X in den Formeln (VI) und NH₂-R¹² wie in Formel (I), gemäß Anspruch 1 definiert sind; oder
f) eine Verbindung der Formel (VI) mit einer Organometallverbindung umgesetzt wird, 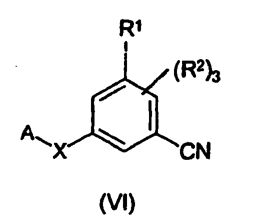
wobei R¹, R², A und X in Formel (VI) wie in Formel (I), gemäß Anspruch 1 definiert sind.

## Claims

1. A compound of the formula (I) and/or a salt thereof 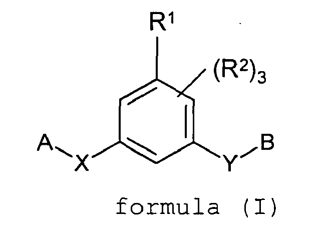 where A is a substituted pyrazolyl, thienyl or pyridyl radical of the formulae (1a), (1c) or (1d) 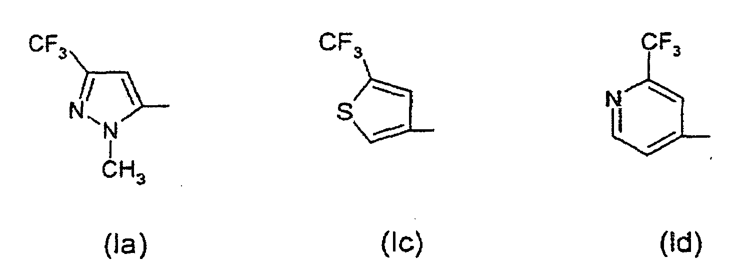 where
X is 0, S or CH₂,
R¹ is hydroxyl, halogen, CN, NC, CHO, CO(C₁-C₈)-alkyl, where the alkyl group is unsubstituted or substituted, CONH₂, CSNH₂, nitro, SF₅, (C₁-C₈)-alkyl, (C₂-C₈)-alkenyl, (C₂-C₈)-alkynyl, (C₁-C₈)-alkoxy, [(C₁-C₈)-alkyl]carbonyl or (C₁-C₈)-alkylsulfonyl, where each of the six last-mentioned radicals is unsubstituted or substituted, or
S(O)ₚ-R³, where
p = 0, 1 or 2 and
R³ is (C₁-C₈)-alkyl, (C₁-C₈)-haloalkyl or NR⁴R⁵, where R⁴,R⁵ independently of one another are identical or different radicals H, (C₁-C₈)-alkyl, (C₂-C₈)-alkenyl, (C₇-C₁₀) -arylalkyl, (C₇-C₁₀) -alkylaryl or (C₆-C₁₀)-aryl, where each of the five last-mentioned radicals is unsubstituted or substituted,
or is NR⁴R⁵, where R⁴, R⁵ independently of one another are identical or different radicals H, (C₁-C₈)-alkyl, (C₂-C₈)-alkenyl, (C₇-C₁₀)-arylalkyl, (C₇-C₁₀)-alkylaryl or (C₆-C₁₀)-aryl, where each of the five last-mentioned radicals is unsubstituted or substituted, or R¹ is a group of the formula 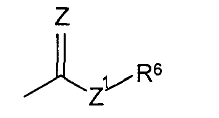 where R⁶ is (C₁-C₈)-alkyl which is unsubstituted or substituted,
Z = O or S and
Z¹ = 0 or S,
R² are identical or different radicals H, halogen, CN or (C₁-C₈)-alkyl, which are unsubstituted or substituted,
Y is O-(CR⁸R⁹)_{q}, S(O)_{q}, NH, CO(CR⁸R⁹)_{q} or CR⁸R⁹ and, if B is a substituted or unsubstituted aryl radical, a substituted or unsubstituted heterocyclyl radical, halogen or CN, Y may also be a bond,
where R⁸ and R⁹ are identical or different radicals H, hydroxyl, halogen, CN, (C₁-C₈)-alkoxy or (C₁-C₈)-alkyl,
where each of the two last-mentioned radicals is unsubstituted or substituted, and
q = 0, 1 or 2, and
B is an unsubstituted or substituted aryl radical, an unsubstituted or substituted heterocyclic radical, H, OH, halogen, CN, nitro, SF₅, (C₁-C₈)-alkyl, (C₂-C₈)-alkenyl or (C₂-C₈)-alkynyl, where the 3 last-mentioned radicals are unsubstituted or substituted, or an acyl radical or NR¹¹R¹², where
R¹¹, R¹² independently of one another are identical or different radicals H, (C₁-C₈)-alkyl, (C₂-C₈)-alkenyl,
(C₇-C₁₀) -arylalkyl, (C₇-C₁₀) -alkylaryl, (C₆-C₁₀) -aryl or heteroaryl, where each of the six last-mentioned radicals is unsubstituted or substituted, or an acyl radical, or
B is a group of the formula 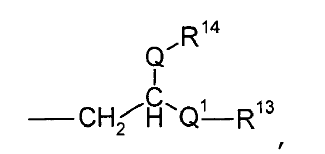 where R¹³ is (C₁-C₈)-alkyl which is unsubstituted or substituted,
R¹⁴ is (C₁-C₈)-alkyl which is unsubstituted or substituted, or R¹³ and R¹⁴ together form a ring,
Q = 0 or S and
Q¹ = 0 or S.

2. A herbicidal or plant-growth-regulating composition, comprising a) at least one compound of the formula (I) and/or a salt thereof, as claimed in claim 1, and b) auxiliaries customary in crop protection.

3. A method for controlling harmful plants or for regulating the growth of crop plants, which comprises applying an effective amount of at least one compound of the formula (I) and/or a salt thereof, as claimed in claim 1 or 2, to the plants, to plant seeds or to the area in which they grow.

4. The use of at least one compound of the formula (I) and/or a salt thereof, as claimed in claim 1, as herbicides or plant growth regulators.

5. The use as claimed in claim 4, where the compound of the formula (I) and/or a salt thereof is used for controlling harmful plants or for regulating the growth of crop plants.

6. The use as claimed in claim 5, where the crop plants are transgenic crop plants.

7. A process for preparing a compound of the formula (I) and/or a salt thereof, as claimed in claim 1, which comprises
a) reacting a compound of the formula (II) 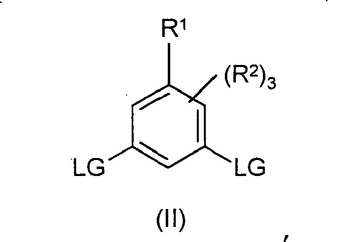 where R¹ and R² are defined as in formula (I) claim 1 and LG are identical or different leaving groups with nucleophiles of the formula A-X-H and B-Y-H, where A, B, X and Y are as defined in formula (I) as set forth in claim 1; or
b) reacting a compound of the formula (III) with a compound of the formula (IV) 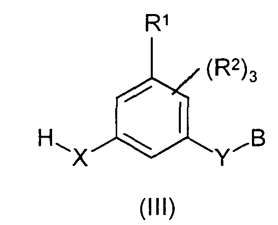
A-Bor(OH)₂ (IV)
or a compound of the formula (III') with a compound of the formula (IV') 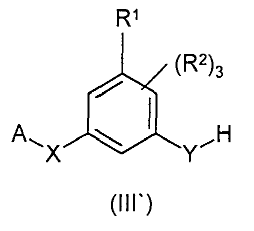
B-Bor(OH)₂ (IV')
where R¹, R², A, B, X and Y in the formulae (III), (III'), (IV) and (IV') are as defined in formula (I) as set forth in claim 1; or
c) reacting a compound of the formula (V) with a compound of the formula A-X-H 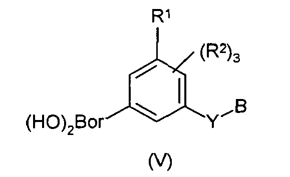 or a compound of the formula (V') with a compound of the formula B-Y-H 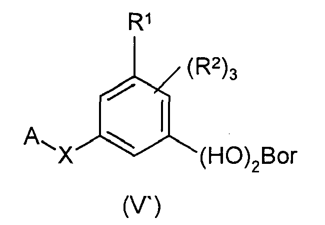 where R¹, R², A, B, X and Y in the formulae (V), (V'), A-X-H and B-Y-H are as defined in formula (I) as set forth in claim 1; or
d) reducing and acylating a compound of the formula (VI) 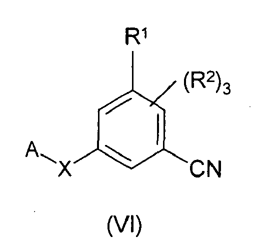 where R¹, R², A and X in formula (VI) are as defined in formula (I) as set forth in claim 1; or
e) hydrolyzing a compound of the formula (VI) and reacting it with an amine of the formula NH₂-R¹² 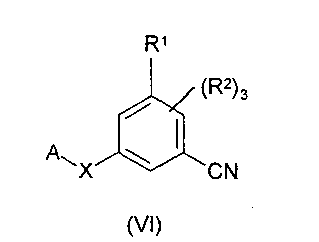 where R¹, R², R¹², A and X in the formulae (VI) and NH₂-R¹² are as defined in formula (I) as set forth in claim 1; or
f) reacting a compound of the formula (VI) with an organometallic compound 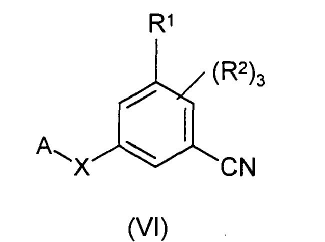
where R¹, R², A and X in formula (VI) are as defined in formula (I) as set forth in claim 1.

## Revendications

1. Composé de formule (1) et/ou ses sels 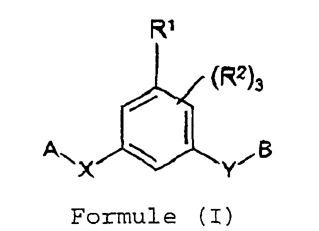 dans laquelle A représente un radical pyrazolyle, thiényle ou pyridyle de formule (Ia), (Ic) ou (Id), 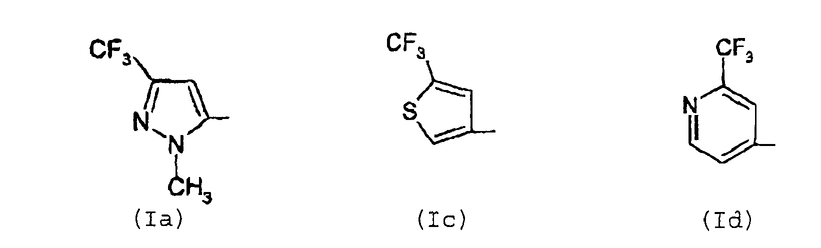 dans laquelle
X représente O, S ou CH₂,
R¹ représente un hydroxy, halogène, CN, NC, CHO, CO-alkyle en C₂ à C₈, dans lesquels le groupe alkyle est non substitué ou substitué, CONH₂, CSNH₂, nitro, SF₅, alkyle en C₁ à C₈, alcényle en C₂ à C₈, alcynyle en C₂ à C₈, alcoxy en C₁ à C₈, (alkyle en C₁ à C₈)-carbonyle ou (alkyle en C₁ à C₈)-sulfonyle, chacun des six derniers radicaux cités étant non substitué ou substitué, ou est S(O)ₚ-R³, dans lequel
p = 0, 1 ou 2 et
R³ représente un alkyle en C₁ à C₈, halogéno-alkyle en C₁ à C₈ ou NR⁴R⁵, R⁴, R⁵ étant indépendamment l'un de l'autre, identiques ou différents, H, un alkyle en C₁ à C₈, alcényle en C₂ à C₈, arylalkyle en C₇ à C₁₀, alkylaryle en C₇ à C₁₀ ou aryle en C₆ à C₁₀, chacun des cinq derniers radicaux cités étant non substitué ou substitué,
ou représente NR⁴R⁵, dans lequel R⁴, R⁵ sont indépendamment l'un de l'autre, identiques
ou différents, H, un alkyle en C₁ à C₈, alcényle en C₂ à C₈, arylalkyle en C₇ à C₁₀, alkylaryle en C₇ à C₁₀ ou aryle en C₆ à C₁₀, chacun des cinq derniers radicaux cités étant non substitué ou substitué,
ou R¹ est un groupe de formule 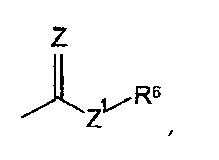
dans laquelle R⁶ est un alkyle en C₁ à C₈, qui est non substitué ou substitué,
Z représente O ou S, et
Z¹ représente O ou S,
R² identiques ou différents, représentent H, un halogène, CN ou un alkyle en C₁ à C₈, qui sont non substitués ou substitués,
Y représente O-(CR⁸R⁹)_{q}, S(O)_{q}, NH, CO(CR⁸R⁹)_{q} ou CR⁸R⁹ et Y dans le cas où B est un radical aryle éventuellement substitué, un radical hétérocyclyle éventuellement substitué, un halogène ou CN, peut aussi être une liaison, R⁸ et R⁹ identiques ou différents, représentant H, un hydroxy, halogène, CN, alcoxy en C₁ à C₈ ou alkyle en C₁ à C₈, chacun des deux derniers radicaux cités étant non substitué ou substitué, et
q est égal à 0, 1 ou 2, et
B est un radical aryle éventuellement substitué, un radical hétérocyclique éventuellement substitué, H, OH, un halogène, CN, un nitro, SF₅, alkyle en C₁ à C₈, alcényle en C₂ à C₈ ou alcynyle en C₂ à C₈, chacun des trois derniers radicaux cités étant non substitué ou substitué, ou un radical acyle ou
est NR¹¹R¹², dans lequel
R¹¹, R¹² indépendamment l'un de l'autre sont, identiques ou différents, H, un alkyle en C₁ à C₈, alcényle en C₂ à C₈, arylalkyle en C₇ à
C₁₀, alkylaryle en C₇ à C₁₀, aryle ou hétéroaryle en C₆ à C₁₀, chacun des six derniers radicaux cités étant non substitué
ou substitué, ou sont un radical acyle, ou
B est un groupe de formule 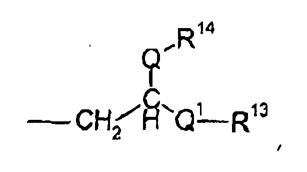 dans laquelle R¹³ est un alkyle en C₁ à C₈, qui est non substitué ou substitué,
R¹⁴ est un alkyle en C₁ à C₈, qui est non substitué ou substitué,
ou R¹³ et R¹⁴ forment ensemble un cycle,
Q représente O ou S, et
Q¹ représente O ou S.

2. Agent herbicide ou de régulation de la croissance végétale, contenant a) au moins un composé de formule (I) et/ou de ses sels, selon la revendication 1, et b) des adjuvants usuels dans la protection des plantes.

3. Procédé de lutte contre les plantes nuisibles ou de régulation de croissance de plantes cultivées, dans lequel on applique une quantité efficace d'au moins un composé de formule (I) et/ou de ses sels, selon la revendication 1 ou 2 sur les plantes, les semences des plantes ou la surface sur laquelle elles poussent.

4. Utilisation d'au moins un composé de formule (I) et/ou de ses sels, selon la revendication 1 comme herbicides ou régulateurs de croissance végétale.

5. Utilisation selon la revendication 4, dans laquelle on utilise le composé de formule (I) et/ou de ses sels pour la lutte contre les plantes nuisibles et/ou pour la régulation de la croissance végétale de plantes cultivées.

6. Utilisation selon la revendication 5, dans laquelle les plantes cultivées sont des plantes cultivées transgéniques.

7. Procédé de préparation d'un composé de formule (I) et/ou de ses sels, selon la revendication 1, dans lequel
a) on met à réagir un composé de formule (II) 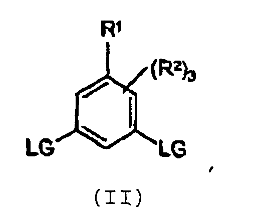 dans laquelle R¹ et R² sont définis comme à la formule (I), revendication 1, et LG sont des groupes partants identiques ou différents entre eux, avec des nucléophiles de formule A-X-H et B-Y-H, dans lesquelles A, B, X et Y sont définis comme à la formule (I) selon la revendication 1 ; ou
b) on met à réagir un composé de formule (III) avec un composé de formule (IV), 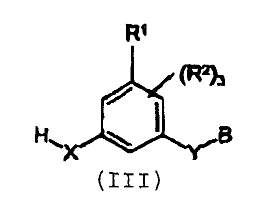
**A-Bor(OH)₂** (IV)
ou on met à réagir un composé de formule (III') avec un composé de formule (IV'), 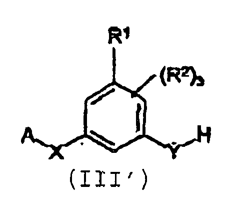
**B-Bor(OH)₂** (IV')
dans lesquelles R¹, R², A, B, X et Y dans les formules (III), (III'), (IV) et (IV') sont définis comme dans la formule (I) selon la revendication 1 ;
ou
c) on met à réagir un composé de formule (V) avec un composé de formule A-X-H, 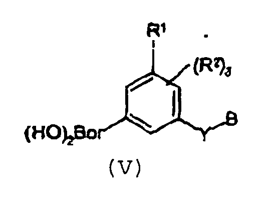 ou on met à réagir un composé de formule (V') avec un composé de formule B-Y-H, 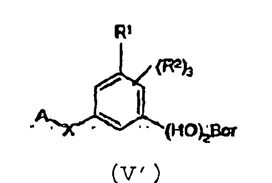 dans lesquelles R¹, R², A, B, X et Y dans les formules (V), (V'), A-X-H et B-Y-H sont définis comme à la formule (I), selon la revendication 1 ; ou
d) on réduit et on acyle un composé de formule (VI), 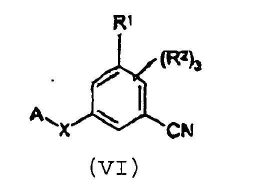 dans laquelle R¹, R², A et X dans la formule (VI) sont définis comme dans la formule (I), selon la revendication 1 ; ou
e) on saponifie un composé de formule (VI) et on le met à réagir avec une amine de formule NH₂-R¹², 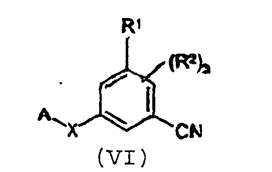 dans laquelle R¹, R², R¹², A et X dans la formule (VI) et NH₂-R¹² sont définis comme dans la formule (I), selon la revendication 1 ; ou
f) on met à réagir un composé de formule (VI) avec un composé organométallique, 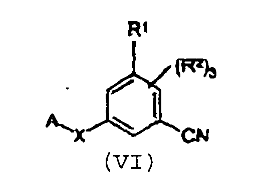
dans laquelle R¹, R², A et X dans la formule (VI) sont définis comme dans la formule (I), selon la revendication 1.
